(19) Europäisches Patentamt
European Patent Office
Office européen des brevets

(11) EP 4 312 986 B1

(12) **EUROPEAN PATENT SPECIFICATION**

(45) Date of publication and mention of the grant of the patent:
**23.10.2024 Bulletin 2024/43**

(21) Application number: **22718720.0**

(22) Date of filing: **31.03.2022**

(51) International Patent Classification (IPC):
***A61K 9/107*** *(2006.01)* ***A61K 31/4704*** *(2006.01)*
***A61K 9/08*** *(2006.01)* ***A61K 47/10*** *(2017.01)*
***A61K 47/18*** *(2017.01)* ***A61K 47/32*** *(2006.01)*
***A61K 47/38*** *(2006.01)* ***A61K 47/44*** *(2017.01)*
***A61P 27/02*** *(2006.01)* ***A61P 27/06*** *(2006.01)*

(52) Cooperative Patent Classification (CPC):
**A61K 9/0048; A61K 9/08; A61K 9/107; A61K 31/4704; A61K 47/10; A61K 47/183; A61K 47/186; A61K 47/32; A61K 47/38; A61K 47/44; A61P 27/02; A61P 27/06**

(86) International application number:
**PCT/EP2022/058523**

(87) International publication number:
**WO 2022/207773 (06.10.2022 Gazette 2022/40)**

(54) **LAQUINIMOD FORMULATION FOR OCULAR USE**

LAQUINIMODFORMULIERUNG ZUR OKULAREN VERWENDUNG

FORMULATION DE LAQUINIMOD POUR UTILISATION OCULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.04.2021 EP 21166691**

(43) Date of publication of application:
**07.02.2024 Bulletin 2024/06**

(73) Proprietor: **Active Biotech AB**
**223 63 Lund (SE)**

(72) Inventors:
- **WÄNNMAN, Hans**
**262 65 ÄNGELHOLM (SE)**
- **TÖRNGREN, Marie**
**247 98 GENARP (SE)**
- **ERIKSSON, Helena**
**247 45 TORNA-HÄLLESTAD (SE)**
- **BUHL, Andreas**
**82152 MARTINSRIED (DE)**

(74) Representative: **Brann AB**
**P.O. Box 3690**
**Sveavägen 63**
**103 59 Stockholm (SE)**

(56) References cited:
**WO-A1-2015/073697 WO-A2-2013/184650**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 312 986 B1

## Description

FIELD OF THE INVENTION

The present invention relates to pharmaceutical formulations for ocular use. More particularly, the invention relates to pharmaceutical formulations containing laquinimod, for ocular use, preferably topical ocular use.

BACKGROUND OF THE INVENTION

Laquinimod is a synthetic quinoline carboxamide with high oral bioavailability that has been suggested as an oral formulation for the treatment of e.g. multiple sclerosis (MS). Laquinimod and pharmaceutically acceptable salts thereof have been described in U.S. Pat. No. 6,077,851.

The use of laquinimod in the treatment of eye diseases, such as glaucoma, inflammatory eye diseases and diseases associated with excessive vascularisation of the eye has been previously disclosed. Thus, laquinimod for use in the treatment of glaucoma was disclosed in the international application No. PCT/US2014/065497, published as WO 2015/073697. US patent application No. 15/816402, published as US 2018/0071275 A1, discloses the treatment of ocular inflammatory diseases by use of laquinimod. The use of laquinimod for the treatment of diseases associated with excessive vascularisation of the eye is disclosed in the international application No. PCT/EP2020/086993, published as WO 2021/123142 A1. For each type of ocular disease, laquinimod is proposed to be administered systemically or topically, and in the latter case, ocular or ophthalmic administration is mentioned.

However, several challenges must be overcome in connection with topical ocular formulations and their effective use in the treatment of an eye disorder, in particular a disorder affecting the posterior parts of the eye. Indeed, treatment of disorders where the drug must reach the posterior parts of the eye is often hampered by inefficient delivery of the active agent to the target site, largely due to the corneal barrier with its several compartments of opposite lipophilic or hydrophilic nature.

SUMMARY OF THE INVENTION

Provided herein is a pharmaceutical formulation as defined in the claims, comprising laquinimod or a pharmaceutically acceptable salt thereof for use in the treatment of an ocular disease.

The formulation provided herein is useful in a method of treating a subject suffering from an ocular disease, the method comprising local, preferably topical, administration to the eye of the subject of the formulation as described herein, containing a therapeutically effective amount laquinimod or a pharmaceutically acceptable salt thereof effective to treat the subject.

One aspect therefore relates to a pharmaceutical formulation as defined in the claims, for local, preferably topical, administration to the eye of a patient (local ocular use, or topical ocular use), comprising a therapeutically effective amount of laquinimod or a pharmaceutically acceptable salt thereof, as a therapeutically active agent.

Thus, in one aspect, a pharmaceutical formulation for ocular administration is provided, said formulation comprising, in an aqueous phase:

(i) a therapeutically effective amount of laquinimod or a pharmaceutically acceptable salt thereof as active ingredient,
(ii) a pharmaceutically acceptable viscosity agent in an amount sufficient to provide a dynamic viscosity of 2 to 200 mPas, as measured at 20 °C,
(iii) a pharmaceutically acceptable tonicity adjusting agent, in an amount sufficient to provide an osmolality of 200 to 600 mOsm/kg,
(iv) a pharmaceutically acceptable humectant,
(v) a pharmaceutically acceptable antioxidant, and
(vi) a pharmaceutically acceptable pH regulating agent.

In a further aspect, a pharmaceutical formulation is provided, for ocular administration of laquinimod or a pharmaceutically acceptable salt of laquinimod, said formulation having a viscosity of 2 mPas to 200 mPas, as measured at 20 °C, an osmolality of 200 mOsm/kg to 600 mOsm/kg, and a pH of 6.8 to 8.5.

In some embodiments, therefore, the pharmaceutical formulation for ocular administration comprises:

(i) laquinimod or a pharmaceutically acceptable salt thereof as active ingredient, in a therapeutically effective amount;
(ii) a pharmaceutically acceptable viscosity agent, in an amount sufficient to provide a dynamic viscosity of 2 to 200 mPas, as measured at 20 °C;
(iii) a pharmaceutically acceptable tonicity adjusting agent, in an amount sufficient to provide an osmolality of 200

to 600 mOsm/kg;
(iv) a pharmaceutically acceptable humectant;
(v) a pharmaceutically acceptable antioxidant; and
(vi) a pharmaceutically acceptable pH regulating agent in an amount sufficient to provide a pH of 6.8 to 8.5.

In some embodiments, the formulation further comprises one or more components selected from (vii) a pharmaceutically acceptable preservative, (viii) a pharmaceutically acceptable surfactant (surface active agent), (ix) a pharmaceutically acceptable solubilizer, and (x) a pharmaceutically acceptable oil.
The formulation may be in the form of a gel or a water and oil containing emulsion (i.e. an oil-in-water emulsion, or a water-in-oil emulsion), containing laquinimod or a pharmaceutically acceptable salt of laquinimod in the form of solute and/or suspended particles.
Thus, in some embodiments, the formulation is an oil-in-water emulsion, comprising a pharmaceutically acceptable oil and an aqueous phase in the form of a suspension or solution of laquinimod or a pharmaceutically acceptable salt of laquinimod.
In some further embodiments, the formulation is a water-in-oil emulsion, comprising an aqueous phase in the form of a suspension or solution of laquinimod or a pharmaceutically acceptable salt of laquinimod, in a pharmaceutically acceptable oil phase. Preferably, the emulsion provided herein is an oil-in-water emulsion.
In some further embodiments, the formulation is a solution or suspension of laquinimod or a pharmaceutically acceptable salt of laquinimod, the formulation being a viscous gel.
The pharmaceutical formulation provided herein is useful for the treatment of various ocular diseases, e.g. glaucoma, ocular inflammatory diseases and diseases associated with excessive vascularisation of the eye.
In some embodiments, a pharmaceutical formulation is provided herein for the treatment of glaucoma. In some further embodiments, an ocular formulation is provided herein for the treatment an ocular inflammatory disease. In some further embodiments, an ocular formulation is provided herein for the treatment of a disease associated with excessive vascularisation of the eye.
In some embodiments, the ocular disease is one affecting the intermediary or posterior parts of the eye.
An advantageous feature of the formulation is a high stability against chemical degradation of laquinimod or the pharmaceutically acceptable salt of laquinimod present in the formulation.
A further advantageous feature of the formulation is a high homogeneity throughout the formulation.
A further advantageous feature is its capacity of containing a wide range of concentrations of laquinimod or a pharmaceutically acceptable salt of laquinimod.
A further advantageous feature of the formulation is a high delivery of the therapeutically active agent, across the cornea of the eye, advantageously allowing for local (e.g. topical) administration of laquinimod to the eye of a subject.
A further advantageous aspect is a high efficacy of laquinimod when administered locally, e.g. topically, to the eye of a patient, e.g. at a level comparable to that obtained by oral administration. Local administration of a drug may advantageously avoid unwanted systemic effects. Thus, one aspect is a pharmaceutical formulation for ocular use comprising, in an aqueous phase:

(i) a therapeutically effective amount of laquinimod or a pharmaceutically acceptable salt thereof as active ingredient,
(ii) a pharmaceutically acceptable viscosity agent in an amount sufficient to provide a dynamic viscosity of 2 to 200 mPas, as measured at 20 °C,
(iii) a pharmaceutically acceptable tonicity adjusting agent, in an amount sufficient to provide an osmolality of 200 to 600 mOsm/kg,
(iv) a pharmaceutically acceptable humectant,
(v) a pharmaceutically acceptable antioxidant, and
(vi) a pharmaceutically acceptable pH regulating agent,
and optionally one or more components selected from
(vii) a pharmaceutically acceptable preservative,
(viii) a pharmaceutically acceptable surfactant,
(ix) a pharmaceutically acceptable solubilizing agent, and
(x) a pharmaceutically acceptable oil.

A further aspect is a dosage container containing the pharmaceutical ocular formulation as provided herein. Thus, also provided herein is a dosage container containing a pharmaceutical formulation for ocular administration, comprising:

(i) a therapeutically effective amount of laquinimod or a pharmaceutically acceptable salt thereof as active ingredient,
(ii) a pharmaceutically acceptable viscosity agent in an amount sufficient to provide a dynamic viscosity of 2 to 200

mPas, as measured at 20 °C,
(iii) a pharmaceutically acceptable tonicity adjusting agent, in an amount sufficient to provide an osmolality of 200 to 600 mOsm/kg,
(iv) a pharmaceutically acceptable humectant,
(v) a pharmaceutically acceptable antioxidant, and
(vi) a pharmaceutically acceptable pH regulating agent
and optionally one or more components selected from:
(vii) a pharmaceutically acceptable preservative,
(viii) a pharmaceutically acceptable surfactant,
(ix) a pharmaceutically acceptable solubilizing agent, and
(x) a pharmaceutically acceptable oil.

The pharmaceutically acceptable pH regulating agent preferably is present in an amount providing a pH of at least 6.8 in the formulation.
A further aspect is a kit comprising a dosage container as mentioned herein, and instructions for use.
The inventive formulation is useful in the manufacture of a medicament for the treatment of an ocular disorder, e.g. an ocular disorder selected from glaucoma, an ocular inflammatory disease, and a disease associated with excessive vascularization of the eye.
Advantageously, a formulation as disclosed herein provides a means for local administration, preferably topical administration, of laquinimod to the eye of a patient (a mammal, such as an animal or human, preferably a human) with few or low (acceptable) side effects, such as stinging of the eye, temporarily blurred vision, and/or tearing of the eye.
Thus, a pharmaceutical, preferably topical, formulation of laquinimod or of a pharmaceutically acceptable salt of laquinimod is provided herein, allowing for the treatment of an eye disease by ocular administration, to a mammal, e.g. a human, in need of such treatment.
Further aspects and advantageous embodiments will become apparent from the following detailed description and examples.

BRIEF DESCRIPTION OF THE DRAWINGS

FIG. 1 shows a photo of a vial containing a formulation as prepared herein, with arrows indicating the points of sampling (top, middle or bottom of vial) for the sedimentation analyses.

FIG. 2 shows a photo of vials with formulations E1, E2, and S1-S8, as described herein, containing laquinimod (With API), and a photo of vials with similar formulations but not containing laquinimod (Without API) at t=0, after re-suspension.

FIG. 3 shows a series of photos of the vials with laquinimod-containing formulations E1, E2, and S1-S8, as described herein, after t=1 week of storage at different temperatures (5°C, 25°C, 30°C and 40°C), after re-suspension.

FIG. 4 shows a series of photos of the vials with laquinimod-containing formulations E1, E2, and S1-S8, as described herein, after t=2 weeks of storage at different temperatures (5°C, 25°C, 30°C and 40°C), before and after re-suspension.

FIG. 5 shows a FlowCam® micrograph of particles in gel formulation S2 (a suspension) and of particles in emulsion E1.

FIG. 6 is a graph showing the cumulative absorption of laquinimod ($\mu g/cm^2$), across bovine cornea *in vitro* (n=6), as a function of time (minutes) over a 4-h period of exposure to formulation S3.

FIG. 7 is a graph showing the cumulative absorption of laquinimod ($\mu g/cm^2$), across bovine cornea *in vitro* (n=6), as a function of time (minutes) over a 4-h period of exposure to formulation S4.

FIG. 8 is a graph showing the cumulative absorption of laquinimod ($\mu g/cm^2$), across bovine cornea *in vitro* (n=6) as a function of time (minutes) over a 4-h period of exposure to formulation S7.

FIG. 9 is a graph showing the cumulative absorption of laquinimod ($\mu g/cm^2$), across bovine cornea *in vitro* (n=6), as a function of time (minutes) over a 4-h period of exposure to formulation E2-2A.

FIG. 10 is a graph showing the cumulative absorption of laquinimod ($\mu g/cm^2$), across bovine cornea *in vitro* (n=6), as a function of time (minutes) over a 4-h period of exposure to formulation S3-1.

FIG. 11 is a graph showing the cumulative absorption of laquinimod ($\mu g/cm^2$), across bovine cornea *in vitro* (n=6), as a function of time (minutes) over a 4-h period of exposure to formulation S3-3A.

FIG. 12 is a graph showing the cumulative absorption of laquinimod ($\mu g/cm^2$), across bovine cornea (n=6), as a function of time (minutes) over a 4-h period of exposure to formulation S3-6.

FIG. 13 is a graph showing the cumulative absorption of laquinimod ($\mu g/cm^2$), across bovine cornea (n=6), as a function of time (minutes) for a period of 4 hours following exposure to formulation S3-7.

FIG. 14 is a graph showing the cumulative absorption of laquinimod ($\mu g/cm^2$), across bovine cornea (n=6), as a function of time (minutes) for a period of 4 hours following exposure to formulation S3-8A.

FIG. 15 is a graph showing the cumulative absorption of laquinimod ($\mu g/cm^2$), across bovine cornea *in vitro* (n=6), as a function of time (minutes) over a 4-h period of exposure to laquinimod in PBS.

FIG. 16 is a graph showing the cumulative absorption of laquinimod ($\mu g/cm^2$), across bovine cornea *in vitro* (n=6), as a function of time (minutes) over a 4-h period of exposure to formulation S3-3A.

FIG. 17 is a graph showing the cumulative absorption of laquinimod ($\mu g/cm^2$), across bovine cornea *in vitro* (n=6), as a function of time (minutes) over a 4-h period of exposure to formulation S7-1.

FIG. 18 is a graph showing mean permeation profiles for laquinimod ($\mu g/cm^2$) across bovine corneas in vitro (n=6) over a 4 h exposure to the PBS formulation and to formulations S3-3A and S7-1, respectively.

FIG. 19 is a graph showing the total posterior uveitis clinical signs in an experimental in vivo mouse model of uveitis, obtained by summing up the clinical scores observed in the left (L) and right (R) eye of each animal in every experimental group, for a period of 20 days after an immunisation and with topical ocular administration of formulation S3-3A to the mice, oral administration of laquinimod, or both topical and oral administration of laquinimod, and with topical or oral administration of vehicle only.

DETAILED DESCRIPTION OF THE INVENTION

Unless defined otherwise or clearly indicated by context, all technical and scientific terms and abbreviations used herein have the same meaning as commonly understood by one of ordinary skill in the field of art to which this disclosure belongs. However, definitions of some of the terms used herein will be given herein below.

*Definitions*

Unless otherwise specified or apparent from the context, the articles "a" and "an" are used herein to refer to one or to more than one (i.e., to at least one) of the grammatical object of the article. By way of example, "an element" generally means one element or more than one element.

As used herein, "API" stands for "active pharmaceutical ingredient", which in connection with the present disclosure is laquinimod or a pharmaceutically acceptable salt of laquinimod.

As used herein "autoimmune disease-associated ocular inflammation" is the inflammation affecting one or more parts of the eye or surrounding eye tissue secondary to an autoimmune disease.

The term "autoimmune disease" includes cell-mediated (e.g., T-cell) as well as antibody mediated (e.g., B-cell) disorders. Such disorders can be *inter alia* arthritic conditions, demyelinating diseases, and inflammatory diseases. For example, the autoimmune disease can be multiple sclerosis, autoimmune hemolytic anemia, autoimmune oophoritis, autoimmune thyroiditis, autoimmune uveoretinitis, Crohn's disease, chronic immune thrombocytopenic purpura, colitis, contact sensitivity disease, diabetes mellitus, Grave's disease, Guillain-Barré syndrome, Hashimoto's disease, idiopathic myxedema, myasthenia gravis, psoriasis, pemphigus vulgaris, rheumatoid arthritis, or systemic lupus erythematosus.

The term "carbomer copolymer type B" refers to a high molecular weight copolymer of acrylic acid and a long chain alkyl methacrylate cross-linked with allyl ethers of polyalcohols. It is typically used as a thickening agent, stabilizer and emulsifier in various pharmaceutical formulations.

The term "dosage container" as used herein refers to a container, such as a bottle, vial, tube, flask etc, suitable

to contain a volume of the formulation as provided herein, either a volume corresponding to a unit (single), or a volume corresponding to more than one dose (multidose). The dosage container may include means allowing for application of a suitable amount of the formulation to an eye of a patient, or such means may be provided separately from the container.

As used herein, "effective" as in an amount effective to achieve an end, i.e., "therapeutically effective amount", means the quantity of a component that is sufficient to yield an indicated therapeutic response without undue adverse side effects (such as toxicity, irritation, or allergic response) commensurate with a reasonable benefit/risk ratio when used in the manner of this disclosure. An effective amount may vary according to factors known in the art, such as the disease state, age, sex, and weight of the human or animal being treated.

The term "excipient" refers to a pharmaceutically acceptable chemical, such as known to those of ordinary skill in the art of pharmacy to aid in the administration of the medicinal agent. It is a compound that is useful in preparing a pharmaceutical composition, generally safe, non-toxic and neither biologically nor otherwise undesirable, and includes excipients that are acceptable for veterinary use as well as human pharmaceutical use.

The term "extent" as used in relation to vascularization, is taken to mean the severity of the vascularisation. Such extent of vascularisation can be assessed using several different measurable parameters, such as the area of vascularisation, the number of vessels in the vascularised area, the length of the vessels in the vascularised area, or the thickness of the vessels in the vascularised area.

The term "humectant" refers to a hydrophilic compound capable of retaining moisture in a formulation.

The term "laquinimod" refers to the compound 5-chloro-N-ethyl-4-hydroxy-1-methyl-2-oxo-N-phenyl-1,2-dihydroquinoline-3-carboxamide, having the structural formula:

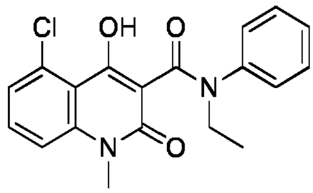

.

Unless otherwise specified or apparent from the context, the term laquinimod includes the free base form of the compound as well as its salt form.

The term "macrogol 15 hydroxystearate" refers to a mixture of mainly monoesters and diesters of 12-hydroxystearic acid and macrogols obtained by the ethoxylation of 12-hydroxystearic acid. Macrogol 15 hydroxystearate is also known as 12-hydroxyoctadecanoic acid polymer with $\alpha$-hydro-$\omega$-hydroxypoly(oxy-1,2-ethanediyl); 12-hydroxystearic acid polyethylene glycol copolymer; macrogol 15 hydroxystearate; polyethylene glycol-15-hydroxystearate; and polyethylene glycol 660 12-hydroxystearate. In some embodiments, the macrogol 15 hydroxystearate is Kolliphor® HS 15 (BASF AG, Germany). Kolliphor® HS 15 consists of polyglycol mono- and di-esters of 12-hydroxystearic acid and about 30% free polyethylene glycol.

The term "mammal" refers to a human or any mammalian animal, e.g. a primate, a farm animal, a pet animal, or a laboratory animal. Examples of such animals are monkeys, cows, sheep, horses, pigs, dogs, cats, rabbits, mice, rats etc. Preferably, the mammal is a human.

The term "surfactant" refers to an organic chemical compound capable of lowering the surface tension (or interfacial tension) between two liquids, between a gas and a liquid, or between a liquid and a solid. A surfactant is an amphiphilic compound, i.e. a compound that contains a hydrophobic moiety ("the hydrophobic tail") as well as a hydrophilic moiety (the "hydrophilic head" or "polar head"). Most commonly, surfactants are classified according to the hydrophilic head. A "non-ionic surfactant" has no electrically charged groups in its head; the hydrophilic head of a "cationic surfactant" carries a net positive electrical charge, and the hydrophilic head of an "anionic surfactant" carries a net negative electrical charge.

The term "tonicity adjusting agent" (or alternatively "tonicity agent"), as used herein, refers to a compound that contributes to the osmolality of a solution. The osmolality of an ocular formulation is preferably adjusted to minimize discomfort to the patient upon ocular administration.

As used herein, the expression "ocular administration" or "ophthalmic administration" etc, of a formulation refers to application of a formulation to the eye of a subject.

The expression "ocular formulation", "ophthalmic formulation" etc as used herein refers to a pharmaceutical composition formulated for administration to the eye of a subject.

As used herein, the term "ocular disease" (which term is considered herein to be synonymous with "ocular disorder", "eye disease", or "eye disorder") refers to a disease affecting the eye of a mammal subject, i.e. an animal or a human, preferably a human.

"Ocular inflammatory disease" or "OID" as used herein means the inflammation affecting one or more parts of

the eye or surrounding eye tissue. OID may include, but is not limited to, inflammation of the orbital tissues, the lacrimal apparatus, the eyelid, the conjunctiva (conjunctivitis), the cornea, the retina, a component of the optic pathway, e.g., the optic nerve, and a component of the uveal tract (uveitis), i.e., the iris, ciliary body and choroid. Specific examples of OID include uveitis, acute conjunctivitis, viral conjunctivitis, nongonococcal bacterial conjunctivitis, adult gonococcal conjunctivitis, inclusion conjunctivitis, seasonal allergic conjunctivitis, chronic conjunctivitis, granular conjunctivitis, perennial allergic conjunctivitis, episcleritis, scleritis, atopic keratoconjunctivitis, and vernal keratoconjunctivitis.

"Optional" or "optionally" means that the subsequently described event or circumstance may or may not occur, and that the description includes instances where the event or circumstance occurs and instances where it does not.

The term "pH regulating agent" refers generally to a compound or mixture of compounds, capable of changing and/or maintaining the pH of an aqueous phase. A common example of a pH regulating agent is a pH buffer (or buffering agent).

By "pharmaceutically acceptable" is meant a material that is not biologically or otherwise undesirable, i.e., the material can be administered to an individual along with the relevant active compound without causing clinically unacceptable biological effects or interacting in a deleterious manner with any of the other components of the formulation in which it is contained.

The term "polyoxyethylene-polyoxypropylene block copolymer" refers to poloxamers of CAS Registry No. 9003-11-6, including salts and known equivalents thereof. Examples of poloxamers are poloxamer 188 and poloxamer 407.

The term "polyoxyl castor oil", (also sometimes referred to as ethoxylated castor oil, or polyethylene glycol castor oil) of CAS Registry No. 61791-12-6, mixture of triricinoleate esters of ethoxylated glycerol with small amounts of polyethylene glycol ricinoleate and the corresponding free glycols. It is a nonionic surfactant, which may be used as emulsifying agent or solubilising agent. The mixture may also be referred to as polyoxyl n castor oil, where n represents the number of oxyethylene units in the compound. An example of a commercially available product is Kolliphor® EL, which is polyoxyl 35 castor oil.

The term "polysorbate 80" refers to the compound of CAS Registry No. 9005-65-6, also known as polyoxyethylene (80) sorbitan monooleate, sorbitan monooleate ethoxylate, and the like, as well as salts and known equivalents thereof.

A "preservative", as used herein, refers to an additive which inhibits both microbial growth and kills microorganisms that contaminate a formulation exposed to the surroundings.

The term "solubilizing agent" (or alternatively "solubilizer") refers to a compound that, when added to a solvent phase or formulation, is capable of increasing the solubility of another compound in said solvent phase or formulation.

The term "solubilizing effective amount" of a substance ("solubilizer") within a formulation refers to an amount of the substance sufficient to solubilize another component of the composition. For example, an "API-solubilizing effective amount" is an amount sufficient to solubilize an API (which in the present case is laquinimod or a pharmaceutically acceptable salt thereof) such that the API is more therapeutically effective as compared to the absence of the solubilizer. In some embodiments an "API-solubilizing effective amount" is an amount sufficient to solubilize an API such that the API is more therapeutically effective as compared to the absence of the solubilizer in an ocular (ophthalmic) formulation, e.g. a topical ocular formulation.

As used herein, "treating" encompasses, e.g., inducing inhibition, regression, or stasis of a disease, disorder or condition, or ameliorating or alleviating a symptom of a disease, disorder or condition. "Ameliorating" or "alleviating" a condition or state as used herein shall mean to relieve or lessen the symptoms of that condition or state. "Inhibition" of disease progression or disease complication in a subject as used herein means preventing or reducing the disease progression and/or disease complication in the subject.

The term "unit dose" as used herein is the amount of the inventive formulation to be administered to the subject in a single administration, or the amount of laquinimod, or salt of laquinimod contained in said amount of the inventive formulation. The unit dose disclosed herein can be administered once daily, twice daily, three times daily, four times daily, five times daily, every other day, weekly, twice weekly, three times weekly, four times weekly, five times weekly or six times weekly.

By "vascularisation" and "neovascularisation" is meant a process by which new blood vessels form. The terms "vascularisation" and "neovascularisation" are used interchangeably herein.

As used herein, the expression "vascularisation of the eye" is synonymous to ocular neovascularisation.

By "excessive vascularisation" is meant an event wherein vascularisation occurs to an extent that is deleterious to the normal functioning of the affected tissue. Such excessive vascularisation occurs during or as an effect of eye diseases or eye disorders such as corneal neovascularisation, neovascularisation of the iris, neovascularisation of the ciliary body, corneal pannus, choroidal neovascularisation, proliferative diabetic retinopathy, retinopathy of prematurity, ischemic retinopathy, retinal neovascularisation, hypertensive retinopathy, and wet age-related macular degeneration.

In the context of the present disclosure, the terms "an ocular disease or ocular disorder associated with excessive vascularisation of the eye" and "an ocular disease or ocular disorder associated with vascularisation of the eye" are

taken to mean any ocular disease or ocular disorder which is considered by those of skill in the art to be caused by, and/or affect vascularisation of one or more tissues of the eye, e.g. in which said vascularisation is deleterious to the normal functioning of the affected tissue. Such diseases or disorders can lead to loss of vision.

The term "viscosity" as used herein refers to the dynamic viscosity.

The term "viscosity agent" (which in the technical field of the invention may also be referred to as viscosity enhancing agent, viscosity enhancer, viscosity modifying agent, viscosity imparting agent, thickening agent, thickener etc.) refers to an agent capable of increasing the viscosity of a liquid when admixed with the liquid.

*The pharmaceutical formulation*

Provided herein is a pharmaceutical formulation for ocular administration comprising, in an aqueous phase:

(i) a therapeutically effective amount of laquinimod or a pharmaceutically acceptable salt thereof as active ingredient,
(ii) a pharmaceutically acceptable viscosity agent in an amount sufficient to provide a dynamic viscosity of 2 to 200 mPas, as measured at 20 °C,
(iii) a pharmaceutically acceptable tonicity adjusting agent, in an amount sufficient to provide an osmolality of 200 to 600 mOsm/kg,
(iv) a pharmaceutically acceptable humectant,
(v) a pharmaceutically acceptable antioxidant, and
(vi) a pharmaceutically acceptable pH regulating agent,
and optionally one or more components selected from
(vii) a pharmaceutically acceptable preservative,
(viii) a pharmaceutically acceptable surfactant,
(ix) a pharmaceutically acceptable solubilizing agent, and
(x) a pharmaceutically acceptable oil.

*The active ingredient*

The ocular (e.g. topical ocular) formulation provided herein is a pharmaceutical formulation and comprises laquinimod or a pharmaceutically acceptable salt thereof at a concentration suitable to provide a therapeutically effective amount of laquinimod or pharmaceutically effective amount by administration to the eye (ocular administration), preferably by topical adminstration to the eye, e.g. as an eye drop formulation.

In some embodiments, laquinimod is present in the free base form (i.e. non-salt form). In other embodiments, the formulation comprises a pharmaceutically acceptable salt of laquinimod, e.g. a metal salt, e.g. a salt comprising a metal selected from lithium, sodium, potassium, magnesium, calcium, manganese, copper, zinc, aluminum and iron. In some embodiments, the pharmaceutically acceptable salt of laquinimod is laquinimod sodium.

In some embodiments, the concentration of laquinimod in the formulation is about 5 g/l to about 100 g/l (or a corresponding concentration of a pharmaceutically acceptable salt of laquinimod), e.g. about 10 to about 100 g/l, or about 20 to about 100 g/l, or about 50 to about 100 g/l.

In some embodiments, the concentration of laquinimod in the formulation is within the range of 5 to 70 g/l, 10 g/l to 70 g/l, or 20 to 70 g/l. In some embodiments, the concentration of laquinimod in the formulation is within the range of 5 to 50 g/l, 10 to 50 g/l, or 20 to 50 g/l. In some embodiments, the concentration of laquinimod in the formulation is within the range of 5 to 30 g/l, e.g. 10 to 30 g/l, or 20 to 30 g/l.

In some embodiments, the concentration of laquinimod in the formulation is within the range of 5 to 25 g/l, 10 to 25 g/l, or 20 to 25 g/l.

In some embodiments, the concentration of laquinimod in the formulation is within the range of 5 to 20 g/l, 5 to 19 g/l, 5 to 18 g/l, 5 to 17 g/l, 5 to 16 g/l, 5 to 15 g/l, 5 to 14 g/l, 5 to 13 g/l, 5 to 12 g/l, or 5 to 11 g/l.

In some embodiments, the concentration of laquinimod in the formulation is within the range of 6 to 20 g/l, 6 to 19 g/l, 6 to 18 g/l, 6 to 17 g/l, 6 to 16 g/l, 6 to 15 g/l, 6 to 14 g/l, 6 to 13 g/l, 6 to 12 g/l, or 6 to 11 g/l.

In some embodiments, the concentration of laquinimod in the formulation is within the range of 7 to 20 g/l, 7 to 19 g/l, 7 to 18 g/l, 7 to 17 g/l, 7 to 16 g/l, 7 to 15 g/l, 7 to 14 g/l, 7 to 13 g/l, 7 to 12 g/l, or 7 to 11 g/l.

In some embodiments, the concentration of laquinimod in the formulation is within the range of 8 to 20 g/l, 8 to 19 g/l, 8 to 18 g/l, 8 to 17 g/l, 8 to 16 g/l, 8 to 15 g/l, 8 to 14 g/l, 8 to 13 g/l, 8 to 12 g/l, or 8 to 11 g/l.

In some embodiments, the concentration of laquinimod in the formulation is within the range of 9 to 20 g/l, 9 to 19 g/l, 9 to 18 g/l, 9 to 17 g/l, 9 to 16 g/l, 9 to 15 g/l, 9 to 14 g/l, 9 to 13 g/l, 9 to 12 g/l, or 9 to 11 g/l.

In some embodiments, the concentration of laquinimod in the formulation is within the range of 10 to 20 g/l, 10 to 19 g/l, 10 to 18 g/l, 10 to 17 g/l, 10 to 16 g/l, 10 to 15 g/l, 10 to 14 g/l, 10 to 13 g/l, 10 to 12 g/l, or 10 to 11 g/l.

In some embodiments, the concentration of laquinimod in the formulation is 5 g/l. In some embodiments, the

concentration of laquinimod is 10 g/l. In some embodiments, the concentration of laquinimod is 15 g/l. In some embodiments, the concentration of laquinimod is 20 g/l. In some embodiments, the concentration of laquinimod is 25 g/l. In some embodiments, the concentration of laquinimod is 30 g/l. In some embodiments, the concentration of laquinimod is 35 g/l. In some embodiments, the concentration of laquinimod is 40 g/l. In some embodiments, the concentration of laquinimod is 50 g/l. In some embodiments, the concentration of laquinimod is 60 g/l. In some embodiments, the concentration of laquinimod is 70 g/l. In some embodiments, the concentration of laquinimod is 80 g/l. In some embodiments, the concentration of laquinimod is 90 g/l. In some embodiments, the concentration of laquinimod is 100 g/l.

As used herein "g/l" designates the amount (g) of laquinimod (as a free base or in the form of a pharmaceutically acceptable salt), per volume (l) of formulation. It is noted that the concentration may also be expressed in mg/ml, 1 g/l being equivalent to 1 mg/ml. Unless otherwise specified or apparent from the context, the indicated amount refers to the free base form, and the person of ordinary skill will be well capable of calculating the corresponding concentration or amount of a salt of laquinimod, if such is used.

In the pharmaceutical formulation provided herein, laquinimod will have a high stability against chemical decomposition (or "degradation"), e.g. by oxidation, which advantageously allows for a long shelf life. Thus, in some advantageous embodiments, the amount of oxidation decomposition product present in the composition is not more than 0.1% w/w relative to the amount of laquinimod or, more preferably is not more than 0.05% w/w even more preferably is not more than 0.01% w/w, or is undetectable, e.g. after a period of at least 3 months, preferably at least 6 months, more preferably at least 1 year, even more preferably at least 2 years, e.g. when kept at room temperature (about 18-25 °C) in a sealed container suitable for pharmaceutical formulations.

*The pharmaceutically acceptable viscosity agent*

The formulation provided herein has a (dynamic) viscosity of from about 2 mPas to about 200 mPas when measured at a temperature of 20 °C using a method as described herein, (the falling ball method). In some embodiments, the viscosity ranges from about 2 mPas to about 180 mPas, from about 2 mPas to about 160 mPas, from about 2 mPas to about 150 mPas, from about 2 mPas to about 140 mPas, from about 2 mPas to about 130 mPas, from about 2 mPas to about 120 mPas, from about 2 mPas to about 110 mPas, from about 2 mPas to about 100 mPas, from about 2 mPas to about 90 mPas, or from about 2 mPas to about 80 mPas, e.g. from about 2 mPas to about 75 mPas.

In some of these embodiments, the viscosity is at least 3 mPas, at least 4 mPas, at least 5 mPas, at least 6 mPas, at least 7 mPas, at least 8 mPas, at least 9 mPas, at least 10 mPas, at least 11 mPas, at least 12 mPas, at least 13 mPas, at least 14 mPas, or at least 15 mPas.

In some of these embodiments, the viscosity is at most 180 mPas, at most 170 mPas, at most 160 mPas, at most 150 mPas, at most 130 mPas, at most 120 mPas, at most 110 mPas, at most 100 mPas, at most 90 mPas, at most 80 mPas, at most 75 mPas, at most 70 mPas, at most 65 mPas, at most 60 mPas, at most 55 mPas, at most 50 mPas, at most 45 mPas, at most 40 mPas, at most 35 mPas, at most 30 mPas, at most 25 mPas, or at most 20 mPas.

For example, in some embodiments, the viscosity is within a range of from about 2 mPas to about 70 mPas, or from about 2 mPas to about 65 mPas, or from about 2 mPas to about 60 mPas, or from about 2 mPas to about 55 mPas, or from about 2 mPas to about 50 mPas, or from about 2 mPas to about 45 mPas, or from about 2 mPas to about 40 mPas, or from about 2 mPas to about 35 mPas, or from about 2 mPas to about 30 mPas, or from about 2 mPas to about 25 mPas, or from about 2 mPas to about 20 mPas. In some of these embodiments, the viscosity is at least 3 mPas, at least 4 mPas, at least 5 mPas, at least 6 mPas, at least 7 mPas, at least 8 mPas, at least 9 mPas, at least 10 mPas, at least 11 mPas, at least 12 mPas, at least 13 mPas, at least 14 mPas, or at least 15 mPas.

Thus, in some embodiments, the viscosity of the formulation is within a range of from about 5 mPas to about 75 mPas, or about 5 mPas to about 70 mPas, or from about 5 mPas to about 65 mPas, or from about 5 mPas to about 60 mPas, or from about 5 mPas to about 55 mPas, or from about 5 mPas to about 50 mPas, or from about 5 mPas to about 45 mPas, or from about 5 mPas to about 40 mPas, or from about 5 mPas to about 35 mPas, or from about 5 mPas to about 30 mPas, or from about 5 mPas to about 25 mPas, or from about 5 mPas to about 20 mPas.

In some further embodiments, the viscosity of the formulation is within a range of from about 10 mPas to about 75 mPas, or about 10 mPas to about 70 mPas, or from about 10 mPas to about 65 mPas, or from about 10 mPas to about 60 mPas, or from about 10 mPas to about 55 mPas, or from about 10 mPas to about 50 mPas, or from about 10 mPas to about 45 mPas, or from about 10 mPas to about 40 mPas, or from about 10 mPas to about 35 mPas, or from about 10 mPas to about 30 mPas, or from about 10 mPas to about 25 mPas, or from about 10 mPas to about 20 mPas. In some of these embodiments, the viscosity is at least 11 mPas, at least 12 mPas, at least 13 mPas, or at least 14 mPas.

Thus, in some further embodiments, the viscosity of the formulation is within a range of from about 12 mPas to about 75 mPas, or about 12 mPas to about 70 mPas, or from about 12 mPas to about 65 mPas, or from about 12 mPas to about 60 mPas, or from about 12 mPas to about 55 mPas, or from about 12 mPas to about 50 mPas, or from about 12 mPas to about 45 mPas, or from about 12 mPas to about 40 mPas, or from about 12 mPas to about 35 mPas, or from about 12 mPas to about 30 mPas, or from about 12 mPas to about 25 mPas, or from about 12 mPas to about

20 mPas.

In some further embodiments, the viscosity of the formulation is within a range of from about 15 mPas to about 75 mPas, or about 15 mPas to about 70 mPas, or from about 15 mPas to about 65 mPas, or from about 15 mPas to about 60 mPas, or from about 15 mPas to about 55 mPas, or from about 15 mPas to about 50 mPas, or from about 15 mPas to about 45 mPas, or from about 15 mPas to about 40 mPas, or from about 15 mPas to about 35 mPas, or from about 15 mPas to about 30 mPas, or from about 15 mPas to about 25 mPas, or from about 15 mPas to about 20 mPas.

The herein indicated viscosity is the dynamic viscosity as measured at a temperature of 20°C, e.g. using a falling ball viscometer as described herein.

The formulation comprises one or more pharmaceutically acceptable viscosity agents.

Suitable viscosity agents for use herein include polyvinyl alcohol, poly(acrylic acid) homo- or copolymers (carbomers), and various cellulose-based polymers, e.g. hydroxypropylmethylcellulose and sodium carboxymethyl cellulose.

In some embodiments, the pharmaceutically acceptable viscosity agent comprises one or more of the group consisting of polyvinyl alcohol, poly(acrylic acid) homo- or copolymers (carbomers), polyvinylpyrrolidone, and cellulose derivatives, such as hydroxypropylmethylcellulose and sodium carboxymethyl cellulose.

In some embodiments, the viscosity agent is selected from cellulose-based polymers, polyvinyl alcohol, and poly(acrylic acid) homo- and copolymers, and combinations thereof.

In some embodiments, the viscosity agent comprises a cellulose-based polymer (or cellulose derivative), such as hydroxypropylmethylcellulose or sodium carboxymethyl cellulose, e.g. the viscosity agent comprises sodium carboxymethyl cellulose. In some embodiments, the viscosity agent comprises a polyvinyl alcohol. In some embodiments, the viscosity agent comprises a poly(acrylic acid) homopolymer. In some embodiments, the viscosity agent comprises a poly(acrylic acid) copolymer.

In a carbomer as used herein, the polymer may be a homopolymer of acrylic acid, or may be crosslinked with an allyl ether of pentaerythritol, allyl ether of sucrose, or allyl ether of propylene. In some embodiments, the carbomer is a homopolymer of acrylic acid, (a carbomer homopolymer, e.g. carbomer homopolymer type B). In some other embodiments, the carbomer is a crosslinked copolymer.

The viscosity agent (which term may refer to either one particular viscosity agent or a mixture of such agents) is present in a total amount sufficient to provide the desired viscosity in the formulation, i.e. a viscosity within the ranges as mentioned herein above. As will be apparent to the person of ordinary skill in the art, the exact amount will vary as a function of the particular viscosity agent(s) selected, and furthermore will depend on the other ingredients in the formulation, and the concentrations of these other ingredients. The person of ordinary skill in the art will be able to determine the required amount of viscosity agent in light of the present description and illustrating examples provided herein.

It has been noted that the viscosity of the formulation has a tendency to decrease when the concentration of laquinimod is increased, and the amount of any given viscosity agent will generally have to be determined and adjusted depending on in particular the laquinimod concentration of the formulation.

*The pharmaceutically acceptable tonicity adjusting agent*

Lachrymal fluid is isotonic with blood having an isotonicity value corresponding to that of a 0.9% NaCl solution. Ideally, therefore, an ophthalmic formulation will be isotonic with lachrymal fluid, though a tonicity ranging from about that of a 0.6% NaCl solution to a 2% NaCl solution is generally tolerable to the eye. In case a small volume of an ocular formulation is administered, the tonicity may deviate from within this range, because dilution with lachrymal fluid may quickly reduce discomfort. Preferably, though the formulation should be approximately isotonic. The formulation provided herein comprises a pharmaceutically acceptable tonicity adjusting agent, preferably a non-ionic tonicity adjusting agent, e.g. one or more compounds selected from mannitol, sorbitol, glycerol, polyethylene glycol (PEG), polypropylene glycol (PPG), and sorbitol, although the tonicity agent is not limited to this selection, as other alternative tonicity agents are well-known within the technical field. A preferred non-ionic tonicity adjusting agent is mannitol.

In some embodiments, the tonicity adjusting agent comprises mannitol. In some embodiments, the tonicity adjusting agent is mannitol.

In some embodiments, the tonicity adjusting agent (e.g. mannitol), is present in the formulation at a concentration of about 0.5 to about 5 g/l of formulation, about 0.5 to about 4.5 g/l, about 0.5 to about 4 g/l, about 0.5 to about 3.5 g/l, or about 0.5 to about 3 g/l.

In some embodiments, the tonicity adjusting agent (e.g. mannitol), is present in the formulation at a concentration of about 1 to about 5 g/l, about 1 to about 4.5 g/l, about 1 to about 4 g/l, about 1 to about 3.5 g/l, or about 1 to about 3 g/l.

In some embodiments, the tonicity adjusting agent (e.g. mannitol), is present in the formulation at a concentration of about 1.5 to about 5 g/l, about 1.5 to about 4.5 g/l, about 1.5 to about 4 g/l, about 1.5 to about 3.5 g/l, or about 1.5 to about 3 g/l.

In some embodiments, the tonicity adjusting agent (e.g. mannitol), is present in the formulation at a concentration

of about 2 to about 5 g/l, about 2 to about 4.5 g/l, about 2 to about 4 g/l, about 2 to about 3.5 g/l, or about 2 to about 3 g/l.

In some embodiments, the tonicity adjusting agent (e.g. mannitol), is present in the formulation at a concentration of about 2.5 to about 5 g/l, about 2.5 to about 4.5 g/l, about 2.5 to about 4 g/l, about 2.5 to about 3.5 g/l, or about 2.5 to about 3 g/l.

Ideally, a pharmaceutical formulation for ocular administration should preferably have an osmolality within a range of 200 to 600 mOsm/kg, in order not to cause discomfort on application to the eye, though values somewhat outside this range may be tolerated in case the amount of formulation applied to the eye is small.

In some embodiments, the formulation of the invention has an osmolality within a range of about 200 to about 550 mOsm/kg, about 200 to about 500 mOsm/kg, about 200 to about 450 mOsm/kg, or about 200 to about 400 mOsm/kg, or about 200 to about 350 mOsm/kg.

In some embodiments, the formulation of the invention has an osmolality within a range of about 300 to about 600 mOsm/kg, about 300 to about 550 mOsm/kg, about 300 to about 500 mOsm/kg, about 300 to about 450 mOsm/kg, about 300 to about 400 mOsm/kg, or about 300 to about 350 mOsm/kg.

In some embodiments, the formulation of the invention has an osmolality within a range of about 400 to about 600 mOsm/kg, about 400 to about 550 mOsm/kg, about 400 to about 500 mOsm/kg, or about 400 to about 450 mOsm/kg.

In some embodiments, the formulation of the invention has an osmolality within a range of about 450 to about 600 mOsm/kg, about 450 to about 550 mOsm/kg, or about 450 to about 500 mOsm/kg.

In some embodiments, the formulation of the invention has an osmolality within a range of about 500 to about 600 mOsm/kg, or about 500 to about 550 mOsm/kg.

In some embodiments, the formulation of the invention has an osmolality within a range of about 250 to about 500 mOsm/kg, about 250 to about 450 mOsm/kg, about 250 to about 400 mOsm/kg, about 250 to about 375 mOsm/kg, about 250 to about 350 mOsm/kg, or about 250 to about 325 mOsm/kg.

In some embodiments, the formulation of the invention has an osmolality within a range of about 260 to about 375 mOsm/kg, about 270 to about 375 mOsm/kg, about 280 to about 375 mOsm/kg, or about 290 to about 375 mOsm/kg.

In some embodiments, the formulation of the invention has an osmolality within a range of about 260 to about 350 mOsm/kg, about 270 to about 350 mOsm/kg, about 280 to about 350 mOsm/kg, or about 290 to about 350 mOsm/kg.

In some embodiments, the formulation of the invention has an osmolality within a range of about 260 to about 320 mOsm/kg, about 270 to about 320 mOsm/kg, or about 280 to about 320 mOsm/kg, or about 285 to about 315 mOsm/kg, or about 290 to about 310 mOsm/kg, or about 295 to about 305 mOsm/kg, e.g. about 300 mOsm/kg.

*The pharmaceutically acceptable humectant*

The formulation of the invention comprises a humectant (within the technical field also sometimes referred to as "wetting agent"), e.g. a polyol, such as a C3-C6 polyol, e.g. a C3-C5 polyol, or a C3-C4 polyol, such as sorbitol, xylitol, or glycerol, or a mixture of one or more such polyols. Preferably, the humectant comprises glycerol. In some embodiments, the humectant is glycerol.

In some embodiments, the humectant (e.g. glycerol), is present in the formulation at a concentration of about 5 to about 50 g/l of formulation, about 5 to about 45 g/l, about 5 to about 40 g/l, about 5 to about 35 g/l, or about 5 to about 30 g/l. In some embodiments, the humectant (e.g. glycerol), is present in the formulation at a concentration of about 10 to about 50 g/l, about 10 to about 45 g/l, about 10 to about 40 g/l, about 10 to about 35 g/l, about 10 to about 30 g/l, about 10 to 25 g/l, or about 10 to 20 g/l. In some embodiments, the humectant (e.g. glycerol), is present in the formulation at a concentration of about 15 to about 50 g/l, about 15 to about 45 g/l, about 15 to about 40 g/l, about 15 to about 35 g/l, about 15 to about 30 g/l, about 15 to 25 g/l, or about 15 to 20 g/l. In some embodiments, the humectant is present in the formulation at a concentration of about 16 to about 20 g/l, about 16 to about 19 g/l, or about 16 to about 18 g/l, e.g. about 17 g/l. In some embodiments, the humectant (e.g. glycerol), is present in the formulation at a concentration of about 20 to about 50 g/l, about 20 to about 45 g/l, about 20 to about 40 g/l, about 20 to about 35 g/l, or about 20 to about 30 g/l.

*The pharmaceutically acceptable antioxidant*

In order to protect in particular laquinimod from chemical degradation, the formulation contains a pharmaceutically acceptable antioxidant, such as the disodium salt of ethylenediaminetetraacetic acid (EDTA).

In some embodiments, the pharmaceutically acceptable antioxidant comprises disodium salt of ethylenediaminetetraacetic acid (EDTA). In some embodiments, the pharmaceutically acceptable antioxidant is disodium salt of ethylenediaminetetraacetic acid (EDTA).

In some embodiments, the concentration of the antioxidant in the formulation is withing the range of about 0.1 g/l to about 5 g/l, about 0.2 g/l to about 5 g/l, or about 0.5 g/l to about 5 g/l. In some embodiments, the concentration of the antioxidant is withing the range of about 0.1 g/l to about 2 g/l, about 0.2 g/l to about 2 g/l, or about 0.5 g/l to about 2

g/l. In some embodiments, the concentration of the antioxidant is withing the range of about 0.1 g/l to about 1.5 g/l, about 0.2 g/l to about 1.5 g/l, or about 0.5 g/l to about 1.5 g/l.

The amount of antioxidant present in the formulation will generally depend on the amount of laquinimod contained therein. In some embodiments, the formulation may contain antioxidant in a weight ratio to laquinimod (weight of antixodiant : weight of laquinimod) in a range of about 1:50 to about 1:5, about 1:25 to about 1:5, about 1:20 to about 1:5, or about 1:15 to about 1:5; e.g. about 1:50 to about 1:8, about 1:25 to about 1:8, about 1:20 to about 1:8, about 1:15 to about 1:8, or about 1:12 to about 1:8, such as about 1:10.

*The pharmaceutically acceptable pH regulating agent*

It is preferred that the pH regulation agent be present in the formulation in an amount sufficient to provide a pH of at least 6.8 in the formulation. Preferably, the formulation provided herein has a pH of from about 6.8 to about 8.5, e.g. a pH of from 7 to 8.5, or a pH of from 7.4 to 8.5 (when measured at a temperature of 25 °C). In some embodiments, the pH is at most 8.4. For example, in some embodiments the formulation has a pH of from 7.0 to 8.4, e.g. from 7.4 to 8.4, or from 8.0 to 8.4. In some embodiments, the pH is at most 8.0. For example, in some embodiments the formulation has a pH of from 7.0 to 8.0, e.g. from 7.4 to 8.0. In some further embodiments, the formulation has a pH in the range of 6.8 to 8.0, e.g. 6.8 to 7.9, or 6.8 to 7.8, or 6.8 to 7.7, or 6.8 to 7.6, or 6.8 to 7.5, or 6.8 to 7.4. In some further embodiments, the formulation has a pH in the range of 6.9 to 8.0, e.g. 6.9 to 7.9, or 6.9 to 7.8, or 6.9 to 7.7, or 6.9 to 7.6, or 6.9 to 7.5. In some further embodiments, the formulation has a pH in the range of 7.0 to 7.9, or 7.0 to 7.8, or 7.0 to 7.7, or 7.0 to 7.6, or 7.0 to 7.5. In some further embodiments, the formulation has a pH in the range of 7.1 to 7.9, or 7.1 to 7.8, or 7.1 to 7.7, or 7.1 to 7.6, or 7.1 to 7.5. In some further embodiments, the formulation has a pH in the range of 7.2 to 7.9, or 7.2 to 7.8, or 7.2 to 7.7, or 7.2 to 7.6, or 7.2 to 7.5. In some further embodiments, the formulation has a pH in the range of 7.3 to 7.9, or 7.3 to 7.8, or 7.3 to 7.7, or 7.3 to 7.6, or 7.3 to 7.5. In some embodiments, the formulation has a pH of about 7.4.

The formulation contains a pH regulating agent to provide a pH within the above-mentioned ranges. For example, the formulation may contain from about 1 to about 5 g/l of pH regulating agent, e.g. about 1 to about 2 g/l of pH regulating agent.

In some embodiments, the pH regulating agent comprises one or more pH buffering agents, e.g. selected from TRIS (tris(hydroxymethyl)aminomethane, IUPAC name: 2-amino-2-(hydroxymethyl)propane-1,3-diol) and disodium hydrogen phosphate dihydrate. In some embodiments, the pH regulating agent comprises TRIS. In some embodiments, the pH regulating agent comprises disodium hydrogen phosphate dihydrate. In some embodiments, the pH regulating agent is TRIS. In some embodiments, the pH regulating agent is disodium hydrogen phosphate dihydrate.

If necessary, the pH of the formulation may also be adjusted by addition of a base or an acid, e.g. a strong base such as sodium hydroxide, or a strong acid, such as a hydrochloric acid, and optionally maintained at the desired pH by use of a suitable buffering agent, e.g. as mentioned herein above.

*The pharmaceutically acceptable preservative*

In some embodiments, the formulation comprises an API-preserving effective amount of a pharmaceutically acceptable preservative. In some embodiments, the formulation comprises a preservative selected from benzalkonium chloride and benzethonium chloride (IUPAC name: N-benzyl-N,N-dimethyl-2-{2-[4-(2,4,4-trimethylpentan-2-yl)phenoxy] ethoxy}ethanaminium chloride). In some embodiments, the preservative comprises is benzalkonium chloride. In some embodiments, the preservative comprises is benzalkonium chloride. Benzalkonium chloride (CAS No. 8001-54-5) is a mixture of alkylbenzyldimethylammonium chlorides of the general formula

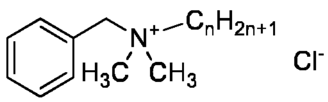

wherein n is an integer in the range of 8 to 16.

The concentration of preservative may typically range from about 0.01 g/l to about 0.2 g/l, from about 0.02 g/l to about 0.2 g/l, from about 0.05 g/l to about 0.2 g/l, or from about 0.1 g/l to about 0.2 g/l. In some embodiments, the concentration of preservative is at most 0.15 g/l. Thus, in some embodiments, the concentration of preservative is within the range from about 0.01 g/l to about 0.15 g/l, from about 0.02 g/l to about 0.15 g/l, from about 0.05 g/l to about 0.15 g/l, or from about 0.1 g/l to about 0.15 g/l.

In some embodiments, the formulation provided herein does not contain any preservative. For example, in some embodiments, when the formulation is provided in a single-use container (i.e. used at only one occasion), or a

single-dose container (i.e. containing only one dose), a preservative may be omitted. In some of these embodiments, the formulation contains no preservative.

There are also multidose containers that obviate the need for a preservative, such as the Novelia® PFMD bottle sold by Nemera (France), and similar devices, such as a multidose container as described e.g. in the article "OPHTHALMIC SQUEEZE DISPENSER - Eliminating the Need for Additives in Multidose Preservative-Free Eyecare Formulations" (Drug Development & Delivery, October 2017, Vol. 17, No. 7, pp. 40-44). Thus, in some further embodiments, the formulation is a preservative-free formulation provided in a multidose container suitable for dispensing preservative-free formulations to the eye.

*The pharmaceutically acceptable surfactant*

In some embodiments, the formulation additionally comprises a surfactant (surface active agent), preferably a nonionic surfactant. For example, the formulation may comprise a nonionic surfactant selected from a polysorbate, such as polysorbate 80, and a poloxamer, such as poloxamer 188 or poloxamer 407. In some embodiments, the formulation comprises polysorbate 80 as a surfactant.

In a formulation, e.g. a gel form formulation, as described herein, a suitable concentration of surfactant may range from e.g. 0.1 to 5 g/l, from 0.2 to 5 g/l, from 0.5 to 5 g/l, from 0.1 to 4 g/l, from 0.2 to 4 g/l, from 0.5 to 4 g/l, from 0.1 to 3 g/l, from 0.2 to 3 g/l, from 0.5 to 3 g/l; from 0.1 to 2 g/l, from 0.2 to 2 g/l or from 0.5 to 2 g/l. In some embodiments, in a formulation, e.g. a gel formulation, as described herein, a suitable concentration of surfactant is within the range of 0.1 to 1.5 g/l, 0.2 to 1.5 g/l, 0.5 to 1.5 g/l or 0.8 to 1.5 g/l; e.g. within the range of from 0.1 to 1.2 g/l, 0.2 to 1.2 g/l, 0.5 to 1.2 g/l, or 0.8 to 1.2 g/l. In some embodiments, a suitable concentration of surfactant in a gel formulation as described herein is within the range of from 0.9 to 1.1 g/l, e.g. about 1.0 g/l. In some of these embodiments, the surfactant is polysorbate 80.

In an emulsion form formulation as described herein, the concentration of surfactant will generally be higher than in a gel formulation, and a suitable concentration of surfactant may range from e.g. 5 to 50 g/l, from 10 to 50 g/l, from 15 to 50 g/l, from 5 to 45 g/l, from 10 to 45 g/l from 15 to 45 g/l, from 5 to 40 g/l, from 10 to 40 g/l, or from 15 to 40 g/l.

In some embodiments, the formulation provided herein does not contain any surfactant. In some embodiments, the formulation is a gel formulation that does not contain any surfactant.

*The pharmaceutically acceptable solubilizing agent*

In some embodiments, the formulation provided herein comprises a solubilizing effective amount of a pharmaceutically acceptable solubilizer (or solubilizing agent), such as macrogol 15 hydroxystearate, polyoxyl castor oil, polyvinylpyrrolidone or cyclodextrin (e.g. beta-cyclodextrin).

In some embodiments, the formulation contains cyclodextrin, e.g. beta-cyclodextrin, e.g. at a concentration in the range of from about 10 to about 100 g/l, e.g. from 20 to 100 g/l, or from 50 to 100 g/l, for example from 70 to 100 g/l, or from 80 to 100 g/l.

In some embodiments, the formulation contains macrogol 15 hydroxystearate, e.g. at a concentration in in the range of from about 0.5 to 5 g/l, e.g. from 1 to 4.5 g/l, or from 1.5 to 4 g/l, for example from 2 to 3 g/l.

In some embodiments, the formulation contains polyoxyl castor oil, e.g. at a concentration in the range of from about 10 to about 100 g/l, e.g. from 20 to 80 g/l, or from 30 to 70 g/l, for example from 40 to 60 g/l.

In some embodiments, the formulation contains polyoxyl castor oil, e.g. at a concentration in the range of from about 1 to about 30 g/l, e.g. from 2 to 25 g/l, or from 4 to 20 g/l, for example from 7 to 15 g/l.

In some embodiments, the formulation provided herein does not contain any solubilizing agent.

Finally, it is noted that that some constituents possess several functional attributes, and therefore may be present in the formulation of the invention in different capacities. For example, the person of ordinary skill in the art will know that carbomer copolymer (Type B) has been used in the field of pharmaceutical formulations as a thickening agent, as a gel-forming agent, as a stabilizer, and as an emulsifier. Likewise, polyoxyl castor oil has been applied as a nonionic solubilizer, but also as an oil-in-water emulsifier. Other components mentioned herein that have multiple applications are, for example macrogol 15 hydroxystearate (nonionic solubilizer, emulsifier), polyvinylpyrrolidone (solubilizer, viscosity enhancer) etc.

It is also noted that any functional agent as referred to herein (e.g. viscosity agent, tonicity agent, humectant etc) generally may comprise one or more compounds having the required functionality, i.e. unless otherwise specified or apparent from the context, the functional agent may consist of one such compound only, or may comprise a mixture of two or more such compounds.

In some embodiments, the formulation provided herein does not contain any of the optional components (vii) to (ix) or, if a gel formulation, does not contain any of the optional components (vii) to (x).

In some further embodiments, the formulation provided herein comprises:

(i) laquinimod or a pharmaceutically acceptable salt thereof as active ingredient, in a therapeutically effective amount;
(ii) a pharmaceutically acceptable viscosity agent, e.g. selected from a carbomer copolymer (e.g. of Type B) and/or a cellulose derivative such as sodium carboxymethyl cellulose, in an amount sufficient to provide a viscosity as defined herein, preferably a viscosity of about 2 to 50 mPas, about 2 to 40 mPas, about 2 to 30 mPas, about 10 to 50 mPas, about 10 to 40 mPas, about 10 to 30 mPas, about 10 to about 25 mPas, about 15 to 50 mPas, about 15 to 40 mPas, about 15 to 30 mPas, or about 15 to about 25 mPas;
(iii) a pharmaceutically acceptable tonicity adjusting agent, e.g a non-ionic tonicity adjusting agent such as mannitol, in an amount sufficient to provide an osmolality as indicated herein, preferably an osmolality of about 200 to 400 mOsm/kg, or about 250 to 350 mOsm/kg, or about 280 to 320 mOsm/kg;
(iv) a pharmaceutically acceptable humectant, e.g. a polyol, such as glycerol;
(v) a pharmaceutically acceptable antioxidant, e.g. Na EDTA; and
(vi) a pharmaceutically acceptable pH regulating agent, e.g. a basic buffer, such as TRIS or disodium hydrogen phosphate dihydrate, in an amount sufficient to provide a pH of at least 6.8, e.g. a pH of about 7 to 8.4, e.g. about 7.4 to about 8.4, about 7.4 to 8.0, or about 8.0 to 8.4; and optionally one or more components selected from:
(vii) a pharmaceutically acceptable preservative, e.g. benzalkonium chloride;
(viii) a pharmaceutically acceptable surfactant, e.g. a polysorbate, such as polysorbate 80;
(ix) a pharmaceutically acceptable solubilizer, e.g. macrogol 15 hydroxystearate; and
(x) a pharmaceutically acceptable oil, e.g. a vegetable oil, such as castor oil.

In some embodiments, the formulation for ocular administration comprises:

(i) laquinimod or a pharmaceutically acceptable salt thereof as active ingredient, in a therapeutically effective amount;
(ii) a pharmaceutically acceptable viscosity agent, in an amount sufficient to provide a dynamic viscosity of 10 to 45 mPas, e.g 15 to 45 mPas, as measured at 20 °C;
(iii) a pharmaceutically acceptable tonicity adjusting agent, in an amount sufficient to provide an osmolality of 200 to 400 mOsm/kg, e.g. 250 to 375 mOsm/kg;
(iv) a pharmaceutically acceptable humectant;
(v) a pharmaceutically acceptable antioxidant; and
(vi) a pharmaceutically acceptable pH regulating agent in an amount sufficient to provide a pH of 6.8 to 8.0.

In some embodiments, the formulation for ocular administration comprises:

(i) laquinimod or a pharmaceutically acceptable salt thereof as active ingredient, in a therapeutically effective amount;
(ii) a pharmaceutically acceptable viscosity agent, in an amount sufficient to provide a dynamic viscosity of 10 to 30 mPas, as measured at 20 °C;
(iii) a pharmaceutically acceptable tonicity adjusting agent, in an amount sufficient to provide an osmolality of 200 to 400 mOsm/kg;
(iv) glycerol;
(v) a pharmaceutically acceptable antioxidant; and
(vi) a pharmaceutically acceptable pH regulating agent in an amount sufficient to provide a pH of 6.8 to 8.5, e.g. 7.4 to 8.4.

In some embodiments, the formulation provided herein has a viscosity of about 2 to 50 mPas, about 2 to 45 mPas, about 2 to 40 mPas, about 2 to 30 mPas, about 10 to 50 mPas, about 10 to 45 mPas, about 10 to 40 mPas, about 10 to 30 mPas, about 15 to 50 mPas, about 15 to 45 mPas, about 15 to 40 mPas, or about 15 to 30 mPas; an osmolality of about 200 to 400 mOsm/kg, about 250 to 375 mOsm/kg, about 250 to 350 mOsm/kg, or about 250 to 320 mOsm/kg; and a pH of about 6.8 to 8.5, about 6.8 to 8.4, about 6.8 to 8.0, about 7.0 to about 8.0, e.g about 7.4 to 8.0.
In some embodiments, the formulation provided herein has a viscosity of about about 15 to 45 mPas; an osmolality of about 250 to 375 mOsm/kg; and a pH of about 6.8 to about 8.0.
In some embodiments, the formulation provided herein has a viscosity of about 20 to 40 mPas; an osmolality of about 280 to 320 mOsm/kg; and a pH of about 7.0 to about 8.0, e.g. about 7.4.
In some embodiments, the formulation comprises:

(i) laquinimod or a pharmaceutically acceptable salt thereof as active ingredient, in a therapeutically effective amount;
(ii) a pharmaceutically acceptable viscosity agent, e.g. a viscosity agent as mentioned herein, in an amount sufficient to provide a viscosity as defined herein, preferably a viscosity of about 2 to 50 mPas, about 2 to 45 mPas, about 2 to 40 mPas, about 2 to 35 mPas, about 10 to 50 mPas, about 10 to 45 mPas, about 10 to 40 mPas, about 10 to 35 mPas, about 15 to 50 mPas, about 15 to 45 mPas, about 15 to 40 mPas, or about 15 to 35 mPas;

(iii) a pharmaceutically acceptable tonicity adjusting agent, e.g a tonicity agent as mentioned herein, in an amount sufficient to provide an osmolality as indicated herein, preferably an osmolality of about 200 to 400 mOsm/kg, about 250 to 350 mOsm/kg, about 250 to 375 mOsm/kg, or about 280 to 320 mOsm/kg;
(iv) a pharmaceutically acceptable humectant, e.g. a humectant as mentioned herein, e.g. glycerol;
(v) a pharmaceutically acceptable antioxidant, e.g. an antioxidant as mentioned herein, e.g. Na-EDTA; and
(vi) a pharmaceutically acceptable pH regulating agent, e.g. a pH regulating agent as mentioned herein, in an amount sufficient to provide a pH of at least 6.8, e.g. a pH of about 6.8 to 8.5, or about 6.8 to 8.0, or about 7.0 to 8.0, e.g. about 7.4; and optionally one or more components selected from:
(vii) a pharmaceutically acceptable preservative;
(viii) a pharmaceutically acceptable surfactant;
(ix) a pharmaceutically acceptable solubilizer; and
(x) a pharmaceutically acceptable oil.

The formulation provided herein is either a gel formulation or a water and oil containing emulsion formulation. In some embodiments, the water and oil containing emulsion formulation is an oil-in-water emulsion. The water present in the formulation should be suitable for pharmaceutical use, such as distilled water, purified water, or water for injection

*Water and oil containing emulsion*

In some embodiments, the water and oil containing emulsion formulation, e.g. an oil-in-water emulsion.
In a water and oil containing emulsion formulation, the oil is a pharmaceutically acceptable oil, e.g. an oil selected from vegetable oils, such as castor oil, maize oil, olive oil or camelina oil. In some embodiments, the oil is castor oil. Depending on whether the oil forms the outer phase or the inner phase, the proportion of the oil and the water phase will vary, as readily understood by the person of ordinary skill in the art. Thus, in a water-in-oil emulsion, the volume of oil will normally be higher than the volume of the aqueous phase, and vice versa for an oil-in water emulsion. It is considered that in an emulsion the inner phase (e.g. the oil phase) may be present in an amount of e.g. 10-100 g/l, or from 20 to 80 g/l, e.g. from 30 to 70 g/l, such as about 40 to 60 g/l.
In some embodiments, the formulation provided herein is a water and oil containing emulsion, preferably an oil-in-water emulsion, comprising:

- laquinimod or a pharmaceutically acceptable salt thereof;
- a viscosity agent, such as a carbomer copolymer and/or sodium carboxymethylcellulose at a concentration sufficient to provide a viscosity within the range indicated herein;
- a tonicity agent, such as mannitol;
- a humectant, such as glycerol;
- an antioxidant, such as Na-EDTA;
- a pH buffering agent providing a pH in the range of from 6.8 to 8.5, or from 7.0 to 8.5, or from 7.4 to 8.4, or from 8.0 to 8.4, e.g. a pH buffering agent selected from disodium hydrogen phosphate dihydrate and TRIS;
- optionally a preservative, such as benzalkonium chloride;
- optionally a surfactant such as a polysorbate (e.g. polysorbate 80) and/or a poloxamer (e.g. poloxamer 407);
- optionally a solubilizer (or emulsifier), such as a macrogol 15 hydroxystearate, or a polyoxyl castor oil; and
- a vegetable oil, such as castor oil.

In some embodiments, the water and oil containing emulsion (preferably oil-in-water emulsion) comprises:

- laquinimod or a pharmaceutically acceptable salt thereof;
- a viscosity agent, such as a carbomer copolymer and/or sodium carboxymethylcellulose at a concentration sufficient to provide a viscosity within the range indicated herein;
- a tonicity agent, such as mannitol;
- a humectant, such as glycerol;
- an antioxidant, such as Na-EDTA;
- a pH buffering agent providing a pH in the range of from 6.8 to 8.5, or from 7.0 to 8.5, or from 7.4 to 8.4, or from 8.0 to 8.4, e.g. a pH buffering agent selected from disodium hydrogen phosphate dihydrate and TRIS;
- a preservative, such as benzalkonium chloride;
- a surfactant such as a polysorbate (e.g. polysorbate 80) and/or a poloxamer (e.g. poloxamer 407);
- a solubilizer (or emulsifier), such as a macrogol 15 hydroxystearate, or a polyoxyl castor oil; and
- a vegetable oil, such as castor oil.

In some embodiments, the water and oil containing emulsion (preferably oil-in-water emulsion) comprises:

- laquinimod or a pharmaceutically acceptable salt thereof in a therapeutically effective amount, e.g. about 5 to about 100 g/l, about 10 to about 100 g/l, about 20 to 80 g/l, or about 50 g/l, of laquinimod or a corresponding amount of a pharmaceutically acceptable salt thereof;
- about 2 to about 10 g/l, e.g. about 3 to about 8 g/l, or about 4 to about 6 g/l of sodium carboxymethylcellulose and/or about 0.2 to 1 g/l, e.g. about 0.3 to about 0.8, such as about 0.5 g/l of a carbomer copolymer (e.g. carbomer polymer Type B);
- about 1.5 to 4 g/l, e.g. about 2.5 to 3 g/l, such as about 2.7 g/l, of mannitol;
- about 10 to 40 g/l, e.g. about 20 to about 30 g/l, such as about 25 g/l, of glycerol;
- about 0.2 to 2 g/l, e.g. about 0.5 to 1.5 g/l, or about 0.8 to 1.2 g/l, of Na-EDTA;
- a pH buffering agent in an amount effective to provide a pH of from 7.0 to 8.5, e.g. a pH of from 7.4 to 8.4, or a pH of from 8.0 to 8.4, e.g. a pH of about 8.4, e.g. a pH buffering agent selected from disodium hydrogen phosphate dihydrate and TRIS (e.g. TRIS in an amount of about 1-1.5 g/l);
- optionally about 0.05 to about 0.2 g/l, e.g. about 0.1 to about 0.15 g/l of benzalkonium chloride;
- optionally about 10 to about 50 g/l, e.g. about 20 to about 40 g/l, of a surfactant, preferably a nonionic surfactant, such as a polysorbate and/or a poloxamer, e.g. polysorbate 80 and/or poloxamer 407;
- optionally about 1 to about 5 g/l, e.g. about 1.5 to about 3.5 g/l, such as about 2.5 g/l of macrogol 15 hydroxystearate, or about 30 to about 70 g/l, e.g. about 40 to about 60 g/l, such as about 50 g/l of polyoxyl castor oil (e.g. polyoxyl 35 castor oil); and
- about 20 to about 100 g/l, e.g. about 30 to about 70 g/l, such as about 50 g/l of a vegetable oil, such as castor oil.

In some embodiments, the water and oil containing emulsion (preferably oil-in-water emulsion), comprises:

- laquinimod or a pharmaceutically acceptable salt thereof in a therapeutically effective amount, e.g. e.g. about 5 to about 100 g/l, about 10 to about 100 g/l, about 20 to 80 g/l, or about 50 g/l, of laquinimod or a corresponding amount of a pharmaceutically acceptable salt thereof;
- about 2 to about 10 g/l, e.g. about 3 to about 8 g/l, or about 4 to about 6 g/l of sodium carboxymethylcellulose and/or about 0.2 to 1 g/l, e.g. about 0.3 to about 0.8, such as about 0.5 g/l of a carbomer copolymer (e.g. carbomer polymer Type B);
- about 1.5 to 4 g/l, e.g. about 2.5 to 3 g/l, such as about 2.7 g/l, of mannitol;
- about 10 to 40 g/l, e.g. about 20 to about 30 g/l, such as about 25 g/l, of glycerol;
- about 0.2 to 2 g/l, e.g. about 0.5 to 1.5 g/l, or about 0.8 to 1.2 g/l, of Na-EDTA;
- a pH buffering agent in an amount effective to provide a pH of from 7.0 to 8.5, e.g. a pH of from 7.4 to 8.4, or a pH of from 8.0 to 8.4, e.g. a pH of about 8.4, e.g. a pH buffering agent selected from disodium hydrogen phosphate dihydrate and TRIS (e.g. TRIS in an amount of about 1-1.5 g/l);
- about 0.05 to about 0.2 g/l, e.g. about 0.1 to about 0.15 g/l of benzalkonium chloride;
- about 10 to about 50 g/l, e.g. about 20 to about 40 g/l, of a surfactant, preferably a nonionic surfactant, such as a polysorbate and/or a poloxamer, e.g. polysorbate 80 and/or poloxamer 407;
- about 1 to about 5 g/l, e.g. about 1.5 to about 3.5 g/l, such as about 2.5 g/l of macrogol 15 hydroxystearate, or about 30 to about 70 g/l, e.g. about 40 to about 60 g/l, such as about 50 g/l of polyoxyl castor oil (e.g. polyoxyl 35 castor oil); and
- about 20 to about 100 g/l, e.g. about 30 to about 70 g/l, such as about 50 g/l of a vegetable oil, such as castor oil.

Some further emulsion formulations provided herein are as indicated in TABLES 1 to 12.

TABLE 1

| **Ingredient** | **g/l** |
|---|---|
| laquinimod | 40-60 |
| carbomer copolymer (Type B) | 0.3-0.7 |
| polysorbate 80 | 30-60 |
| mannitol | 2.5-3 |
| glycerol | 20-30 |
| Na-EDTA | 0.5-1.5 |

(continued)

| Ingredient | g/l |
|---|---|
| benzalkonium chloride | 0.1-0.15 |
| castor oil | 40-60 |
| a buffering agent providing a pH of about 7.4, e.g. disodium hydrogen phosphate dihydrate | |
| purified water | |

TABLE 2

| Ingredient | g/l |
|---|---|
| laquinimod | 40-60 |
| carbomer copolymer (Type B) | 0.3-0.7 |
| polysorbate 80 | 30-60 |
| mannitol | 2.5-3 |
| glycerol | 20-30 |
| Na-EDTA | 0.5-1.5 |
| benzalkonium chloride | 0.1-0.15 |
| castor oil | 40-60 |
| a buffering agent providing a pH of about 8.4, e.g. TRIS | |
| purified water | |

TABLE 3

| Ingredient | g/l |
|---|---|
| laquinimod | 40-60 |
| sodium carboxymethlycellulose | 3-7 |
| carbomer copolymer (Type B) | 0.3-0.7 |
| polysorbate 80 | 30-50 |
| mannitol | 2.5-3 |
| glycerol | 20-30 |
| Na-EDTA | 0.5-1.5 |
| benzalkonium chloride | 0.1-0.15 |
| castor oil | 40-60 |
| a buffering agent providing a pH of about 8.4, e.g. TRIS | |
| purified water | |

TABLE 4

| Ingredient | g/l |
|---|---|
| laquinimod | 40-60 |
| sodium carboxymethlycellulose | 3-6 |
| macrogol 15 hydroxystearate) | 2-3 |

(continued)

| Ingredient | g/l |
|---|---|
| polysorbate 80 | 20-30 |
| poloxamer 407 | 10-20 |
| mannitol | 2.5-3 |
| glycerol | 20-30 |
| Na-EDTA | 0.5-1.5 |
| benzalkonium chloride | 0.1-0.15 |
| castor oil | 40-60 |
| a buffering agent providing a pH of about 8.4, e.g. TRIS | |
| purified water | |

TABLE 5

| Ingredient | g/l |
|---|---|
| laquinimod | 40-60 |
| sodium carboxymethlycellulose | 3-6 |
| polyoxyl castor oil | 30-70 |
| polysorbate 80 | 20-30 |
| poloxamer 407 | 10-20 |
| mannitol | 2.5-3 |
| glycerol | 20-30 |
| Na-EDTA | 0.5-1.5 |
| benzalkonium chloride | 0.1-0.15 |
| castor oil | 40-60 |
| a buffering agent providing a pH of about 8.4, e.g. TRIS | |
| purified water | |

TABLE 6

| Ingredient | g/l |
|---|---|
| laquinimod | 40-60 |
| sodium carboxymethlycellulose | 3-6 |
| polyoxyl castor oil | 30-70 |
| poloxamer 407 | 15-25 |
| mannitol | 2.5-3 |
| glycerol | 20-30 |
| Na-EDTA | 0.5-1.5 |
| benzalkonium chloride | 0.1-0.15 |
| castor oil | 40-60 |
| a buffering agent providing a pH of about 8.4, e.g. TRIS | |

(continued)

| Ingredient | g/l |
|---|---|
| purified water | |

TABLE 7

| Ingredient | g/l |
|---|---|
| laquinimod | 10-15 |
| carbomer copolymer (Type B) | 0.3-0.7 |
| polysorbate 80 | 30-60 |
| mannitol | 2.5-3 |
| glycerol | 15-20 |
| Na-EDTA | 0.5-1.5 |
| benzalkonium chloride | 0.1-0.15 |
| castor oil | 40-60 |
| a buffering agent providing a pH of about 7.4, e.g. disodium hydrogen phosphate dihydrate | |
| purified water | |

TABLE 8

| Ingredient | g/l |
|---|---|
| laquinimod | 10-15 |
| carbomer copolymer (Type B) | 0.3-0.7 |
| polysorbate 80 | 30-60 |
| mannitol | 2.5-3 |
| glycerol | 15-20 |
| Na-EDTA | 0.5-1.5 |
| benzalkonium chloride | 0.1-0.15 |
| castor oil | 40-60 |
| a buffering agent providing a pH of about 8.4, e.g. TRIS | |
| purified water | |

TABLE 9

| Ingredient | g/l |
|---|---|
| laquinimod | 10-15 |
| sodium carboxymethlycellulose | 2-4 |
| carbomer copolymer (Type B) | 0.3-0.7 |
| polysorbate 80 | 30-50 |
| mannitol | 2.5-3 |
| glycerol | 15-20 |
| Na-EDTA | 0.5-1.5 |

(continued)

| Ingredient | g/l |
|---|---|
| benzalkonium chloride | 0.1-0.15 |
| castor oil | 40-60 |
| a buffering agent providing a pH of about 8.4, e.g. TRIS | |
| purified water | |

TABLE 10

| Ingredient | g/l |
|---|---|
| laquinimod | 10-15 |
| sodium carboxymethlycellulose | 2-4 |
| macrogol 15 hydroxystearate) | 2-3 |
| polysorbate 80 | 20-30 |
| poloxamer 407 | 10-20 |
| mannitol | 2.5-3 |
| glycerol | 15-20 |
| Na-EDTA | 0.5-1.5 |
| benzalkonium chloride | 0.1-0.15 |
| castor oil | 40-60 |
| a buffering agent providing a pH of about 8.4, e.g. TRIS | |
| purified water | |

TABLE 11

| Ingredient | g/l |
|---|---|
| laquinimod | 10-15 |
| sodium carboxymethlycellulose | 2-4 |
| polyoxyl castor oil | 30-70 |
| polysorbate 80 | 20-30 |
| poloxamer 407 | 10-20 |
| mannitol | 2.5-3 |
| glycerol | 15-20 |
| Na-EDTA | 0.5-1.5 |
| benzalkonium chloride | 0.1-0.15 |
| castor oil | 40-60 |
| a buffering agent providing a pH of about 8.4, e.g. TRIS | |
| purified water | |

TABLE 12

| Ingredient | g/l |
|---|---|
| laquinimod | 10-15 |
| sodium carboxymethlycellulose | 2-4 |
| polyoxyl castor oil | 30-70 |
| poloxamer 407 | 15-25 |
| mannitol | 2.5-3 |
| glycerol | 15-20 |
| Na-EDTA | 0.5-1.5 |
| benzalkonium chloride | 0.1-0.15 |
| castor oil | 40-60 |
| a buffering agent providing a pH of about 8.4, e.g. TRIS | |
| purified water | |

*Gel formulation*

In some embodiments, the formulation is a gel formulation. In some embodiments, the gel formulation comprises:

- laquinimod or a pharmaceutically acceptable salt thereof;
- a viscosity agent, such as a carbomer copolymer, hydroxypropyl methylcellulose, polyvinylalcohol, and/or sodium carboxymethylcellulose at a concentration sufficient to provide a viscosity within the range indicated herein;
- a tonicity agent, such as mannitol;
- a humectant, such as glycerol;
- an antioxidant, such as Na-EDTA;
- a pH buffering agent providing a pH in the range of from 6.8 to 8.5, from 7.0 to 8.5, from 7.4 to 8.4, from 7.4 to 8.0, or from 8.0 to 8.4, e.g. a pH buffering agent selected from disodium hydrogen phosphate dihydrate and TRIS;
- optionally a preservative, such as benzalkonium chloride;
- optionally a surfactant such as a polysorbate (e.g. polysorbate 80) and/or a poloxamer (e.g. poloxamer 188, or poloxamer 407); and
- optionally a solubilizer, such as a macrogol 15 hydroxystearate, polyvinyl pyrrolidone.

In some embodiments, the gel formulation comprises:

- laquinimod or a pharmaceutically acceptable salt thereof;
- a viscosity agent, such as a carbomer copolymer, hydroxypropyl methylcellulose, polyvinylalcohol, and/or sodium carboxymethylcellulose at a concentration sufficient to provide a viscosity within the range indicated herein;
- a tonicity agent, such as mannitol;
- a humectant, such as glycerol;
- an antioxidant, such as Na-EDTA;
- a preservative, such as benzalkonium chloride;
- a pH buffering agent providing a pH in the range of from 6.8 to 8.5, from 7.0 to 8.5, from 7.4 to 8.4, from 7.4 to 8.0, or from 8.0 to 8.4, e.g. a pH buffering agent selected from disodium hydrogen phosphate dihydrate and TRIS;
- a surfactant such as a polysorbate (e.g. polysorbate 80) and/or a poloxamer (e.g. poloxamer 188, or poloxamer 407); and
- optionally a solubilizer, such as a macrogol 15 hydroxystearate, polyvinyl pyrrolidone, or a polyoxyl castor oil.

In some embodiments, the gel formulation comprises:

- laquinimod or a pharmaceutically acceptable salt thereof in a therapeutically effective amount, e.g. about 10 to about 100 g/l, about 20 to 80 g/l, or about 50 g/l, of laquinimod or a corresponding amount of a pharmaceutically acceptable salt thereof;
- about 1 to about 10 g/l, e.g. about 2 to about 8 g/l, or about 3 to about 5 g/l of sodium carboxymethylcellulose, and/or

about 0.5 to about 6 g/l, e.g. about 1 to about 5 g/l, such as about 1.5 to about 3 g/l of a carbomer copolymer (e.g. carbomer polymer Type B), or about 2 to about 10 g/l, e.g. about 3 to about 7 g/l, such as about 5 g/l, of hydroxypropyl methyl cellulose, or about 20 to about 60 g/l, e.g. about 30 to about 50 g/l, such as about 40 g/l of polyvinyl alcohol;
- about 1.5 to 4 g/l, e.g. about 2.5 to 3 g/l, such as about 2.7 g/l, of mannitol;
- about 10 to 40 g/l, e.g. about 20 to about 30 g/l, such as about 25 g/l, of glycerol;
- about 0.2 to 2 g/l, e.g. about 0.5 to 1.5 g/l, or about 0.8 to 1.2 g/l, of Na-EDTA;
- a pH buffering agent in an amount effective to provide a pH of from 6.8 to 8.5, or from 7.0 to 8.5, e.g. a pH of from 7.4 to 8.4, a pH of from 7.4 to 8.0, or a pH of from 8.0 to 8.4, e.g. a pH of about 8.0, e.g. a pH buffering agent selected from disodium hydrogen phosphate dihydrate and TRIS (e.g. TRIS in an amount of about 1-1.5 g/l);
- optionally about 0.05 to about 0.2 g/l, e.g. about 0.1 to about 0.15 g/l of benzalkonium chloride;
- optionally about 0.1 to about 5 g/l, e.g. about 0.2 to about 3 g/l, or about 0.5 to about 2 g/l, of a surfactant, preferably a nonionic surfactant, such as a polysorbate and/or a poloxamer, e.g. polysorbate 80 and/or poloxamer 407 or poloxamer 188; and
- optionally about 1 to about 5 g/l, e.g. about 1.5 to about 3.5 g/l, such as about 2.5 g/l of macrogol 15 hydroxystearate, or about 30 to about 70 g/l, e.g. about 40 to about 60 g/l, such as about 50 g/l of polyoxyl castor oil (e.g. polyoxyl 35 castor oil), or about 5 to about 20 g/l, e.g. about 8 to about 15 g/l such as about 10 g/l of polyvinylpyrrolidone.

In some embodiments, the gel formulation comprises:

- laquinimod or a pharmaceutically acceptable salt thereof in a therapeutically effective amount, e.g. about 10 to about 100 g/l, about 20 to 80 g/l, or about 50 g/l, of laquinimod or a corresponding amount of a pharmaceutically acceptable salt thereof;
- about 1 to about 10 g/l, e.g. about 2 to about 8 g/l, or about 3 to about 5 g/l of sodium carboxymethylcellulose, and/or about 0.5 to about 6 g/l, e.g. about 1 to about 5 g/l, such as about 1.5 to about 3 g/l of a carbomer copolymer (e.g. carbomer polymer Type B), or about 2 to about 10 g/l, e.g. about 3 to about 7 g/l, such as about 5 g/l, of hydroxypropyl methyl cellulose, or about 20 to about 60 g/l, e.g. about 30 to about 50 g/l, such as about 40 g/l of polyvinyl alcohol;

- about 1.5 to 4 g/l, e.g. about 2.5 to 3 g/l, such as about 2.7 g/l, of mannitol;
- about 10 to 40 g/l, e.g. about 20 to about 30 g/l, such as about 25 g/l, of glycerol;
- about 0.2 to 2 g/l, e.g. about 0.5 to 1.5 g/l, or about 0.8 to 1.2 g/l, of Na-EDTA;
- a pH buffering agent in an amount effective to provide a pH of from 6.8 to 8.5, or from 7.0 to 8.5, e.g. a pH of from 7.4 to 8.4, a pH of from 7.4 to 8.0, or a pH of from 8.0 to 8.4, e.g. a pH of about 8.0, e.g. a pH buffering agent selected from disodium hydrogen phosphate dihydrate and TRIS (e.g. TRIS in an amount of about 1-1.5 g/l);
- about 0.05 to about 0.2 g/l, e.g. about 0.1 to about 0.15 g/l of benzalkonium chloride;
- optionally about 0.1 to about 5 g/l, e.g. about 0.2 to about 3 g/l, or about 0.5 to about 2 g/l, of a surfactant, preferably a nonionic surfactant, such as a polysorbate and/or a poloxamer, e.g. polysorbate 80 and/or poloxamer 407 or poloxamer 188; and
- optionally about 1 to about 5 g/l, e.g. about 1.5 to about 3.5 g/l, such as about 2.5 g/l of macrogol 15 hydroxystearate, or about 30 to about 70 g/l, e.g. about 40 to about 60 g/l, such as about 50 g/l of polyoxyl castor oil (e.g. polyoxyl 35 castor oil), or about 5 to about 20 g/l, e.g. about 8 to about 15 g/l such as about 10 g/l of polyvinylpyrrolidone.

In some embodiments, the gel formulation comprises:

- laquinimod or a pharmaceutically acceptable salt thereof in a therapeutically effective amount;
- sodium carboxymethylcellulose, and/or a carbomer copolymer (e.g. carbomer polymer Type B);
- a tonicity agent;
- a humectant;
- an antioxidant;
- a pH buffering agent in an amount effective to provide a pH of from 6.8 to 8.5, or from 7.0 to 8.5, e.g. a pH of from 7.4 to 8.4, a pH of from 7.4 to 8.0, or a pH of from 8.0 to 8.4, e.g. a pH of about 8.0;
- a preservative;
- optionally a surfactant, e.g. a nonionic surfactant; and
- optionally macrogol 15 hydroxystearate, polyoxyl castor oil (e.g. polyoxyl 35 castor oil), or polyvinylpyrrolidone.

In some embodiments, the gel formulation comprises:

- laquinimod or a pharmaceutically acceptable salt thereof in a therapeutically effective amount, e.g. about 10 to about 100 g/l, about 20 to 80 g/l, or about 50 g/l, of laquinimod or a corresponding amount of a pharmaceutically acceptable

salt thereof;

- about 1 to about 10 g/l, e.g. about 2 to about 8 g/l, or about 3 to about 5 g/l of sodium carboxymethylcellulose, and/or about 0.5 to about 6 g/l, e.g. about 1 to about 5 g/l, such as about 1.5 to about 3 g/l of a carbomer copolymer (e.g. carbomer polymer Type B);
- a tonicity agent;
- a humectant;
- an antioxidant;
- a pH buffering agent in an amount effective to provide a pH of from 6.8 to 8.5, or from 7.0 to 8.5, e.g. a pH of from 7.4 to 8.4, a pH of from 7.4 to 8.0, or a pH of from 8.0 to 8.4, e.g. a pH of about 8.0;
- a preservative;
- optionally a surfactant, e.g. a nonionic surfactant; and
- optionally about 1 to about 5 g/l, e.g. about 1.5 to about 3.5 g/l, such as about 2.5 g/l of macrogol 15 hydroxystearate, or about 30 to about 70 g/l, e.g. about 40 to about 60 g/l, such as about 50 g/l of polyoxyl castor oil (e.g. polyoxyl 35 castor oil), or about 5 to about 20 g/l, e.g. about 8 to about 15 g/l such as about 10 g/l of polyvinylpyrrolidone.

In some embodiments, the gel formulation comprises:

- laquinimod or a pharmaceutically acceptable salt thereof in a therapeutically effective amount, e.g. about 10 to 100 g/l;
- about 1 to about 10 g/l, and/or about 0.5 to about 6 g/l, of a carbomer copolymer (e.g. carbomer polymer Type B);
- a tonicity agent;
- a humectant;
- an antioxidant;
- a pH buffering agent in an amount effective to provide a pH of from 6.8 to 8.5, or from 7.0 to 8.5, e.g. a pH of from 7.4 to 8.4, a pH of from 7.4 to 8.0, or a pH of from 8.0 to 8.4, e.g. a pH of about 8.0;
- a preservative;
- optionally a surfactant, e.g. a nonionic surfactant; and
- optionally about 1 to about 5 g/l of macrogol 15 hydroxystearate, or about 30 to about 70 g/l of polyoxyl castor oil (e.g. polyoxyl 35 castor oil), or about 5 to about 20 g/l of polyvinylpyrrolidone.

In some embodiments, the gel formulation comprises:

- laquinimod or a pharmaceutically acceptable salt thereof in a therapeutically effective amount, e.g. about 10 to 100 g/l;
- about 1 to about 10 g/l, and/or about 0.5 to about 6 g/l, of a carbomer copolymer (e.g. carbomer polymer Type B);
- about 1.5 to 4 g/l of mannitol;
- about 10 to 40 g/l of glycerol;
- about 0.2 to 2 g/l of Na-EDTA;
- a pH buffering agent in an amount effective to provide a pH of from 6.8 to 8.5, or from 7.0 to 8.5, e.g. a pH of from 7.4 to 8.4, a pH of from 7.4 to 8.0, or a pH of from 8.0 to 8.4, e.g. a pH of about 8.0;
- about 0.05 to about 0.2 g/l of benzalkonium chloride;
- optionally a surfactant, e.g. a nonionic surfactant, e.g. about 0.1 to about 5 g/l of a surfactant, preferably a nonionic surfactant; and
- optionally about 1 to about 5 g/l of macrogol 15 hydroxystearate, or about 30 to about 70 g/l of polyoxyl castor oil (e.g. polyoxyl 35 castor oil), or about 5 to about 20 g/l of polyvinylpyrrolidone.

In some embodiments, the gel formulation comprises:

- laquinimod or a pharmaceutically acceptable salt thereof in a therapeutically effective amount, e.g. about 10 to about 100 g/l, about 20 to 80 g/l, or about 50 g/l, of laquinimod or a corresponding amount of a pharmaceutically acceptable salt thereof;
- about 1 to about 10 g/l, e.g. about 2 to about 8 g/l, or about 3 to about 5 g/l of sodium carboxymethylcellulose, and/or about 0.5 to about 6 g/l, e.g. about 1 to about 5 g/l, such as about 1.5 to about 3 g/l of a carbomer copolymer (e.g. carbomer polymer Type B);
- about 1.5 to 4 g/l, e.g. about 2.5 to 3 g/l, such as about 2.7 g/l, of mannitol;
- about 10 to 40 g/l, e.g. about 20 to about 30 g/l, such as about 25 g/l, of glycerol;
- about 0.2 to 2 g/l, e.g. about 0.5 to 1.5 g/l, or about 0.8 to 1.2 g/l, of Na-EDTA;
- a pH buffering agent in an amount effective to provide a pH of from 6.8 to 8.5, or from 7.0 to 8.5, e.g. a pH of from 7.4 to 8.4, a pH of from 7.4 to 8.0, or a pH of from 8.0 to 8.4, e.g. a pH of about 8.0, e.g. a pH buffering agent selected from disodium hydrogen phosphate dihydrate and TRIS (e.g. TRIS in an amount of about 1-1.5 g/l);

- about 0.05 to about 0.2 g/l, e.g. about 0.1 to about 0.15 g/l of benzalkonium chloride;
- optionally a surfactant, e.g. a nonionic surfactant, e.g. about 0.1 to about 5 g/l, e.g. about 0.2 to about 3 g/l, or about 0.5 to about 2 g/l, of a surfactant, preferably a nonionic surfactant, such as a polysorbate and/or a poloxamer, e.g. polysorbate 80 or poloxamer 407; and
- about 1 to about 5 g/l, e.g. about 1.5 to about 3.5 g/l, such as about 2.5 g/l of macrogol 15 hydroxystearate, or about 30 to about 70 g/l, e.g. about 40 to about 60 g/l, such as about 50 g/l of polyoxyl castor oil (e.g. polyoxyl 35 castor oil), or about 5 to about 20 g/l, e.g. about 8 to about 15 g/l such as about 10 g/l of polyvinylpyrrolidone.

In some embodiments, the gel formulation comprises:

- laquinimod, or a pharmaceutically acceptable salt thereof in a therapeutically effective amount, e.g. about 10 to about 100 g/l, about 20 to 80 g/l, or about 20 to about 50 g/l, of laquinimod or a corresponding amount of a pharmaceutically acceptable salt thereof;
- about 1 to about 10 g/l, e.g. about 2 to about 8 g/l, or about 3 to about 5 g/l of sodium carboxymethylcellulose, and/or about 0.5 to about 6 g/l, e.g. about 1 to about 5 g/l, such as about 1.5 to about 3 g/l of a carbomer copolymer (e.g. carbomer polymer Type B);
- about 1.5 to 4 g/l, e.g. about 2.5 to 3 g/l, such as about 2.7 g/l, of mannitol;
- about 10 to 40 g/l, e.g. about 20 to about 30 g/l, such as about 25 g/l, of glycerol;
- about 0.2 to 2 g/l, e.g. about 0.5 to 1.5 g/l, or about 0.8 to 1.2 g/l, of Na-EDTA;
- a pH buffering agent in an amount effective to provide a pH of from 6.8 to 8.5, or from 7.0 to 8.5, e.g. a pH of from 7.4 to 8.4, a pH of from 7.4 to 8.0, or a pH of from 8.0 to 8.4, e.g. a pH of about 8.0, e.g. a pH buffering agent selected from disodium hydrogen phosphate dihydrate and TRIS (e.g. TRIS in an amount of about 1-1.5 g/l, or disodium hydrogen phosphate dihydrate in an amount of about 1.5 to 2 g/l);

and optionally one or more components selected from:

- a pharmaceutically acceptable preservative, e.g. about 0.05 to about 0.2 g/l, e.g. about 0.1 to about 0.15 g/l of benzalkonium chloride;
- a pharmaceutically acceptable surfactant, e.g. a nonionic surfactant, e.g. about 0.1 to about 5 g/l, e.g. about 0.2 to about 3 g/l, or about 0.5 to about 2 g/l, e.g. about 1 g/l of a surfactant, preferably a nonionic surfactant, such as a polysorbate, e.g. polysorbate 80; and
- a pharmaceutically acceptable solubilizer, e.g. about 1 to about 5 g/l, e.g. about 1.5 to about 3.5 g/l, such as about 2.5 g/l of macrogol 15 hydroxystearate.

In some embodiments, the gel formulation comprises:

- laquinimod or a pharmaceutically acceptable salt thereof in a therapeutically effective amount, e.g. about 10 to about 100 g/l, about 20 to 80 g/l, or about 20 to about 50 g/l, of laquinimod, or a corresponding amount of a pharmaceutically acceptable salt thereof;
- about 1 to about 10 g/l, e.g. about 2 to about 8 g/l, or about 3 to about 5 g/l of sodium carboxymethylcellulose, and/or about 0.5 to about 6 g/l, e.g. about 1 to about 5 g/l, such as about 1.5 to about 3 g/l of a carbomer copolymer (e.g. carbomer polymer Type B);
- about 1.5 to 4 g/l, e.g. about 2.5 to 3 g/l, such as about 2.7 g/l, of mannitol;
- about 10 to 40 g/l, e.g. about 20 to about 30 g/l, such as about 25 g/l, of glycerol;
- about 0.2 to 2 g/l, e.g. about 0.5 to 1.5 g/l, or about 0.8 to 1.2 g/l, of Na-EDTA;
- a pH buffering agent in an amount effective to provide a pH of from 6.8 to 8.5, or from 7.0 to 8.5, e.g. a pH of from 7.4 to 8.4, a pH of from 7.4 to 8.0, or a pH of from 8.0 to 8.4, e.g. a pH of about 8.0, e.g. a pH buffering agent selected from disodium hydrogen phosphate dihydrate and TRIS (e.g. TRIS in an amount of about 1-1.5 g/l);

and optionally one or more components selected from:

- a pharmaceutically acceptable preservative, e.g. about 0.05 to about 0.2 g/l, e.g. about 0.1 to about 0.15 g/l of benzalkonium chloride; and
- a pharmaceutically acceptable solubilizer, e.g. about 1 to about 5 g/l, e.g. about 1.5 to about 3.5 g/l, such as about 2.5 g/l of macrogol 15 hydroxystearate.

In some embodiments, the gel formulation comprises:

- laquinimod or a pharmaceutically acceptable salt thereof in a therapeutically effective amount, e.g. about 10 to about 100 g/l, about 20 to 80 g/l, or about 20 to about 50 g/l, of laquinimod, or a corresponding amount of a pharmaceutically acceptable salt thereof;
- about 1 to about 10 g/l, e.g. about 2 to about 8 g/l, or about 3 to about 5 g/l of sodium carboxymethylcellulose, and/or about 0.5 to about 6 g/l, e.g. about 1 to about 5 g/l, such as about 1.5 to about 3 g/l of a carbomer copolymer (e.g. carbomer polymer Type B);
- about 1.5 to 4 g/l, e.g. about 2.5 to 3 g/l, such as about 2.7 g/l, of mannitol;
- about 10 to 40 g/l, e.g. about 20 to about 30 g/l, such as about 25 g/l, of glycerol;
- about 0.2 to 2 g/l, e.g. about 0.5 to 1.5 g/l, or about 0.8 to 1.2 g/l, of Na-EDTA;
- a pH buffering agent in an amount effective to provide a pH of from 6.8 to 8.5, or from 7.0 to 8.5, e.g. a pH of from 7.4 to 8.4, a pH of from 7.4 to 8.0, or a pH of from 8.0 to 8.4, e.g. a pH of about 8.0, e.g. a pH buffering agent selected from disodium hydrogen phosphate dihydrate and TRIS (e.g. TRIS in an amount of about 1-1.5 g/l);

and optionally one or more components selected from:

- a pharmaceutically acceptable preservative, e.g. about 0.05 to about 0.2 g/l, e.g. about 0.1 to about 0.15 g/l of benzalkonium chloride; and
- a pharmaceutically acceptable surfactant, e.g. a nonionic surfactant, e.g. about 0.1 to about 5 g/l, e.g. about 0.2 to about 3 g/l, or about 0.5 to about 2 g/l, e.g. about 1 g/l of a surfactant, preferably a nonionic surfactant, such as a polysorbate, e.g. polysorbate 80.

In some embodiments, the gel formulation comprises:

- laquinimod or a pharmaceutically acceptable salt thereof in a therapeutically effective amount, e.g. about 10 to about 100 g/l, about 10 to 80 g/l, about 10 to about 50 g/l, or about 10 to about 20 g/l, of laquinimod, or a corresponding amount of a pharmaceutically acceptable salt thereof, e.g. the sodium salt of laquinimod;
- about 1 to about 10 g/l, e.g. about 2 to about 8 g/l, or about 3 to about 5 g/l of sodium carboxymethylcellulose, and/or about 0.5 to about 6 g/l, e.g. about 1 to about 5 g/l, such as about 1.5 to about 3 g/l of a carbomer copolymer (e.g. carbomer polymer Type B);
- about 1.5 to 4 g/l, e.g. about 2.5 to 3 g/l, such as about 2.7 g/l, of mannitol;
- about 10 to 40 g/l, e.g. about 10 to about 30 g/l, or about 15 to about 20 g/l of glycerol;
- about 0.2 to 2 g/l, e.g. about 0.5 to 1.5 g/l, or about 0.8 to 1.2 g/l, of Na-EDTA;
- a pH buffering agent in an amount effective to provide a pH of from 6.8 to 8.5, or from 6.8 to 8.0, e.g. a pH of from 7.0 to 8.0, or a pH of from 7.4 to 8.0, e.g. a pH of about 7.4, e.g. a pH buffering agent selected from disodium hydrogen phosphate dihydrate and TRIS (e.g. TRIS in an amount of about 1-1.5 g/l, or disodium hydrogen phosphate dihydrate in an amount of about 1.5 to 2 g/l);

and optionally one or more components selected from:

- a pharmaceutically acceptable preservative, e.g. about 0.05 to about 0.2 g/l, e.g. about 0.1 to about 0.15 g/l of benzalkonium chloride; and
- a pharmaceutically acceptable surfactant, e.g. a nonionic surfactant, e.g. about 0.1 to about 5 g/l, e.g. about 0.2 to about 3 g/l, or about 0.5 to about 2 g/l, e.g. about 1 g/l of a surfactant, preferably a nonionic surfactant, such as a polysorbate, e.g. polysorbate 80.

In some embodiments, the gel formulation comprises:

- laquinimod or a pharmaceutically acceptable salt thereof in a therapeutically effective amount, e.g. about 10 to about 100 g/l, about 10 to 80 g/l, about 10 to about 50 g/l, or about 10 to about 20 g/l, of laquinimod, or a corresponding amount of a pharmaceutically acceptable salt thereof, e.g. the sodium salt of laquinimod;
- about 1 to about 10 g/l, e.g. about 2 to about 8 g/l, or about 3 to about 5 g/l of sodium carboxymethylcellulose, and about 0.5 to about 6 g/l, e.g. about 1 to about 5 g/l, such as about 1.5 to about 3 g/l of a carbomer copolymer (e.g. carbomer polymer Type B);
- about 1.5 to 4 g/l, e.g. about 2.5 to 3 g/l, such as about 2.7 g/l, of mannitol;
- about 10 to 40 g/l, e.g. about 10 to about 30 g/l, or about 15 to about 20 g/l of glycerol;
- about 0.2 to 2 g/l, e.g. about 0.5 to 1.5 g/l, or about 0.8 to 1.2 g/l, of Na-EDTA;
- a pH buffering agent in an amount effective to provide a pH of from 6.8 to 8.5, or from 6.8 to 8.0, e.g. a pH of from 7.0 to 8.0, or a pH of from 7.4 to 8.0, e.g. a pH of about 7.4, e.g. a pH buffering agent selected from disodium

hydrogen phosphate dihydrate and TRIS (e.g. TRIS in an amount of about 1-1.5 g/l, or disodium hydrogen phosphate dihydrate in an amount of about 1.5 to 2 g/l);

and optionally one or more components selected from:

- a pharmaceutically acceptable preservative, e.g. about 0.05 to about 0.2 g/l, e.g. about 0.1 to about 0.15 g/l of benzalkonium chloride; and
- a pharmaceutically acceptable surfactant, e.g. a nonionic surfactant, e.g. about 0.1 to about 5 g/l, e.g. about 0.2 to about 3 g/l, or about 0.5 to about 2 g/l, e.g. about 1 g/l of a surfactant, preferably a nonionic surfactant, such as a polysorbate, e.g. polysorbate 80.

In some embodiments, the gel formulation comprises:

- laquinimod or a pharmaceutically acceptable salt thereof in a therapeutically effective amount, e.g. about 10 to about 100 g/l, about 10 to 80 g/l, about 10 to about 50 g/l, or about 10 to about 20 g/l, of laquinimod, or a corresponding amount of a pharmaceutically acceptable salt thereof, e.g. the sodium salt of laquinimod;
- about 3 to about 5 g/l of sodium carboxymethylcellulose, and about 1.5 to about 3 g/l of a carbomer copolymer (e.g. carbomer polymer Type B);
- about 2.5 to 3 g/l of mannitol;
- about 15 to about 20 g/l of glycerol;
- about 0.8 to 1.2 g/l, of Na-EDTA;
- a pH buffering agent in an amount effective to provide a pH of from 6.8 to 8.0, e.g. a pH of about 7.4, (e.g. disodium hydrogen phosphate dihydrate in an amount of about 1.5 to 2 g/l); and preferably one or more (e.g. both) components selected from:
    - about 0.1 to about 0.15 g/l of benzalkonium chloride; and
    - about 0.5 to about 1.5 g/l of polysorbate 80.

In some embodiments, the gel formulation comprises:

(i) laquinimod or a pharmaceutically acceptable salt thereof as active ingredient, in a therapeutically effective amount;
(ii) a pharmaceutically acceptable viscosity agent, e.g. selected from a carbomer copolymer (e.g. of Type B) and/or a cellulose derivative such as sodium carboxymethyl cellulose, in an amount sufficient to provide a viscosity as defined herein, preferably a viscosity of about 2 to 50 mPas, about 2 to 40 mPas, about 2 to 30 mPas, about 10 to 50 mPas, about 10 to 40 mPas, about 10 to 30 mPas, about 10 to about 25 mPas, about 15 to 50 mPas, about 15 to 45 mPas, about 15 to 40 mPas, about 15 to 35 mPas, about 15 to 30 mPas, or about 15 to about 25 mPas;
(iii) a pharmaceutically acceptable tonicity adjusting agent, e.g a non-ionic tonicity adjusting agent such as mannitol, in an amount sufficient to provide an osmolality as indicated herein, preferably an osmolality of about 200 to 400 mOsm/kg, about 250 to 375 mOsm/kg, or about 250 to 350 mOsm/kg, or about 280 to 320 mOsm/kg;
(iv) a pharmaceutically acceptable humectant, e.g. a polyol, such as glycerol;
(v) a pharmaceutically acceptable antioxidant, e.g. Na-EDTA; and
(vi) a pharmaceutically acceptable pH regulating agent, e.g. a basic buffer, such as TRIS or disodium hydrogen phosphate dihydrate, in an amount sufficient to provide a pH of at least 6.8, e.g. a pH of about 6.8 to about 8.4, about 7.0 to about 8.4, e.g. about 7.4 to about 8.4, 6.8 to about 8.0, about 7.0 to about 8.0, about 7.4 to 8.0, or about 8.0 to 8.4; and optionally one or more components selected from:
(vii) a pharmaceutically acceptable preservative, e.g. benzalkonium chloride;
(viii) a pharmaceutically acceptable surfactant, e.g. a polysorbate, such as polysorbate 80; and
(ix) a pharmaceutically acceptable solubilizer, e.g. macrogol 15 hydroxystearate.

In some further embodiments, the gel formulation comprises:

(i) 10 to 100 g/l of laquinimod, or a corresponding amount of a pharmaceutically acceptable salt of laquinimod;
(ii) 1 to 10 g/l of sodium carboxymethylcellulose, and/or 0.5 to about 6 g/l of a carbomer copolymer;
(iii) 1.5 to 4 g/l of mannitol;
(iv) 10 to 40 g/l of glycerol;
(v) 0.2 to 2 g/l of Na-EDTA; and
(vi) a pH regulating agent in an amount effective to provide a pH of from 6.8 to 8.4, e.g. from 7.4 to 8.4.

In some embodiments, the gel formulation for ocular administration, e.g. topical ocular administration, comprises:

(i) 10 to 12 g/l of laquinimod, or a corresponding amount of a pharmaceutically acceptable salt of laquinimod, e.g. the sodium salt of laquinimod;
(ii) 2 to 4 g/l of sodium carboxymethylcellulose and 1 to 2 g/l of a carbomer copolymer;
(iii) 2 to 3 g/l of mannitol;
(iv) 15 to 20 g/l of glycerol;
(v) 0.5 to 1.5 g/l of Na-EDTA; and
(vi) a pH regulating agent in an amount effective to provide a pH in the range of 6.8 to 8.0, e.g. about 7.4.

In some embodiments, the gel formulation, in addition to components (i) to (vi), further comprises:

(vii) a pharmaceutically acceptable preservative; and
(viii) a pharmaceutically acceptable surfactant (surface active agent).

In some embodiments, the gel formulation comprises:

(i) 10 to 12 g/l of laquinimod, or a corresponding amount of a pharmaceutically acceptable salt of laquinimod, e.g. the sodium salt of laquinimod;
(ii) 2 to 4 g/l of sodium carboxymethylcellulose and 1 to 2 g/l of a carbomer copolymer;
(iii) 2 to 3 g/l of mannitol;
(iv) 15 to 20 g/l of glycerol;
(v) 0.5 to 1.5 g/l of Na-EDTA;
(vi) a pH regulating agent in an amount effective to provide a pH in the range of 6.8 to 8.0, e.g. about 7.4;
(vii) 0.05-0.1 g/l of benzalkonium chloride; and
(viii) 0.5-1.5 g/l of Polysorbate 80.

In some embodiments, the gel formulation provided herein does not contain any surfactant. In some embodiments, the gel formulation provided herein does not contain any solubilizing agent. In some embodiments, the gel formulation provided herein does not contain any preservative. In some embodiments, the gel formulation provided herein does not contain any of the optional components (vii) to (ix).

Some further gel formulations provided herein are as indicated in TABLES 13 to 37.

TABLE 13

| **Ingredient** | **g/l** |
|---|---|
| laquinimod | 10-15 |
| carbomer copolymer (Type B) | 1-2 |
| Sodium carboxymethylcellulose | 3-5 |
| polysorbate 80 | 0.5-1.5 |
| mannitol | 2.5-3 |
| glycerol | 15-20 |
| Na-EDTA | 0.5-1.5 |
| benzalkonium chloride | 0.05-0.15 |
| a buffering agent providing a pH of about 7.4, e.g. $Na_2HPO_4$ in an amount of 1.5-2.5 g/l | |
| purified water | |

TABLE 14

| **Ingredient** | **g/l** |
|---|---|
| laquinimod | 40-60 |

(continued)

| Ingredient | g/l |
|---|---|
| carbomer copolymer (Type B) | 1.5-2 |
| mannitol | 2.5-3 |
| glycerol | 20-30 |
| Na-EDTA | 0.5-1.5 |
| benzalkonium chloride | 0.1-0.15 |
| a basic buffering agent providing a pH of about 8.0, e.g. TRIS | |
| purified water | |

TABLE 15

| Ingredient | g/l |
|---|---|
| laquinimod | 10-15 |
| carbomer copolymer (Type B) | 1.5-2 |
| mannitol | 2.5-3 |
| glycerol | 15-20 |
| Na-EDTA | 0.5-1.5 |
| benzalkonium chloride | 0.1-0.15 |
| a basic buffering agent providing a pH of about 8.0, e.g. TRIS | |
| purified water | |

TABLE 16

| Ingredient | g/l |
|---|---|
| laquinimod | 10-15 |
| sodium carboxymethylcellulose | 2-4 |
| carbomer copolymer (Type B) | 1.5-2 |
| mannitol | 2.5-3 |
| glycerol | 15-20 |
| Na-EDTA | 0.5-1.5 |
| benzalkonium chloride | 0.1-0.15 |
| a basic buffering agent providing a pH of about 8.0, e.g. TRIS | |
| purified water | |

TABLE 17

| Ingredient | g/l |
|---|---|
| laquinimod | 10-15 |
| sodium carboxymethylcellulose | 3-5 |
| carbomer copolymer (Type B) | 2-4 |
| mannitol | 2.5-3 |

(continued)

| Ingredient | g/l |
|---|---|
| glycerol | 15-20 |
| Na-EDTA | 0.5-1.5 |
| benzalkonium chloride | 0.1-0.15 |
| a basic buffering agent providing a pH of about 8.0, e.g. TRIS | |
| purified water | |

TABLE 18

| Ingredient | g/l |
|---|---|
| laquinimod | 10-15 |
| sodium carboxymethylcellulose | 2-4 |
| macrogol 15 hydroxystearate | 2-3 |
| mannitol | 2.5-3 |
| glycerol | 15-20 |
| Na-EDTA | 0.5-1.5 |
| benzalkonium chloride | 0.1-0.15 |
| a basic buffering agent providing a pH of about 8.0, e.g. TRIS | |
| purified water | |

TABLE 19

| Ingredient | g/l |
|---|---|
| laquinimod | 10-15 |
| sodium carboxymethylcellulose | 3-5 |
| polyoxyl castor oil | 40-60 |
| mannitol | 2.5-3 |
| glycerol | 15-20 |
| Na-EDTA | 0.5-1.5 |
| benzalkonium chloride | 0.1-0.15 |
| a basic buffering agent providing a pH of about 8.0, e.g. TRIS | |
| purified water | |

TABLE 20

| Ingredient | g/l |
|---|---|
| laquinimod | 10-15 |
| sodium carboxymethylcellulose | 2-4 |
| polyvinylpyrrolidone | 5-15 |
| mannitol | 2.5-3 |
| glycerol | 15-20 |

(continued)

| Ingredient | g/l |
|---|---|
| Na-EDTA | 0.5-1.5 |
| benzalkonium chloride | 0.1-0.15 |
| a basic buffering agent providing a pH of about 8.0, e.g. TRIS | |
| purified water | |

TABLE 21

| Ingredient | g/l |
|---|---|
| laquinimod | 40-60 |
| sodium carboxymethylcellulose | 3-5 |
| carbomer copolymer (Type B) | 1.5-2 |
| mannitol | 2.5-3 |
| glycerol | 20-30 |
| Na-EDTA | 0.5-1.5 |
| benzalkonium chloride | 0.1-0.15 |
| a basic buffering agent providing a pH of about 8.0, e.g. TRIS | |
| purified water | |

TABLE 22

| Ingredient | g/l |
|---|---|
| laquinimod | 40-60 |
| sodium carboxymethylcellulose | 3-5 |
| carbomer copolymer (Type B) | 3-6 |
| mannitol | 2.5-3 |
| glycerol | 20-30 |
| Na-EDTA | 0.5-1.5 |
| benzalkonium chloride | 0.1-0.15 |
| a basic buffering agent providing a pH of about 8.0, e.g. TRIS | |
| purified water | |

TABLE 23

| Ingredient | g/l |
|---|---|
| laquinimod | 40-60 |
| sodium carboxymethylcellulose | 3-5 |
| macrogol 15 hydroxystearate | 2-3 |
| mannitol | 2.5-3 |
| glycerol | 20-30 |
| Na-EDTA | 0.5-1.5 |

(continued)

| Ingredient | g/l |
|---|---|
| benzalkonium chloride | 0.1-0.15 |
| a basic buffering agent providing a pH of about 8.0, e.g. TRIS | |
| purified water | |

TABLE 24

| Ingredient | g/l |
|---|---|
| laquinimod | 40-60 |
| sodium carboxymethylcellulose | 3-5 |
| polyoxyl castor oil | 40-60 |
| mannitol | 2.5-3 |
| glycerol | 20-30 |
| Na-EDTA | 0.5-1.5 |
| benzalkonium chloride | 0.1-0.15 |
| a basic buffering agent providing a pH of about 8.0, e.g. TRIS | |
| purified water | |

TABLE 25

| Ingredient | g/l |
|---|---|
| laquinimod | 40-60 |
| sodium carboxymethylcellulose | 3-5 |
| polyvinylpyrrolidone | 5-15 |
| mannitol | 2.5-3 |
| glycerol | 20-30 |
| Na-EDTA | 0.5-1.5 |
| benzalkonium chloride | 0.1-0.15 |
| a basic buffering agent providing a pH of about 8.0, e.g. TRIS | |
| purified water | |

TABLE 26

| Ingredient | g/l |
|---|---|
| laquinimod | 40-60 |
| sodium carboxymethylcellulose | 3-4 |
| carbomer copolymer (Type B) | 1.5-2 |
| poloxamer 407 | 1-3 |
| mannitol | 2.5-3 |
| glycerol | 20-30 |
| Na-EDTA | 0.5-1.5 |

(continued)

| Ingredient | g/l |
|---|---|
| benzalkonium chloride | 0.1-0.15 |
| a basic buffering agent providing a pH of about 8.0, e.g. TRIS | |
| purified water | |

TABLE 27

| Ingredient | g/l |
|---|---|
| laquinimod | 40-60 |
| sodium carboxymethylcellulose | 3-4 |
| carbomer copolymer (Type B) | 1.5-2 |
| polysorbate (e.g. polysorbate 80) | 0.3-0.7 |
| mannitol | 2.5-3 |
| glycerol | 20-30 |
| Na-EDTA | 0.5-1.5 |
| benzalkonium chloride | 0.1-0.15 |
| a basic buffering agent providing a pH of about 8.0, e.g. TRIS | |
| purified water | |

TABLE 28

| Ingredient | g/l |
|---|---|
| laquinimod | 40-60 |
| sodium carboxymethylcellulose | 3-4 |
| macrogol 15 hydroxystearate | 2-3 |
| poloxamer 407 | 1.5-2-5 |
| mannitol | 2.5-3 |
| glycerol | 20-30 |
| Na-EDTA | 0.5-1.5 |
| benzalkonium chloride | 0.1-0.15 |
| a basic buffering agent providing a pH of about 8.0, e.g. TRIS | |
| purified water | |

TABLE 29

| Ingredient | g/l |
|---|---|
| laquinimod | 40-60 |
| sodium carboxymethylcellulose | 3-4 |
| polyoxyl castor oil | 40-60 |
| poloxamer 407 | 1.5-2-5 |
| mannitol | 2.5-3 |

(continued)

| Ingredient | g/l |
|---|---|
| glycerol | 20-30 |
| Na-EDTA | 0.5-1.5 |
| benzalkonium chloride | 0.1-0.15 |
| a basic buffering agent providing a pH of about 8.0, e.g. TRIS | |
| purified water | |

TABLE 30

| Ingredient | g/l |
|---|---|
| laquinimod | 40-60 |
| sodium carboxymethylcellulose | 3-4 |
| polyvinylpyrrolidone | 5-15 |
| poloxamer 407 | 1.5-2-5 |
| mannitol | 2.5-3 |
| glycerol | 20-30 |
| Na-EDTA | 0.5-1.5 |
| benzalkonium chloride | 0.1-0.15 |
| a basic buffering agent providing a pH of about 8.0, e.g. TRIS | |
| purified water | |

TABLE 31

| Ingredient | g/l |
|---|---|
| laquinimod | 40-60 |
| carbomer copolymer (Type B) | 1-2 |
| mannitol | 2.5-3 |
| glycerol | 20-30 |
| Na-EDTA | 0.5-1.5 |
| benzalkonium chloride | 0.1-0.15 |
| a buffering agent providing a pH in the range of from 6.8-7.4, e.g 7.4, e.g. $Na_2HPO_4$ | |
| purified water | |

TABLE 32

| Ingredient | g/l |
|---|---|
| laquinimod | 40-60 |
| carbomer copolymer (Type B) | 1-2 |
| beta-cyclodextrin | 70-100 |
| mannitol | 2.5-3 |

(continued)

| Ingredient | g/l |
|---|---|
| glycerol | 20-30 |
| Na-EDTA | 0.5-1.5 |
| benzalkonium chloride | 0.1-0.15 |
| a buffering agent providing a pH of about 7.4, e.g. disodium hydrogen phosphate dihydrate | |
| purified water | |

TABLE 33

| Ingredient | g/l |
|---|---|
| laquinimod | 40-60 |
| hydroxypropyl methylcellulose | 3-7 |
| mannitol | 2.5-3 |
| glycerol | 20-30 |
| Na-EDTA | 0.5-1.5 |
| benzalkonium chloride | 0.1-0.15 |
| a buffering agent providing a pH of about 7.4, e.g. disodium hydrogen phosphate dihydrate | |
| purified water | |

TABLE 34

| Ingredient | g/l |
|---|---|
| laquinimod | 40-60 |
| polyvinyl alcohol | 30-50 |
| mannitol | 2.5-3 |
| glycerol | 20-30 |
| Na-EDTA | 0.5-1.5 |
| benzalkonium chloride | 0.1-0.15 |
| a buffering agent providing a pH of about 7.4, e.g. disodium hydrogen phosphate dihydrate | |
| purified water | |

TABLE 35

| Ingredient | g/l |
|---|---|
| laquinimod | 40-60 |
| carbomer copolymer (Type B) | 1-2 |
| polysorbate 80 | 0.5-1.5 |
| mannitol | 2.5-3 |
| glycerol | 20-30 |
| Na-EDTA | 0.5-1.5 |
| benzalkonium chloride | 0.1-0.15 |

(continued)

| Ingredient | g/l |
|---|---|
| a buffering agent providing a pH of about 7.4, e.g. disodium hydrogen phosphate dihydrate | |
| purified water | |

TABLE 36

| Ingredient | g/l |
|---|---|
| laquinimod | 40-60 |
| carbomer copolymer (Type B) | 1-2 |
| poloxamer 188 | 0.5-1.5 |
| mannitol | 2.5-3 |
| glycerol | 20-30 |
| Na-EDTA | 0.5-1.5 |
| benzalkonium chloride | 0.1-0.15 |
| a buffering agent providing a pH of about 7.4, e.g. disodium hydrogen phosphate dihydrate | |
| purified water | |

TABLE 37

| Ingredient | g/l |
|---|---|
| laquinimod | 40-60 |
| carbomer copolymer (Type B) | 1-2 |
| Sodium carboxymethylcellulose | 3-5 |
| polysorbate 80 | 0.5-1.5 |
| mannitol | 2.5-3 |
| glycerol | 20-30 |
| Na-EDTA | 0.5-1.5 |
| benzalkonium chloride | 0.1-0.15 |
| a buffering agent providing a pH of about 7.4, e.g. disodium hydrogen phosphate dihydrate | |
| purified water | |

*Process for preparing*

Also provided herein is a process for preparing the formulations of the invention, comprising the general steps of:

(1) admixing excipients other than viscosity agents with water to obtain an aqueous solution,
(2) admixing the viscosity agent(s) with the aqueous solution, and stirring for a period of 8-12 hours with refrigeration,
(3) adding a suitable pH regulating agent to the viscous solution, e.g. a basic buffer,
(4) optionally adjusting the volume of the viscous solution by adding further water,
(5) admixing laquinimod with the viscous solution, with mechanical dispersion if necessary,
(6) and, for an oil-in-water-emulsion: if necessary, further adjusting the volume of the aqueous phase, and admixing a pharmaceutically acceptable oil with the aqueous phase.

*Use of the formulation*

The formulation provided herein will be administered locally, preferably topically, to the eye of a patient, e.g. from a dosage container allowing for applying a small volume of the formulation to the eye, to allow for administration to an eye of e.g. 1-10 droplets having a droplet volume of 10-100 μl or 10-50 μl, or 10-40 μl e.g. 20-40 μl. In some embodiments, the formulation is applied from a single-dose container, or a single-use container. In some embodiments, the formulation is applied from a multidose container including the PureFlow® technology, as in the Novelia® multidose eye dropper, sold by Nemera (France).

Thus, in some embodiments, the formulation of the invention, advantageously free from any preservative, is provided in a multidose container having a valve construction allowing for effective protection of the formulation from microbial contamination, e.g. Novelia® multidose eye dropper.

The therapeutically effective amount of laquinimod may lie within the range of from 0.05-4.0 mg per administration (or an equivalent amount of a pharmaceutically acceptable salt of laquinimod). In some embodiments, the therapeutically effective amount of laquinimod is 0.05-2.0 mg per administration. In some embodiments, the therapeutically effective amount of laquinimod is about 0.05 mg per administration. In some embodiments, the therapeutically effective amount of laquinimod is about 0.1 mg per administration. In some embodiments, the therapeutically effective amount of is about 0.5 mg per administration. In some embodiments, the therapeutically effective amount of laquinimod is at least 0.05 mg/day. Preferably, the formulation provided herein will be periodically administered 1-6 times a day, e.g. 1-5 times a day, 1-3 times a day, or 1-2 times a day. In some embodiments, the periodic administration is once a day. In some embodiments, the periodic administration is twice a day. In some embodiments, the periodic administration is three times a day. In some embodiments, the periodic administration is once every 2 days. In some embodiments, the formulation is administered once a week.

In some embodiments, the formulation is administered once daily for a period of 2 to 14 days, or for a longer period, e.g. for a period of 1 month to 6 months, 2-6 months, or 3-6 months. In some embodiments, the formulation is administered once a day for a period of 3 days. In some embodiments, the formulation is administered once daily for a period of 5 to 14 days. In some embodiments, the formulation is administered once daily for a period of 10 to 14 days. In some embodiments, the formulation is administered once daily for about 7 days. In some embodiments, the formulation is administered for a period of 1-12 months, or for a period of 1-6 months, or for a period of 1-3 months, e.g. once a week for a period of 3-6 months. The precise dosage regiment and length of the treatment period however will normally be decided upon by the treating physician.

A further aspect is a dosage container, containing the laquinimod formulation as provided herein. The dosage container may be such as to include integral means to allow for administering a suitable dosage of the formulation to the eye of a patient, or such means may be provided separately. In some embodiments, the dosage container is a multidose container, allowing for the application of appropriate doses of the formulation to the eye of a patient. For example, in some embodiments, the dosage container is a bottle of the type sold by Nemera. In some embodiments, the dosage container is a Novelia® PFMD bottle or a bottle of similar type. In some of these embodiments, the formulation provided herein is preservative-free.

A further aspect is a kit (which may also be referred to as a kit-of-parts), comprising a dosage container as disclosed herein, and instructions for use. In some embodiments, such a kit also includes one or more additional containers, containing further appliances or materials useful in connection with the administration of the formulation, such as rinses, wipes, separate dosage means etc.

The ocular formulation disclosed herein is useful for the treatment of ocular diseases for which laquinimod provides a therapeutically beneficial effect, such as glaucoma, an ocular inflammatory disease, and a disease associated with excessive vascularization of the eye. In some embodiments, the OID is a disease affecting the intermediary or posterior parts of the eye.

The therapeutic activity of laquinimod in the treatment of such diseases has been described in patent documents referred to herein above.

In some embodiments, the ocular disease is an inflammatory ocular disease (OID). In some embodiments, the OID is selected from uveitis, bacterial conjunctivitis, viral conjunctivitis, or an inflammation of the orbital tissue, the lacrimal apparatus, the eyelid, the cornea, the retina or the optic pathway.

In some embodiments, the OID is selected from uveitis, acute conjunctivitis, viral conjunctivitis, nongonococcal bacterial conjunctivitis, adult gonococcal conjunctivitis, inclusion conjunctivitis, seasonal allergic conjunctivitis, chronic conjunctivitis, granular conjunctivitis, perennial allergic conjunctivitis, episcleritis, scleritis, atopic keratoconjunctivitis, and vernal keratoconjunctivitis.

In some embodiments, the OID is uveitis. Uveitis is the inflammation of the uvea or the uveal tract, which includes the iris, the ciliary body and the choroid portions of the eye. Inflammation of the overlying retina, called retinitis, or of the optic nerve, called optic neuritis, may occur with or without accompanying uveitis. Anatomically, uveitis may be classified as anterior, intermediate, posterior or diffuse, depending on the portion of the uveal tract that is affected.

Anterior uveitis is localized primarily to the anterior segment of the eye and includes iritis and iridocyclitis. Intermediate uveitis, also called peripheral uveitis, is centered in the area immediately behind the iris and lens in the region of the ciliary body and pars plana, hence the alternate terms "cyclitis" and "pars planitis". Posterior uveitis signifies any of a number of forms of retinitis, choroiditis, or optic neuritis. Diffuse uveitis implies inflammation involving all parts of the eye, including anterior, intermediate, and posterior structures (The Merck Manual, 1999). Inflammation from uveitis may result in a variety of other eye conditions, including glaucoma, cataracts, and cystoid macular edema, and ultimately may lead to permanent vision loss.

In some embodiments, the uveitis is intermediate, posterior or diffuse uveitis. In some embodiments, the uveitis is posterior or diffuse uveitis. In some embodiments, the uveitis includes posterior uveitis. In some embodiments, the uveitis is posterior uveitis. In some embodiments, the uveitis is diffuse uveitis.

In some embodiments, the OID is a conjunctivitis. In some embodiments, the OID is associated with an autoimmune disease, such as multiple sclerosis, autoimmune hemolytic anemia, autoimmune oophoritis, autoimmune thyroiditis, autoimmune uveoretinitis, Crohn's disease, chronic immune thrombocytopenic purpura, colitis, contact sensitivity disease, diabetes mellitus, Grave's disease, Guillain-Barre's syndrome, Hashimoto's disease, idiopathic myxedema, myasthenia gravis, psoriasis, pemphigus vulgaris, rheumatoid arthritis, or systemic lupus erythematosus. In some embodiments, the OID is associated with Crohn's disease.

In some embodiments, the ocular disease is associated with excessive (or deleterious) vascularisation of the eye, e.g. in response to external stimuli to the eye, or as a natural result of age. The eye consists of many different tissues, such as the cornea, the iris, the ciliary body, the choroid, the retina, and the macula, which tissues may be subject to deleterious vascularisation. In some embodiments, the ocular disease is associated with excessive vascularisation of the cornea, the iris, the ciliary body, the choroid, the retina, and/or the macula. In some embodiments, the ocular disease is associated with excessive vascularisation of a tissue in an anterior part of the eye, such as the cornea, the iris, or the ciliary body.

In some embodiments the ocular disease is selected from the group consisting of corneal neovascularisation, neovascularisation of the iris, neovascularisation of the ciliary body, corneal pannus, choroidal neovascularisation, retinal neovascularisation, hypertensive retinopathy, wet age-related macular degeneration, proliferative diabetic retinopathy, retinopathy of prematurity, and ischemic retinopathy.

In some further embodiments the ocular disease is associated with excessive vascularisation of a tissue in a posterior part of the eye, such as the choroid, the retina, or the macula.

In some embodiments, the ocular disease associated with excessive vascularisation is retinal neovascularisation. In some further embodiments, the ocular disease associated with excessive vascularisation is vascularisation of the macula, also known as wet age-related macular degeneration.

EXAM PLES

The following abbreviations may be used herein below:

| | |
|---|---|
| beta-CD | Beta-Cyclodextrin |
| EP | European pharmacopoeia |
| HPLC | High performance liquid chromatography |
| HPMC | Hydroxypropylmethylcellulose |
| kDa | kilo Dalton (kg/mol) |
| MFI | Micro flow imaging |
| Na CMC | Sodium Carboxymethylcellulose |
| p.a. | pro analysis |
| PVA | Polyvinyl alcohol |
| PVDF | Polyvinylidene fluoride |
| rH / RH | Relative humidity |
| RP-HPLC | Reverse phase high performance liquid chromatography |
| RT | Room temperature |
| SD | Standard deviation |
| SVP | Subvisible particles |
| USP | United States Pharmacopoeia |
| WFI | Water for injection |

*Materials*

All excipients used for the formulations were sourced in a quality compliant with Unites States Pharmacopoeia

(USP) and/or European Pharmacopeia (EP). The excipients were selected for ophthalmic administration. All chemicals used for analytical methods were sourced in a quality adequate for the individual method (e.g. *pro analysis*).

*Preparation of gel and emulsion formulations*

The formulations were prepared as follows: weighing of excipients, dissolution in water for injection (WFI) overnight at 2-8°C on magnetic stirrer in about 80% of final volume, adjustment of pH in formulations tempered to 23-25°C, filling to final volume and dissolving of laquinimod. The pH adjustment in highly viscous formulations was carried out on day 2 and aliquotation of formulations and start of accelerated aging study was carried out on day 3.

The weighing of excipients was done in a solubility-dependent order, meaning readily solubilized excipients were introduced first to the formulation preparation and excipients of low solubility, i.e. gelling agents were added last. In detail this process looked as follows: Providing of -80% of final volume with water (Aqua B. Braun, Braun) in non-sterile 0.5 L PP-beakers (Sarstedt), stepwise supplementation of excipients as described above with stirring of formulation preparation in between steps with magnetic bar on stirrer (CIMARECi Poly, Thermo Scientific).

The adjustment of pH required the complete dissolving of all excipients, which was not readily achieved for every formulation even after overnight stirring due to excipients of low-solubility like Carbomer Type B being present in some of the formulations. Therefore, the formulations were inspected by eye after overnight stirring and if incomplete dissolution of excipients was detected a high-performance rod disperser (T 18 digital ULTRA-TURRAX®, IKA) fitted with dispersion tool (S 18 N - 19 G, IKA) was applied to the formulation.

A sufficient volume of formulation was saved for placebo samples and laquinimod was dissolved in the gel formulations at 50 mg/ml using a vessel-fitted disperser (ULTRA-TURRAX® Tube Drive P control, IKA) equipped with single-use mixing vessels (DIS-300-S-M.10, IKA). Laquinimod and formulation were combined in the mixing vessel, the disperser set to 950 rpm and the formulation stirred for at least 5 min or until laquinimod was completely dissolved as judged by eye.

In the case of emulsion formulations, the aqueous phase of the formulation was blended with the oil phase, i.e. castor oil was added after dissolving laquinimod in the aqueous phase. Hence, the volume of the aqueous phase was corrected for the volume of oil before laquinimod was dissolved like described before. Emulsification was achieved with the disperser set to stirring speeds yielding a vortex between stirrer and surface of the liquid (1200 rpm for 100 ml formulation, 750 rpm for 50 ml placebo). Stirring took place for 2 min while the oil was added slowly to the aqueous solution over a period of -20 seconds utilizing reverse-pipetting technique to account for the high viscosity of oil (500-5000 μl pipette, Eppendorf).

*Adjustment of pH*

The adjustment of the final pH of all formulations and buffers as well as the pH measurement of the obtained formulations were performed with calibrated pH-electrode (VWR) connected to a SevenEasy pH-Meter (Mettler Toledo) at a temperature between 23 °C and 25 °C (compliant with both, EP and USP).

*Vial filling*

A manual filling procedure was performed using standard laboratory pipettes and sterile tips. Prior to filling, the formulations were mixed and emulsified, to ensure that the formulations were homogenous, and 3 ml of each formulation was transferred into sterile, particle-free 2R-vials (Adelphi) under a laminar air flow cabinet. The vials were closed with sterile FluroTec-coated (ethylene tetrafluoroethylene; ETFE) chlorobutyl stoppers (13 mm; Adelphi) and crimped with suitable aluminum caps.

*Reverse phase high performance liquid chromatography (RP-HPLC)*

The analysis by RP-HPLC was externalized to EpiQMmax with the parameters shown in TABLE 38.

TABLE 38

| **Chromatographic parameters of the RP-HPLC analysis** | |
|---|---|
| Column | Inertsil ODS-3V, 5 μm, 4.6x250 mm, GL Sciences Inc. |
| Detection | UV at 240 nm |
| Flow rate | 1.0 ml/min |

(continued)

| Chromatographic parameters of the RP-HPLC analysis | |
|---|---|
| Buffer | Acetonitrile: Buffer 7.0, 30:70 (Buffer 7.0: 50 mM ammonium acetate, pH 7.0) |
| Gradient | Isocratic for 30 min |
| Injection volume | 50 μl |
| Concentration of injected sample | 90 μg/ml for standard, about 100 μg/ml for samples |
| Temperature column oven | 45 °C |
| Temperature sampler | 5 °C |

*Determination of osmolality*

Determination of osmolality was performed by freezing point depression in an osmometer (Osmomat 3000, Gonotec). The total osmolality of aqueous solutions is determined by comparative measurements of the freezing points of pure water and of solutions. Each measurement was performed in 50 μl aliquots after calibration with sodium chloride standard (600 mOsm/kg) and purified water. The gels and emulsions were measured in 2 replicates, and 3 replicates if the deviation between duplicate measurements was >10%. Thereafter, the arithmetic mean and SD were calculated.

*Particle size measurement*

The particle sizes were determined by flow imaging analysis using a FlowCam 8100 system, 10x Objective (ANASYSTA) with manual loading of samples. Particle size measurement was performed for formulations containing laquinimod (as API) at a concentration of 50 mg/ml. The acceptance criteria for sub-visible particle (SVP) determination are defined as follows: for every 10 μg of laquinimod, no more than 20 particles > 25 μm; no more than 2 particles > 50 μm; no particle > 90 μm (PhEur monograph "Eye preparations", dosage form monograph no. 1163, version 01/2008). The results were reported as particles/10 μg laquinimod for each of the described particle filter groups.

Sample preparation for particle size measurement encompassed 1:10 dilution of samples with buffer solution of same pH, supersaturated with laquinimod. Sodium phosphate buffers with pH values of 6.8 and 7.4 and Tris buffers with pH values of 8.0 and 8.4 were prepared and laquinimod was added to the point of supersaturation before centrifuging at 2000xg for 3 min and using the supernatant for sample dilution. Prior to diluting samples, a suitable amount of buffer was filtrated with syringe filter (0.02 μm alumina membrane). The FlowCam 8100 system was rinsed with water (Aqua B Braun) between replicate measurements and additionally with 4% surfactant (Hellmanex®, Hellma Analytics) to prevent cross-contamination and ensure reproducibility between measurements.

*Examination of appearance*

Samples were visually controlled on changes during storage. Assessment was performed using an inspection light box equipped with non-flickering fluorescent lamps and a black and a white background plate. The samples were evaluated for 5 sec without magnification.

*Sample storage*

During characterization all samples (duplicates) were stored for up to two weeks at 5°C, 25°C, 30°C, and 40°C and protected from light and, in a follow-up experiment, at 5°C, 30°C, and 40°C. The samples stored at 5 ± 3 °C were kept in refrigerator with external monitoring of the temperature. Samples subjected to an accelerated aging were stored in cabinets (Memmert) with controlled humidity at 25 ± 2 °C / 60 ± 5 % relative humidity (RH), at 30 ± 2 °C / 65 ± 5 % RH and at 40 ± 2 °C / 75 ± 5 % RH (ICH Q1 guideline), respectively. The temperature was monitored throughout the storage period.

*Density measurements*

Sample density was measured with a Gay-Lussac Pycnometer (Carl Roth) and a sample volume of 1.103 ml at room temperature (RT). The pycnometer was weighed using an analytical balance (SECURA 124-1S, Sartorius).

*Viscosity measurements*

The dynamic viscosity of samples was measured with a falling ball viscometer Microviscometer Lovis 2000 ME (Anton Paar) at 20 °C, or at both 20 °C and 37 °C, respectively, using capillaries of different diameters. The dynamic viscosity $\eta$ of the respective samples was calculated based on the known density of the ball ($\rho b$ = 7.66 g/cm$^3$) and the measured sample density ($\rho s$) as well as the characteristic constant of the capillary (C1), according to the equation:

$$\eta = C1 * t1 * (\rho b - \rho s).$$

The falling time (t1) was averaged from the last six individual measurements at an angle of the capillary of 70°. In total, twelve measurements were taken, with the individual falling times reaching an equilibrium within the first six measurement cycles due to the polymeric nature of the samples.

The materials used in EXAMPLES 1-7, as well as the supplier and article number of each material are listed in TABLE 39.

TABLE 39

| Material | Supplier | Article No. |
|---|---|---|
| Carbomer Copolymer (Type B) Pemulen™ Tr-1 NF | Lubrizol/IMCD | PEM1005A |
| (Hydroxypropyl)methylcellulose, Hypromellose meets USP testing specifications | Sigma-Aldrich | H3785-25G |
| Sodium Carboxymethylcellulose | Sigma | 419311 |
| Kolliphor HS15 (Polyoxyl 35 Hydroxystereate) | BASF | 50259817 |
| Kolliphor® ELP (Polyoxyl castor oil) | BASF | 50259800 |
| Kollidon® 17 PF (Polyvinylpyrrolidone) | BASF | 50348142 |
| Polyvinylalcohol 4-88; EMPROVE® ESSENTIAL Ph Eur,USP,JPE | Merck | 1.41350.1000 |
| Beta-cyclodextrin (Kleptose HPB biopharma) | Roquette | 346113101A |
| Polysorbate 80 HP-LQ-(MH), super refined (EP, USP/NF, JP) | Croda | SR48833 |
| Kolliphor® P188 (Poloxamer 188) | BASF | 50424518 |
| Kolliphor® P 407 (Poloxamer 407) | BASF | 50424592 |
| Mannitol, EMPROVE® ESSENTIAL Ph. Eur., BP, USP, JP, FCC, E421, SAFC® | Merck | 1.05980.2500 |
| Glycerol 85 % (Ph.Eur., USP) | Merck | 1.04091.1000 |
| Na-EDTA, Titriplex® III EMPROVE® ESSENTIAL Ph Eur,BP,ChP,JP,USP | Merck | 1.08421.1000 |
| Benzalkonium chloride 50 % (m/v) | Caelo | 7088 |
| di-sodium hydrogen phosphate-dihydrate; EMPROVE® ESSENTIAL Ph Eur, BP,USP | Merck | 1.06576.1000 |
| TRIS, Tris(hydroxymethyl)aminomethane; (Trometamol) EMPROVE® ESSENTIAL Ph Eur,BP,ChP,JPC,USP | Merck | 1.08386.1000 |
| Castor Oil, tested according to Ph. Eur. | Sigma | 83912 |
| DPBS, no calcium, no magnesium | Thermo Fisher | 14190342 |

EXAMPLE 1

Ten different formulations, viz. 2 emulsions (E1 and E2) and 8 gels (S1 to S8), were prepared using the method described herein above. The ingredients of the formulations are shown in TABLE 40.

TABLE 40

| Name | E1 | E2 | S1 | S2 | S3 | S4 | S5 | S6 | S7 | S8 |
|---|---|---|---|---|---|---|---|---|---|---|
| pH | 7.4 | 8.4 | 6.8 | 7.4 | 8.0 | 7.4 | 7.4 | 7.4 | 7.4 | 7.4 |
| Concentration (g/l) | | | | | | | | | | |
| Laquinimod | 50 | 50 | 50 | 50 | 50 | 50 | 50 | 50 | 50 | 50 |
| Carbomer Copolymer (Type B) | 0.50 | 0.50 | 1.65 | 1.65 | 1.65 | 1.65 | | | 1.65 | 1.65 |
| HPMC | | | | | | | 5.0 | | | |
| PVA | | | | | | | | 40.0 | | |
| Polysorbate 80 | 40.0 | 40.0 | | | | | | | 1.0 | |
| Poloxamer 188 | | | | | | | | | | 1.0 |
| Mannitol | 2.7 | 2.7 | 2.7 | 2.7 | 2.7 | 2.7 | 2.7 | 2.7 | 2.7 | 2.7 |
| Glycerol | 25.0 | 25.0 | 25.0 | 25.0 | 25.0 | 25.0 | 25.0 | 25.0 | 25.0 | 25.0 |
| Na-EDTA | 1.0 | 1.0 | 1.0 | 1.0 | 1.0 | 1.0 | 1.0 | 1.0 | 1.0 | 1.0 |
| beta-cyclodextrin | | | | | | 85.0 | | | | |
| Benzalkonium chloride | 0.12 | 0.12 | 0.12 | 0.12 | 0.12 | 0.12 | 0.12 | 0.12 | 0.12 | 0.12 |
| Disodium hydrogen phosphate dihydrate | 1.78 | | 1.78 | 1.78 | | 1.78 | 1.78 | 1.78 | 1.78 | 1.78 |
| TRIS | | 1.21 | | | 1.21 | | | | | |
| Castor Oil | 50.0 | 50.0 | | | | | | | | |

*Chemical stability*

RP-HPLC analysis was performed after one and two weeks of storage at 30°C and after 2 weeks at 40°C, respectively. After two weeks at 30°C, very low degradation of 0.03% (in average) was observed, while at 40°C the degradation was slightly higher (0.34% in average). The highest degradation (based on RP-HPLC peak area) was observed in emulsion formulations E1 and E2, with a decrease of relative area of the main peak of 0.9% or higher compared to the initial concentration (t=0) (TABLE 41).

TABLE 41

| Formulation | Main peak (%) 0 week | Main peak (%) 1 week, 30 °C | Main peak (%) 2 weeks, 30 °C | Main peak (%) 2 weeks, 40 °C |
|---|---|---|---|---|
| E1 | 99.93 % | 99.98 % | 99.76 % | 98.67 % |
| E2 | 99.96 % | 99.96 % | 99.81 % | 99.06 % |
| S1 | 99.96 % | 99.96 % | 99.91 % | 99.68 % |
| S2 | 99.97 % | 99.97 % | 99.92 % | 99.83 % |
| S3 | 99.97 % | 99.97 % | 99.93 % | 99.73 % |
| S4 | 99.96 % | 99.96 % | 99.89 % | 99.65 % |
| S5 | 99.92 % | 99.97 % | 99.93 % | 99.82 % |
| S6 | 99.71 % | 99.97 % | 99.93 % | 99.75 % |
| S7 | 99.81 % | 99.97 % | 99.92 % | 99.71 % |
| S8 | 99.96 % | 99.96 % | 99.92 % | 99.82 % |

*Stability of formulations against particle forming, sedimentation and creaming*

For some of the formulations, some sedimentation was observed, and a sedimentation profile was determined by RP-HPLC. For this purpose, samples were taken at different height (Figure 1) before and after re-suspension. The absolute peak area of samples (after identical dilution) was compared (TABLE 42).

TABLE 42

| Formulation | Peak Area (mAU*min) | | | |
|---|---|---|---|---|
| | **Bottom** | **Middle** | **Top** | **After re-suspension** |
| E1 | 286.9 | 288.1 | 287.1 | 287.8 |
| S1 | 280.0 | 276.3 | 278.8 | 280.0 |
| S2 | 295.5 | 295.7 | 281.8 | 281.0 |
| S3 | 276.9 | 277.9 | 273.5 | 265.8 |
| S5 | 277.4 | 284.9 | 286.9 | 291.4 |

As may be seen from TABLE 42, the absolute peak areas of the main peak of all samples were similar before and after re-suspension. This indicates that visible particles represented only a very small portion of the total amount of laquinimod in the formulations. Therefore, it was concluded that laquinimod in supernatant must either be dissolved, or else suspended particles of laquinimod are small enough not to undergo sedimentation. The sediment was assumed to be an interaction between carbomer and laquinimod and to be influenced by pH. After the titration of formulation S1 from pH 7.4 to pH 8.0, the sediment was cleared, which supported the assumption of a pH-dependent interaction between carbomer and laquinimod.

The samples were visually checked at each time point (Figures 2-4). At t = 0, all gel formulations without laquinimod were optically clear, while the emulsion formulations were slightly turbid. After adding laquinimod, all gel formulations except S3 were turbid. The pH of formulation S3 was 8.0 which resulted in the complete dissolution of laquinimod. In formulation S8, even at t = 0 a slight sedimentation was observed.

During storage the emulsion formulations showed creaming at 5°C and 25°C, while at 30°C and at 40°C the emulsions broke (30°C: formulation E1; 40°C: both, formulation E1 and E2). After re-suspension, all emulsions were visually homogenous again.

In all gel formulations, except in S3, sedimentation was observed. S1 showed the largest sediment, followed by S2 and S8. Only little sediment was observed in formulations S4, S5, S6 and S7. In formulations S5 and S6 the sediment could not be re-suspended completely, while the other gel formulations were homogenous after re-suspension. In formulations S4 and S7 almost no sediment was observed, which suggests that the beta-cyclodextrin in formulation S4 and the Polysorbate in formulation S7 seem to have the stabilizing effect on laquinimod.

In accordance with Ph Eur 10.0 "Eye preparations", the particle size was measured in classes > 25 μm, > 50 μm and > 90 μm. In each class, the particle number was calculated per 10 μg of laquinimod. Particles were found in formulations E1, E2, S1, S2, and S8. In the suspensions (gels), cylindrical particles were detected, while in emulsions mostly oil droplet were detected (Figure 5). An increase in pH was observed to lead to a lower number of particles.

*Osmolality*

The osmolality of formulations E1, E2 and S1-S8 was measured, and, as a comparison, of similar formulations, except for not containing any laquinimod ("empty" formulations). The theoretical and measured osmolality of these formulations and of "empty" formulations, containing no laquinimod are shown in TABLE 43.

TABLE 43

| Formulation | Osmolality (mOsm/kg) | | Osmolality (mOsm/kg) (empty) | |
|---|---|---|---|---|
| | **Theoretical** | **Measured±SD** | **Theoretical** | **Measured±SD** |
| E1 | 545 | 709+9.9 | 405 | 460+6.4 |
| E2 | 525 | 683+4.2 | 384 | 439±3.5 |
| S1 | 461 | 538±9.8 | 320 | 330±9.1 |

(continued)

| Formulation | Osmolality (mOsm/kg) | | Osmolality (mOsm/kg) (empty) | |
|---|---|---|---|---|
| | Theoretical | Measured±SD | Theoretical | Measured±SD |
| S2 | 461 | 640±9.5 | 320 | 419±5.6 |
| S3 | 441 | 618+1.4 | 300 | 400+3.8 |
| S4 | 517 | 803+21 | 377 | 574±7.9 |
| S5 | 456 | 634±2.1 | 316 | 408±5.7 |
| S6 | 456 | 719±16.5 | 316 | 490+3.1 |
| S7 | 457 | 643+4.2 | 316 | 410+3.5 |
| S8 | 456 | 644±11 | 316 | 423±2.5 |

*Viscosity*

In order to assess the optimum concentration of viscosity agents, different concentrations were tested in "placebo" formulations (viz. not containing laquinimod) with target viscosity of 15 mPas. After the addition of laquinimod, the viscosity decreased strongly. This indicates an interaction between laquinimod and viscosity agents. All formulations were in a similar range of viscosity besides formulation S5, which contained HPMC. The viscosity of the different formulations was adjusted using different viscosity agents. The amount of the viscosity agents was determined by placebo formulations. The viscosities of formulations E1-S8, measured at 20 °C, are indicated in TABLE 44, which also lists the type and concentration of the viscosity agent used.

TABLE 44

| Formulation | Viscosity agent | Concentration (g/l) | Viscosity (mPas) |
|---|---|---|---|
| E1 | Carbomer copolymer | 1.65 | 2.2 ± 0.2 |
| E2 | Carbomer copolymer | 1.65 | 2.4 ± 0 |
| S1 | Carbomer copolymer | 1.65 | 3.0 ± 0 |
| S2 | Carbomer copolymer | 1.65 | 2.8 ± 0 |
| S3 | Carbomer copolymer | 1.65 | 3.1 ± 0.1 |
| S4 | Carbomer copolymer | 1.65 | 3.7 ± 0 |
| S5 | HPMC | 3.8 | 25.3 ± 0.7 |
| S6 | PVA | 20 | 5.4 ± 0 |
| S7 | Carbomer copolymer | 1.65 | 2.8 ± 0 |
| S8 | Carbomer copolymer | 1.65 | 2.8 ± 0 |

EXAMPLE 2

Formulations E2 (renamed E2-0) and S3 (renamed S3-0) were selected as a starting point for further formulations, viz. emulsion formulations E2-1 to E2-4 and gel formulations S3-1 to S3-10. In both types of formulations, having a pH of at least 8, laquinimod was completely dissolved.

As there was a substantial difference in viscosity of placebo formulations and those containing laquinimod, the viscosity of laquinimod-containing formulations E2-1 to E2-4 and S3-1 to S3-10 was adjusted by addition of sodium carboxymethyl cellulose.

The ingredients in the emulsion formulations are shown in TABLE 45, and the ingredients in the gel formulations are shown in TABLE 46.

TABLE 45

| Name | E2-0 | E2-1 | E2-2 | E2-3 | E2-4 |
|---|---|---|---|---|---|
| pH | 8.4 | 8.4 | 8.4 | 8.4 | 8.4 |
| Concentration (g/l) | | | | | |
| Laquinimod | 50 | 50 | 50 | 50 | 50 |
| Sodium carboxymethylcellulose | | 5.00 | 4.50 | 4.50 | 4.50 |
| Carbomer Copolymer (Type B) | 0.50 | 0.50 | | | |
| Kolliphor® HS15 (Macrogol 15 hydroxystearate) | | | 2.50 | | |
| Kolliphor® ELP (Polyoxyl castor oil) | | | | 50.0 | 50.0 |
| Polysorbate 80 | 40.0 | 40.0 | 25.0 | 25.0 | |
| Poloxamer 407 | | | 15.0 | 15.0 | 20.0 |
| Mannitol | 2.70 | 2.70 | 2.70 | 2.70 | 2.70 |
| Glycerol | 25.0 | 25.0 | 25.0 | 25.0 | 25.0 |
| Na-EDTA | 1.00 | 1.00 | 1.00 | 1.00 | 1.00 |
| Benzalkonium chloride | 0.12 | 0.12 | 0.12 | 0.12 | 0.12 |
| TRIS | 1.21 | 1.21 | 1.21 | 1.21 | 1.21 |
| Castor Oil | 1.21 | 1.21 | 1.21 | 1.21 | 1.21 |

TABLE 46

| Name | S3-0 | S3-1 | S3-2 | S3-3 | S3-4 | S3-5 | S3-6 | S3-7 | S3-8 | S3-9 | S3-10 |
|---|---|---|---|---|---|---|---|---|---|---|---|
| pH | 8.0 | 8.0 | 8.0 | 8.0 | 8.0 | 8.0 | 8.0 | 8.0 | 8.0 | 8.0 | 8.0 |
| Concentration (g/l) | | | | | | | | | | | |
| Laquinimod | 50 | 50 | 50 | 50 | 50 | 50 | 50 | 50 | 50 | 50 | 50 |
| Sodium carboxymethylcellulose | | 4.00 | 4.00 | 4.00 | 4.00 | 4.00 | 3.50 | 4.00 | 3.50 | 3.50 | 3.50 |
| Carbomer Copolymer (Type B) | 1.65 | 1.65 | 4.50 | | | | 1.65 | 1.65 | | | |
| Kolliphor® HS15 (Macrogol 15 hydroxy stearate) | | | | 2.50 | | | | | 2.50 | | |
| Kolliphor® ELP (Polyoxyl castor oil) | | | | | 50.0 | | | | | 50.0 | |
| Kollidon® 17 PF (Polyvinylpyrrolidone) | | | | | | 10.0 | | | | | 10.0 |
| Polysorbate 80 | | | | | | | | 0.50 | | | |
| Poloxamer 407 | | | | | | | 2.00 | | 2.00 | 2.00 | 2.00 |
| Mannitol | 2.70 | 2.70 | 2.70 | 2.70 | 2.70 | 2.70 | 2.70 | 2.70 | 2.70 | 2.70 | 2.70 |
| Glycerol | 25.0 | 25.0 | 25.0 | 25.0 | 25.0 | 25.0 | 25.0 | 25.0 | 25.0 | 25.0 | 25.0 |
| Na-EDTA | 1.00 | 1.00 | 1.00 | 1.00 | 1.00 | 1.00 | 1.00 | 1.00 | 1.00 | 1.00 | 1.00 |
| Benzalkonium chloride | 0.12 | 0.12 | 0.12 | 0.12 | 0.12 | 0.12 | 0.12 | 0.12 | 0.12 | 0.12 | 0.12 |
| TRIS | 1.21 | 1.21 | 1.21 | 1.21 | 1.21 | 1.21 | 1.21 | 1.21 | 1.21 | 1.21 | 1.21 |

*Chemical stability of laquinimod*

Samples of all formulations were stored for up to 2 weeks at 5°C, 30°C, and 40°C and subsequently analysed by RP-HPLC. During 5°C and 30°C storage no substantial decrease of relative area of main peak was detected, but there was a small decrease after 2 weeks of storage at 40 °C. The results of the RP-HPLC analyses are shown in TABLES 47 and 48.

TABLE 47

| **Formulation** | **t = 0** | **t = 1 week** | | |
|---|---|---|---|---|
| | | **5 °C** | **30 °C** | **40 °C** |
| | **Main peak (%) (mean ± SD)** | | | |
| E2-0 | 99.95 ± 0.00 | 99.95 ± 0.00 | 99.93 ± 0.00 | 99.76 ± 0.03 |
| E2-1 | 99.95 ± 0.00 | 99.95 ± 0.00 | 99.93 ± 0.00 | 99.73 ± 0.01 |
| E2-2 | 99.95 ± 0.00 | 99.95 ± 0.00 | 99.94 ± 0.00 | 99.80 ± 0.03 |
| E2-3 | 99.95 ± 0.00 | 99.95 ± 0.00 | 99.94 ± 0.00 | 99.74 ± 0.02 |
| E2-4 | 99.95 ± 0.00 | 99.95 ± 0.00 | 99.94 ± 0.00 | 99.76 ± 0.01 |
| S3-0 | 99.95 ± 0.00 | 99.95 ± 0.00 | 99.95 ± 0.00 | 99.92 ± 0.00 |
| S3-1 | 99.95 ± 0.00 | 99.95 ± 0.00 | 99.87 ± 0.11 | 99.93 ± 0.00 |
| S3-2 | 99.95 ± 0.00 | 99.89 ± 0.09 | 99.95 ± 0.00 | 99.93 ± 0.00 |
| S3-3 | 99.95 ± 0.00 | 99.94 ± 0.02 | 99.95 ± 0.00 | 99.88 ± 0.08 |
| S3-4 | 99.95 ± 0.00 | 99.95 ± 0.00 | 99.94 ± 0.01 | 99.74 ± 0.01 |
| S3-5 | 99.95 ± 0.00 | * | * | * |
| S3-6 | 99.95 ± 0.00 | 99.91 ± 0.00 | 99.93 ± 0.03 | 99.89 ± 0.01 |
| S3-7 | 99.95 ± 0.00 | 99.88 ± 0.11 | 99.94 ± 0.02 | 99.91 ± 0.00 |
| S3-8 | 99.95 ± 0.00 | 99.95 ± 0.00 | 99.95 ± 0.00 | 99.92 ± 0.03 |
| S3-9 | 99.96 ± 0.01 | 99.96 ± 0.00 | 99.95 ± 0.01 | 99.73 ± 0.01 |
| S3-10 | 99.95 ± 0.00 | * | * | * |

TABLE 48

| **Formulation** | **t=0** | **t = 2 weeks** | | |
|---|---|---|---|---|
| | | **5 °C** | **30 °C** | **40 °C** |
| | **Main peak (%) (mean ± SD)** | | | |
| E2-0 | 99.95 ± 0.00 | 99.95 ± 0.00 | 99.91 ± 0.00 | 99.55 ± 0.04 |
| E2-1 | 99.95 ± 0.00 | 99.95 ± 0.00 | 99.91 ± 0.00 | 99.49 ± 0.01 |
| E2-2 | 99.95 ± 0.00 | 99.95 ± 0.00 | 99.92 ± 0.00 | 99.67 ± 0.00 |
| E2-3 | 99.95 ± 0.00 | 99.96 ± 0.00 | 99.92 ± 0.00 | 99.51 ± 0.01 |
| E2-4 | 99.95 ± 0.00 | 99.95 ± 0.00 | 99.92 ± 0.00 | 99.56 ± 0.01 |
| S3-0 | 99.95 ± 0.00 | 99.95 ± 0.00 | 99.94 ± 0.00 | 99.88 ± 0.02 |
| S3-1 | 99.95 ± 0.00 | 99.95 ± 0.00 | 99.94 ± 0.00 | 99.89 ± 0.02 |
| S3-2 | 99.95 ± 0.00 | 99.95 ± 0.00 | 99.94 ± 0.00 | 99.88 ± 0.02 |
| S3-3 | 99.95 ± 0.00 | 99.95 ± 0.00 | 99.95 ± 0.00 | 99.90 ± 0.03 |
| S3-4 | 99.95 ± 0.00 | 99.95 ± 0.00 | 99.91 ± 0.02 | 99.54 ± 0.01 |

(continued)

| Formulation | t=0 | t = 2 weeks | | |
|---|---|---|---|---|
| | | 5 °C | 30 °C | 40 °C |
| | Main peak (%) (mean ± SD) | | | |
| S3-5 | 99.95 ± 0.00 | * | * | * |
| S3-6 | 99.95 ± 0.00 | 99.92 ± 0.03 | 99.94 ± 0.00 | 99.86 ± 0.00 |
| S3-7 | 99.95 ± 0.00 | 99.95 ± 0.00 | 99.95 ± 0.00 | 99.89 ± 0.01 |
| S3-8 | 99.95 ± 0.00 | 99.95 ± 0.00 | 99.95 ± 0.00 | 99.88 ± 0.01 |
| S3-9 | 99.96 ± 0.01 | 99.96 ± 0.01 | 99.91 ± 0.02 | 99.49 ± 0.00 |
| S3-10 | 99.95 ± 0.00 | * | * | * |

*Stability of formulations against particle forming, sedimentation and creaming*

The visual appearance of samples of all formulations after aging was examined in the same way as described for EXAMPLE 1 and similar results were obtained.

*Osmolality*

The osmolality of formulations E2-0 to E2-4 and S3-0 to S3-10 was measured, as was that of similar formulations, except for not containing any laquinimod ("empty" formulations).

The theoretical and measured osmolality of these formulations are shown in TABLE 49.

TABLE 49

| Formulation | Osmolality (mOsm/kg) | | Osmolality (mOsm/kg) (empty) | |
|---|---|---|---|---|
| | Theoretical | Measured±SD | Theoretical | Measured+SD |
| E2-0 | 672 | 550 ± 23.4 | 391 | 312 ± 3.6 |
| E2-1 | 672 | 571 ± 5.7 | 391 | 322 ± 2.1 |
| E2-2 | 662 | 590 ± 3.5 | 381 | 332 ± 4.2 |
| E2-3 | 662 | 691 ± 10.6 | 381 | 399 ± 4.9 |
| E2-4 | 643 | 637 ± 2.8 | 362 | 357 ± 5.7 |
| S3-0 | 588 | 478 ± 12 | 307 | 263 ± 5.5 |
| S3-1 | 588 | 491 ± 12.6 | 307 | 276 ± 2.8 |
| S3-2 | 588 | 493 ± 4.2 | 307 | 248 ± 4.2 |
| S3-3 | 588 | 505 ± 8.6 | 307 | 281 ± 4.9 |
| S3-4 | 588 | 562 ± 1.4 | 307 | 334 ± 1.4 |
| S3-5 | 589 | 522 ± 2.1 | 308 | 314 ± 13 |
| S3-6 | 588 | 506 ± 9.9 | 307 | 274 ± 6 |
| S3-7 | 589 | 492 ± 9.2 | 308 | 276 ± 4.2 |
| S3-8 | 588 | 499 ± 7.1 | 307 | 285 ± 3.5 |
| S3-9 | 588 | 565 ± 18.2 | 307 | 341 ± 0.7 |
| S3-10 | 589 | 515 ± 0 | 308 | 300 ± 0.7 |

*Viscosity*

The viscosity of some formulations containing laquinimod and carbomer was adjusted with sodium carboxymethyl cellulose. The formulations containing Kolliphor® HS15, Kolliphor® ELP, or Kollidon® 17PF had a lower viscosity.

In addition to the measurement at 20 °C, the viscosity was measured at 37 °C to mimic the conditions in the eye. The results are shown in TABLE 50.

TABLE 50

| Formulation | Without laquinimod | | With laquinimod | |
|---|---|---|---|---|
| | Viscosity (mPas) at 20 °C | Viscosity (mPas) at 37 °C | Viscosity (mPas) at 20 °C | Viscosity (mPas) at 37 °C |
| E2-0 | 4.0 | 2.5 | 2.1 | 1.3 |
| E2-1 | 25.9 | 15.6 | 13.5 | 7.3 |
| E2-2 | 16.4 | 9.5 | 11.6 | 6.3 |
| E2-3 | 23.2 | 13.0 | 12.9 | 6.5 |
| E2-4 | 20.4 | 11.9 | 12.3 | 5.9 |
| S3-0 | 281.7 | 161.1 | 2.8 | 1.8 |
| S3-1 | 75.3 | 43.9 | 14.8 | 8.3 |
| S3-2 | * | * | 73.5 | 47.4 |
| S3-3 | 8.1 | 4.8 | 5.7 | 3.2 |
| S3-4 | 9.8 | 5.7 | 7.5 | 4.1 |
| S3-5 | 9.2 | 5.2 | 6.4 | 3.6 |
| S3-6 | 78.4 | 51.3 | 11.7 | 6.6 |
| S3-7 | 101.3 | 58.9 | 14.1 | 7.0 |
| S3-8 | 7.0 | 4.1 | 5.2 | 3.0 |
| S3-9 | 9.1 | 5.2 | 6.6 | 3.6 |
| S3-10 | 7.5 | 4.4 | 5.7 | 3.2 |
| *Analysis not possible due to high viscosity of sample. | | | | |

EXAMPLE 3

A further gel formulation, formulation S7-1, was prepared, starting from the formulation S7, but with addition of a further viscosity agent. The contents of formulation S7 (for comparison) and S7-1 are shown in TABLE 51.

TABLE 51

| Name | S7 | S7-1 |
|---|---|---|
| pH | 7.4 | 7.4 |
| Concentration (g/l) | | |
| Laquinimod | 50 | 50 |
| Carbomer Copolymer (Type B) | 1.65 | 1.65 |
| Sodium carboxymethylcellulose | | 4.0 |
| Polysorbate 80 | 1.0 | 1.0 |
| Mannitol | 2.7 | 2.7 |
| Glycerol | 25.0 | 25.0 |

(continued)

| Name | S7 | S7-1 |
|---|---|---|
| pH | 7.4 | 7.4 |
| Concentration (g/l) | | |
| Na-EDTA | 1.0 | 1.0 |
| Benzalkonium chloride | 0.12 | 0.12 |
| Disodium hydrogen phosphate dihydrate | 1.78 | 1.78 |

Using the same system for determining viscosity as described herein above, the viscosity of formulation S7-1 at 20 °C was 17.6 mPas.

EXAMPLE 4

The ocular permeation of laquinimod in formulations of the invention was investigated, using bovine cornea.

*Material*

Three formulations of the invention (S3, S4 and S7), containing laquinimod at a concentration of 50 mg/ml, were used in the assay. As a reference, a formulation of laquinimod (50 mg/ml) in saline (0.9% w/v) was used. The formulations were stored in a refrigerator set to maintain +4°C.

Bovine eyes were supplied by ABP, Ruthvenfield Road, Inveralmond Industrial Estate, Perth, PH1 3XB, UK. The eyes had been collected from freshly slaughtered cattle and placed into a container containing cold Hank's balanced salt solution (HBSS). Eyes were kept cold using cool packs during transport to the test facility.

The receptor fluid for drug permeation assays was HEPES solution (0.1 M, pH 7.4±0.1, containing 0.01% w/v EDTA).

*Summary of experimental design*

Six (6) replicates were treated with each formulation for a 4 h exposure period. Permeability samples were collected test item treated corneas only, immediately prior to dosing and at half hour intervals following dosing. The final sample was collected at 4 h post dose (in total 9 samples were collected per cornea). Opacity of corneas was measured prior to dosing and following the final permeability sample collection. The concentration of laquinimod in the permeation samples was measured throughout the exposure period.

*Test System Set Up*

Corneas were dissected from freshly obtained eyes. On arrival, eyes were rinsed with HBSS then examined for obvious defects or signs of damage (e.g. scratches, opacity or neovascularisation). Corneas from undamaged eyes were removed, leaving a sclera margin of ca 3 mm. Collected corneas were placed epithelial side down in HBSS at ambient temperature until required. Corneas were then be mounted epithelial side forward in specially designed corneal holders obtained from Duratec Analysentechnik GmbH, Rheinauer Strasse 4, D-68766 Hockenheim, Germany. These holders consist of anterior and posterior compartments, which allow access to the epithelial and endothelial sides of the cornea, respectively.

Once mounted, both chambers of each corneal holder were filled with pre-warmed Minimum Essential Medium (MEM) without phenol red. The posterior chamber was filled first to encourage the cornea to return to its original curvature and care was taken to avoid the introduction of bubbles into the media. Holders were then equilibrated for ≥1 h in an incubator set to maintain a temperature of 32°C prior to dosing.

At the end of the equilibration period, the media in both chambers were replaced with fresh pre-warmed MEM without phenol red. Baseline opacity readings was then taken and damaged corneas or corneas with opacity >7 opacity units were rejected from further use. Corneas that passed these checks were assigned to study treatment groups. The MEM in the posterior chamber was replaced with pre-warmed receptor fluid using a syringe to allow measurement of the volume of fluid added to the chamber. The volume of the posterior chamber was recorded.

*Pre-dose Treatment*

MEM in the anterior chamber was removed immediately prior to dosing. A pre-dose sample of receptor fluid was collected from each test item treated cornea. No receptor fluid samples were collected from corneas to be treated with the vehicle control. The access ports for the posterior chamber were sealed with tape to prevent leakage during the incubation period.

*Dosing*

The laquinimod formulations and the saline control were applied undiluted. Prior to dosing, cornea holders were tipped forward to avoid contact between dosing solution and the corneal epithelium. The dose (ca 750 μl) was applied via the anterior chamber access ports using a syringe. Each dose was applied to six (6) replicate corneas for a 4 h exposure. Before collecting the dose from the vial, the formulation was resuspended by gentle turning of the vial.

*Exposure*

After dosing, corneal holders were tilted into the horizontal position to begin exposure, taking care to cover the epithelial surface of each cornea with test item. Corneas were exposed to test items or vehicle control for 4 h in an incubator set to maintain a temperature of 32°C.

*Receptor Fluid Sampling*

Receptor fluid samples (ca 1 ml) were collected from the posterior chamber of each test item treated cornea before dosing and at half hour intervals post dose. A final receptor fluid sample was taken 4 h post dose. Immediately following sampling, the removed receptor fluid was replaced with fresh, pre-warmed receptor fluid and the posterior chamber re-sealed taking care not to lose any sample or introduce air bubbles. Collected samples of receptor fluid were stored in a freezer set to maintain a temperature of -20 °C until analysis.

*Rinsing*

Following the final receptor fluid collection, dosing solutions were removed from anterior chambers and corneas were rinsed with MEM with phenol red (ca 5 ml per rinse). Corneas were rinsed at least 3 times for both the anterior and posterior chambers, or until no there was no visual evidence of residual test item. Once rinsing was complete, one additional rinse with MEM without phenol red was performed to remove phenol red, to avoid any interference with subsequent optical measurements. Both chambers were then refilled with MEM without phenol red before proceeding.

*Measurement of Opacity*

Following rinsing, opacity of all corneas was measured using an opacitometer supplied by Duratec Analysentechnik GmbH, by placing each corneal holder into the opacitometer and recording the subsequent lux reading.

*Analysis of Permeation Samples*

Samples were stored in a freezer set to maintain -20°C until analysis. A 10 μl aliquot of calibration standards (in the range 50.0 - 25000 ng/ml), quality control or test samples was transferred in to a 96 round well plate and to each well 10 μl internal standard was added. This was then diluted with 200 μl methanol and 200 μl 0.1% trifluoroacetic acid. The 96 round well plate was then capped, vortex mixed and centrifuged at a temperature of 4 °C for 5 minutes at 2400 g. Samples were then injected on a Sciex API4000™ mass spectrometer coupled with a Waters Acquity UPLC® system to generate known concentrations of laquinimod.

Study samples were extracted and analysed in batches together with calibration standards and quality check (QC) samples using the established method. The calibration samples were extracted in duplicate.

The detector responses were plotted against the concentration of laquinimod to produce a calibration curve, excluding the origin from the regression analysis. The determined concentration for each prepared standard used to construct the calibration curve should be within 100 ± 20 % of the nominal concentration. At least 75% (and a minimum of 6) of the calibration standards should meet the above criteria. The determined concentrations of at least 67% of the bracketing QC samples must be within 100 ± 20 % of the nominal concentrations and at least 50% at each concentration level should fulfil this criterion.

Study samples where the determined concentration was above the analytical range of the assay were re-assayed following dilution. Study samples where the determined concentration was below the analytical range were reported as <LLOQ (not quantifiable).

Data collection was performed using Analyst® Software from Applied Biosystems. Statistical analyses including regression analysis and descriptive statistics including arithmetic means and standard deviations, accuracy and precision were performed using Watson Laboratory Information Management System (Watson LIMS™) and Microsoft® Excel®.

*Sample storage*

Receptor fluid samples were stored in a freezer at a temperature of -20 °C until analysis.

*Computerised systems*

The computerised systems used in the study and the data collected and/or analysed by these systems are indicated in TABLE 52.

TABLE 52

| **System name** | **Data collected and/or analysed** |
|---|---|
| Analyst | Data collection from LC-MS/MS |
| Watson | Statistical Analysis |
| Microsoft Excel | Statistical Analysis |

*Results*

Statistical analyses were limited to derivation of means, standard deviations and coefficients of variation where appropriate.

*Opacity*

Opacity was calculated using the following formula:

$$\text{Opacity} = \frac{\left(\frac{I_0}{I}\right) - 0.9894}{0.0251}$$

where:

$I_0$ = the opacitometer measurement (lux) for a corneal holder containing MEM only, and
I = the measurement (lux) for the holder containing a cornea (either baseline or post-dose).

The opacity change and corrected opacity change were calculated as:

Opacity Change = Post-Dose Opacity - Baseline Opacity, and
Corrected Opacity Change = Opacity Change (test item) - Opacity Change (saline).

In TABLE 53 the results of the pre-dose and post-dose opacity measurements are listed.

TABLE 53

| Treatment | Cornea No. | Pre-dose Opacity (lux) | Post-dose Opacity (lux) | Opacity change* (lux) | Mean* (lux) | SD (lux) |
|---|---|---|---|---|---|---|
| S3 | 1 | 5.37 | 5.55 | -1.12 | 0.56 | 1.53 |
| | 2 | 2.52 | 6.17 | 2.35 | | |
| | 3 | 4.86 | 4.77 | -1.38 | | |
| | 4 | 3.22 | 6.17 | 1.65 | | |
| | 5 | 4.44 | 7.09 | 1.36 | | |
| | 6 | 3.78 | 5.55 | 0.47 | | |
| S4 | 7 | 3.94 | 3.54 | -1.70 | -1.44 | 0.90 |
| | 8 | 3.30 | 2.75 | -1.84 | | |
| | 9 | 3.70 | 3.46 | -1.54 | | |
| | 10 | 3.86 | 3.14 | -2.01 | | |
| | 11 | 3.30 | 2.68 | -1.92 | | |
| | 12 | 2.37 | 4.02 | 0.36 | | |
| S7 | 13 | 5.03 | 9.66 | 3.34 | 3.62 | 2.19 |
| | 14 | 3.22 | 6.53 | 2.02 | | |
| | 15 | 3.14 | 12.19 | 7.75 | | |
| | 16 | 2.68 | 8.14 | 4.17 | | |
| | 17 | 3.70 | 7.18 | 2.19 | | |
| | 18 | 3.06 | 6.63 | 2.27 | | |
| Saline | 19 | 2.75 | 4.94 | 0.89 | 0.00 | 1.99 |
| | 20 | 2.91 | 7.00 | 2.79 | | |
| | 21 | 5.03 | 6.44 | 0.12 | | |
| | 22 | 4.19 | 3.54 | -1.95 | | |
| | 23 | 3.94 | 3.38 | -1.86 | | |
| | 24** | *4.11* | *7.75* | *2.35* | | |

* Corrected for negative control opacity change
**Cornea 24 was rejected as it was suspected to have had a minor damage at the beginning of the experiment despite achieving pre-dose opacity criteria.

Treatment with formulations S3 or S4 caused little to no change in opacity, while treatment with Formulation S7 caused some increase in opacity.

*Laquinimod Permeability*

The total permeation of laquinimod was calculated from the results provided from LC-MS/MS analysis.

The permeability results, in terms of cumulative absorption of laquinimod ($\mu g/cm^2$) across the bovine cornea (n = 6) following exposure to formulations S3, S4 and S7, respectively, are listed in TABLES 54 to 56 and illustrated in Figures 6-8.

TABLE 54

| Cumulative absorption of laquinimod (μg/cm²) following exposure to S3 | | | | | | | | |
|---|---|---|---|---|---|---|---|---|
| Time (min) | Cornea 1 | Cornea 2 | Cornea 3 | Cornea 4 | Cornea 5 | Cornea 6 | Mean | SD |
| 0 | *0.00* | *0.00* | *0.00* | *0.00* | *0.00* | *0.00* | *0.00* | *0.00* |
| 30 | 3.71 | *0.00* | *0.00* | *0.00* | *0.00* | *0.00* | 0.62 | 1.51 |
| 60 | 2.17 | 0.31 | 0.42 | 1.58 | 0.70 | 1.09 | 1.05 | 0.72 |
| 90 | 15.5 | 7.27 | 7.12 | 10.0 | 2.13 | 5.65 | 7.94 | 4.49 |
| 120 | 31.5 | 28.2 | 22.5 | 38.3 | 10.1 | 22.2 | 25.5 | 9.63 |
| 150 | 58.2 | 62.7 | 45.7 | 86.3 | 22.7 | 50.6 | 54.4 | 21.0 |
| 180 | 121 | 103 | 64.6 | 157 | 41.3 | 80.8 | 94.6 | 41.4 |
| 210 | 219 | 146 | 102 | 252 | 70.1 | 131 | 154 | 69.6 |
| *240* | *261* | *209* | *129* | *312* | *105* | *148* | *194* | *80.9* |
| Values in italics were LLOQ so have been corrected to zero (<50 ng/ml) | | | | | | | | |

TABLE 55

| Cumulative absorption of laquinimod (μg/cm²) following exposure to S4 | | | | | | | | |
|---|---|---|---|---|---|---|---|---|
| Time (min) | Cornea 7 | Cornea 8 | Cornea 9 | Cornea 10 | Cornea 11 | Cornea 12 | Mean | SD |
| 0 | *0.00* | *0.00* | *0.00* | *0.00* | *0.00* | *0.00* | *0.00* | *0.00* |
| 30 | *0.00* | *0.00* | *0.00* | *0.00* | *0.00* | *0.00* | 0.00 | 0.00 |
| 60 | 2.00 | 2.56 | 1.82 | 2.40 | 1.94 | 1.07 | 1.96 | 0.52 |
| 90 | 9.89 | 11.2 | 8.45 | 10.2 | 8.34 | 4.44 | 8.77 | 2.39 |
| 120 | 25.9 | 31.5 | 24.0 | 30.5 | 25.5 | 20.4 | 26.3 | 4.13 |
| 150 | 45.6 | 50.7 | 41.0 | 47.1 | 41.3 | 31.6 | 42.9 | 6.63 |
| 180 | 70.3 | 75.7 | 66.0 | 82.4 | 78.8 | 54.1 | 71.2 | 10.2 |
| 210 | 97.5 | 91.9 | 79.1 | 89.2 | 84.6 | 72.8 | 85.9 | 8.96 |
| *240* | 116 | 133 | 122 | 133 | 119 | 102 | 121 | 11.6 |
| Values in italics were LLOQ so have been corrected to zero (<50 ng/ml) | | | | | | | | |

TABLE 56

| Cumulative absorption of laquinimod (μg/cm²) following exposure to S7 | | | | | | | | |
|---|---|---|---|---|---|---|---|---|
| Time (min) | Cornea 13 | Cornea 14 | Cornea 15 | Cornea 16 | Cornea 17 | Cornea 18 | Mean | SD |
| 0 | *0.00* | *0.00* | *0.00* | *0.00* | *0.00* | *0.00* | *0.00* | *0.00* |
| 30 | *0.00* | *0.00* | *0.00* | 0.226 | *0.00* | *0.00* | 0.0377 | 0.0924 |
| 60 | 3.70 | 5.89 | 4.99 | 8.70 | 4.17 | 4.47 | 5.32 | 1.82 |
| 90 | 17.4 | 23.5 | 19.9 | 32.3 | 20.6 | 17.4 | 21.8 | 5.60 |
| 120 | 50.3 | 62.8 | 57.8 | 93.6 | 52.8 | 40.5 | 59.6 | 18.3 |
| 150 | 79.0 | 109 | 98.8 | 175 | 99.1 | 73.4 | 106 | 36.6 |
| 180 | 102 | 180 | 130 | 231 | 117 | 103 | 144 | 51.5 |
| 210 | 144 | 209 | 192 | 278 | 173 | 148 | 191 | 49.6 |
| *240* | 200 | 265 | 243 | 348 | 221 | 190 | 244 | 57.6 |
| Values in italics were LLOQ so have been corrected to zero (<50 ng/ml) | | | | | | | | |

Laquinimod cumulative absorption increased throughout the exposure period for each inventive formulation. The results from all three formulation treatments were of the same magnitude and followed the same general pattern. Formulation S4 resulted in the least laquinimod permeation and was the most consistent between different corneas.

Formulation S3 and Formulation S7 resulted in higher laquinimod permeability but the results were more variable between corneas.

EXAMPLE 5

Formulations E2-2A, S3-3A, and S3-8A were prepared, having the pH values and compositions as described in TABLE 57.

TABLE 57

| **Name** | **E2-2A** | **S3-3A** | **S3-8A** |
|---|---|---|---|
| pH | 8.4 | 8.0 | 8.0 |
| Concentration (g/l) | | | |
| Laquinimod | 50 | 50 | 50 |
| Sodium carboxymethylcellulose | 5.50 | 8.00 | 8.00 |
| Carbomer Copolymer (Type B) | | | |
| Kolliphor® HS15 (Macrogol 15 hydroxy stearate) | 2.50 | 2.50 | 2.50 |
| Polysorbate 80 | 25.00 | | |
| Poloxamer 407 | 15.00 | | 2.00 |
| Mannitol | 2.70 | 2.70 | 2.70 |
| Glycerol | 25.0 | 25.0 | 25.0 |
| Na-EDTA | 1.00 | 1.00 | 1.00 |
| Benzalkonium chloride | 0.12 | 0.12 | 0.12 |
| TRIS | 1.21 | 1.21 | 1.21 |
| Castor oil | 50.00 | | |

The viscosity of formulations E2-2A, S3-3A, and S3-8A, measured at 20 °C using the method as described herein above, were found to be 15.7 mPa.s, 21.5 mPa.s, and 23.7 mPa.s, respectively.

EXAMPLE 6

Using the same test system and protocol as in EXAMPLE 4, corneal opacity change and permeability were determined for formulations E2-2A, S3-1, S3-3A, S3-6, S3-7, and S3-8A. In TABLE 58 the results of the pre-dose and post-dose opacity measurements are listed for the different formulations and for a saline control.

TABLE 58

| **Treatment** | **Cornea No.** | **Predose Opacity (lux)** | **Postdose Opacity (lux)** | **Opacity change* (lux)** | **Mean* (lux)** | **SD (lux)** |
|---|---|---|---|---|---|---|
| E2-2A | 1 | 1.72 | 3.03 | -0.26 | -0.40 | 1.11 |
| | 2 | 1.57 | 3.43 | 0.29 | | |
| | 3 | 1.72 | 1.80 | -1.49 | | |
| | 4 | 3.43 | 4.17 | -0.83 | | |
| | 5 | 3.43 | 6.37 | 1.37 | | |
| | 6 | 4.09 | 4.17 | -1.49 | | |

(continued)

| Treatment | Cornea No. | Predose Opacity (lux) | Postdose Opacity (lux) | Opacity change* (lux) | Mean* (lux) | SD (lux) |
|---|---|---|---|---|---|---|
| S3-1 | 7 | 4.85 | 5.82 | -0.60 | 0.44 | 1.10 |
| | 8 | 2.71 | 3.75 | -0.53 | | |
| | 9 | 1.50 | 3.19 | 0.12 | | |
| | 10 | 2.79 | 5.11 | 0.75 | | |
| | 11 | 2.48 | 6.46 | 2.41 | | |
| | 12 | 0.99 | 3.03 | 0.47 | | |
| S3-3A | 13 | 2.17 | 3.75 | 0.01 | 0.10 | 2.65 |
| | 14 | 3.11 | 4.42 | -0.26 | | |
| | 15 | 2.02 | 8.90 | 5.31 | | |
| | 16 | 3.67 | 3.27 | -1.97 | | |
| | 17 | 2.02 | 2.25 | -1.34 | | |
| | 18 | 3.27 | 3.67 | -1.16 | | |
| S3-6 | 19 | 1.70 | 7.26 | 4.00 | 2.74 | 2.36 |
| | 20 | 1.63 | 4.60 | 1.40 | | |
| | 21 | 1.99 | 10.49 | 6.93 | | |
| | 22 | 2.14 | 5.98 | 2.27 | | |
| | 23 | 2.90 | 5.63 | 1.15 | | |
| | 24 | 3.61 | 5.89 | 0.71 | | |
| S3-7 | 25 | 2.75 | 5.02 | 0.70 | -0.24 | 1.25 |
| | 26 | 2.83 | 5.54 | 1.14 | | |
| | 27 | 4.35 | 5.36 | -0.55 | | |
| | 28 | 4.60 | 4.02 | -2.15 | | |
| | 29 | 1.70 | 3.77 | 0.51 | | |
| | 30 | 3.94 | 4.43 | -1.08 | | |
| S3-8A | 31 | 2.67 | 3.29 | -0.95 | 0.87 | 1.44 |
| | 32 | 2.98 | 6.61 | 2.06 | | |
| | 33 | 2.83 | 4.10 | -0.30 | | |
| | 34 | 1.92 | 4.85 | 1.36 | | |
| | 35 | 3.61 | 5.45 | 0.27 | | |
| | 36 | 4.18 | 8.52 | 2.77 | | |
| Saline | 37 | 2.95 | 4.94 | 0.42 | 0.00 | 1.85 |
| | 38 | 5.46 | 3.35 | -3.68 | | |
| | 39 | 3.61 | 5.54 | 0.36 | | |
| | 40 | 2.14 | 5.02 | 1.31 | | |
| | 41 | 3.22 | 5.28 | 0.49 | | |
| | 42 | 3.22 | 5.89 | 1.11 | | |
| * Corrected for negative control opacity change (mean = 1.57) | | | | | | |

As may be seen from the data in TABLE 58, all treatments caused little to no change in opacity; the results were similar to those of the control group. There was little to no evidence of damage detected in corneas treated with any of these formulations. Formulation S3-6 had the highest increase in opacity (2.74 ± 2.36), however this treatment group also showed the most variation with post dose opacity change ranging from 0.71 to 6.93.

The permeability results, in terms of cumulative absorption of laquinimod (μg/$cm^2$) across the bovine cornea (n = 6) following exposure to formulations E2-2A, S3-1, S3-3A, S3-6, S3-7, and S3-8A, respectively, are listed in TABLES 59 to 64 and represented in Figures 9-14.

TABLE 59

| **Cumulative absorption of laquinimod (μg/$cm^2$) following exposure to E2-2A** | | | | | | | | |
|---|---|---|---|---|---|---|---|---|
| Time (min) | Cornea 1 | Cornea 2 | Cornea 3 | Cornea 4 | Cornea 5 | Cornea 6 | Mean | SD |
| 0 | *0.00* | *0.00* | *0.00* | *0.00* | *0.00* | *0.00* | *0.00* | *0.00* |
| 30 | *0.00* | 0.328 | *0.00* | *0.00* | *0.00* | *0.00* | *0.0547* | *0.134* |
| 60 | 1.99 | 6.26 | 1.48 | 1.13 | 4.12 | 1.77 | 2.79 | 2.00 |
| 90 | 15.2 | 28.0 | 11.1 | 6.01 | 17.8 | 8.79 | 14.5 | 7.86 |
| 120 | 24.0 | 44.3 | 25.6 | 11.5 | 31.4 | 20.0 | 26.1 | 11.1 |
| 150 | 37.2 | 62.9 | 38.4 | 19.8 | 48.5 | 28.3 | 39.2 | 15.1 |
| 180 | 57.2 | 103 | 60.5 | 27.8 | 73.4 | 42.3 | 60.7 | 26.0 |
| 210 | 80.2 | 136 | 92.1 | 46.3 | 106 | 53.8 | 85.8 | 33.5 |
| *240* | 101 | 163 | 104 | 56.1 | 135 | 72.5 | 105 | 39.2 |
| Values in italics were LLOQ so have been corrected to zero (<50 ng/ml) | | | | | | | | |

TABLE 60

| **Cumulative absorption (μg/$cm^2$) following exposure to S3-1** | | | | | | | | |
|---|---|---|---|---|---|---|---|---|
| Time (min) | Cornea 7 | Cornea 8 | Cornea 9 | Cornea 10 | Cornea 11 | Cornea 12 | Mean | SD |
| 0 | *0.00* | *0.00* | *0.00* | *0.00* | *0.00* | *0.00* | *0.00* | *0.00* |
| 30 | *0.00* | *0.00* | *0.00* | *0.00* | *0.00* | *0.00* | *0.00* | *0.00* |
| 60 | 2.20 | 2.64 | 7.58 | 1.51 | 0.607 | 0.689 | 2.54 | 2.60 |
| 90 | 16.5 | 12.6 | 26.2 | 16.1 | 10.5 | 9.19 | 15.2 | 6.13 |
| 120 | 32.8 | 25.0 | 55.6 | 30.6 | 20.1 | 16.9 | 30.2 | 13.8 |
| 150 | 56.1 | 40.0 | 76.4 | 46.4 | 33.9 | 29.2 | 47.0 | 17.2 |
| 180 | 63.8 | 59.0 | 93.1 | 59.1 | 46.7 | 46.0 | 61.3 | 17.2 |
| 210 | 110 | 86.6 | 127 | 102 | 75.8 | 68.5 | 95.0 | 22.1 |
| *240* | 149 | 108 | 175 | 139 | 92.6 | 85.6 | 125 | 35.0 |
| Values in italics were LLOQ so have been corrected to zero (<50 ng/ml) | | | | | | | | |

TABLE 61

| **Cumulative absorption of laquinimod (μg/$cm^2$) following exposure to S3-3A** | | | | | | | | |
|---|---|---|---|---|---|---|---|---|
| Time (min) | Cornea 13 | Cornea 14 | Cornea 15 | Cornea 16 | Cornea 17 | Cornea 18 | Mean | SD |
| 0 | *0.00* | *0.00* | *0.00* | *0.00* | *0.00* | *0.00* | *0.00* | *0.00* |
| 30 | *0.00* | 0.324 | 0.240 | *0.00* | *0.00* | *0.00* | *0.0940* | *0.148* |
| 60 | 2.86 | 8.25 | 7.89 | 4.25 | 2.71 | 3.71 | 4.94 | 2.49 |
| 90 | 14.2 | 24.8 | 30.9 | 18.3 | 13.0 | 26.8 | 21.3 | 7.27 |
| 120 | 26.3 | 45.5 | 61.0 | 32.3 | 24.0 | 38.3 | 37.9 | 13.8 |
| 150 | 36.9 | 67.3 | 85.4 | 49.8 | 40.1 | 60.3 | 56.6 | 18.2 |
| 180 | 56.5 | 115 | 133 | 67.6 | 53.6 | 91.8 | 86.2 | 32.6 |
| 210 | 78.9 | 163 | 179 | 101 | 85.9 | 130 | 123 | 41.5 |

(continued)

| Cumulative absorption of laquinimod (μg/cm²) following exposure to S3-3A | | | | | | | | |
|---|---|---|---|---|---|---|---|---|
| Time (min) | Cornea 13 | Cornea 14 | Cornea 15 | Cornea 16 | Cornea 17 | Cornea 18 | Mean | SD |
| *240* | 103 | 214 | 231 | 131 | 107 | 158 | 157 | 54.6 |
| Values in italics were LLOQ so have been corrected to zero (<50 ng/ml) | | | | | | | | |

TABLE 62

| Cumulative absorption of laquinimod (μg/cm²) following exposure to S3-6 | | | | | | | | |
|---|---|---|---|---|---|---|---|---|
| Time (min) | Cornea 19 | Cornea 20 | Cornea 21 | Cornea 22 | Cornea 23 | Cornea 24 | Mean | SD |
| 0 | *0.00* | *0.00* | *0.00* | *0.00* | *0.00* | *0.00* | *0.00* | *0.00* |
| 30 | 0.247 | *0.00* | *0.00* | *0.00* | *0.00* | *0.00* | *0.0411* | *0.0919* |
| 60 | 3.95 | 0.811 | 2.60 | 1.20 | 0.599 | 0.991 | 1.69 | 1.31 |
| 90 | 22.4 | 11.9 | 17.2 | 10.7 | 11.5 | 8.30 | 13.7 | 5.17 |
| 120 | 37.1 | 25.0 | 34.7 | 17.6 | 19.2 | 19.1 | 25.4 | 8.51 |
| 150 | 66.8 | 48.4 | 65.5 | 41.1 | 36.8 | 42.5 | 50.2 | 12.9 |
| 180 | 90.4 | 70.8 | 93.8 | 57.9 | 50.9 | 56.1 | 70.0 | 18.4 |
| 210 | 129 | 99.8 | 124 | 74.0 | 69.1 | 72.9 | 94.8 | 26.9 |
| *240* | 176 | 123 | 172 | 91.1 | 99.7 | 95.8 | 126 | 38.6 |
| Values in italics were LLOQ so have been corrected to zero (<50 ng/ml) | | | | | | | | |

TABLE 63

| Cumulative absorption of laquinimod (μg/cm²) following exposure to S3-7 | | | | | | | | |
|---|---|---|---|---|---|---|---|---|
| Time (min) | Cornea 25 | Cornea 26 | Cornea 27 | Cornea 28 | Cornea 29 | Cornea 30 | Mean | SD |
| 0 | *0.00* | *0.00* | *0.00* | *0.00* | *0.00* | *0.00* | *0.00* | *0.00* |
| 30 | *0.00* | *0.00* | *0.00* | *0.00* | *0.00* | *0.00* | *0.00* | *0.00* |
| 60 | 1.09 | 0.570 | 1.32 | 0.313 | 1.00 | 0.990 | 0.881 | 0.369 |
| 90 | 11.4 | 9.61 | 11.2 | 6.09 | 10.1 | 10.7 | 9.85 | 1.96 |
| 120 | 27.9 | 20.4 | 23.4 | 14.4 | 18.4 | 20.1 | 20.8 | 4.58 |
| 150 | 49.1 | 38.4 | 43.0 | 32.0 | 38.4 | 42.9 | 40.6 | 5.76 |
| 180 | 69.1 | 53.7 | 58.4 | 43.8 | 57.0 | 61.5 | 57.2 | 8.41 |
| 210 | 97.2 | 76.8 | 78.5 | 68.7 | 98.4 | 85.5 | 84.2 | 11.8 |
| *240* | 127 | 104 | 102 | 88.2 | 111 | 110 | 107 | 12.7 |
| Values in italics were LLOQ so have been corrected to zero (<50 ng/ml) | | | | | | | | |

TABLE 64

| Cumulative absorption of laquinimod (μg/cm²) following exposure to S3-8A | | | | | | | | |
|---|---|---|---|---|---|---|---|---|
| Time (min) | Cornea 31 | Cornea 32 | Cornea 33 | Cornea 34 | Cornea 35 | Cornea 36 | Mean | SD |
| 0 | *0.00* | *0.00* | *0.00* | *0.00* | *0.00* | *0.00* | *0.00* | *0.00* |
| 30 | *0.00* | 0.315 | *0.00* | *0.00* | *0.00* | 0.124 | *0.0733* | *0.129* |
| 60 | 1.01 | 2.68 | 1.05 | 0.762 | 1.89 | 5.29 | 2.11 | 1.71 |
| 90 | 12.2 | 22.6 | 11.4 | 13.9 | 15.8 | 21.3 | 16.2 | 4.72 |
| 120 | 26.6 | 40.9 | 20.6 | 23.0 | 30.5 | 46.5 | 31.3 | 10.3 |
| 150 | 44.0 | 73.5 | 41.1 | 48.3 | 57.6 | 75.4 | 56.7 | 14.9 |
| 180 | 62.3 | 93.3 | 56.9 | 61.9 | 77.2 | 101 | 75.5 | 18.3 |

(continued)

| Cumulative absorption of laquinimod ($\mu g/cm^2$) following exposure to S3-8A | | | | | | | | |
|---|---|---|---|---|---|---|---|---|
| Time (min) | Cornea 31 | Cornea 32 | Cornea 33 | Cornea 34 | Cornea 35 | Cornea 36 | Mean | SD |
| 210 | 83.5 | 149 | 79.1 | 94.2 | 113 | 153 | 112 | 32.3 |
| *240* | 118 | 165 | 112 | 124 | 131 | 183 | 139 | 28.9 |
| Values in italics were LLOQ so have been corrected to zero (<50 ng/ml) | | | | | | | | |

EXAMPLE 7

Using the same test system and protocol as in EXAMPLE 4, corneal opacity change and permeability across bovine corneas were determined for formulations S3-3A and S7-1, and for laquinimod 50 mg/ml in saline (PBS) at pH 8, in vitro, after a single 4 h exposure.

Six replicate corneas were treated with each formulation or saline (negative control) for a 4 h exposure period. Permeability samples were collected from test item treated corneas only, immediately prior to dosing and at half hourly intervals following dosing. The final sample was collected at 4 h post dose (9 samples were collected per cornea in total). The opacity of corneas was measured prior to dosing and following the final permeability sample collection. The concentration of the active ingredient was measured in the permeation samples collected throughout the exposure period by LC-MS/MS. The results of opacity measurements pre- and post-dose are shown in TABLE 65.

**TABLE 65**

| Treatment | Cornea Number | Predose Opacity | Postdose Opacity | Corrected Opacity Change* | Mean | SD |
|---|---|---|---|---|---|---|
| Laquinimod in PBS | 1 | 5.87 | 7.94 | -0.60 | -2.20 | 0.85 |
| | 2 | 5.62 | 5.70 | -2.59 | | |
| | 3 | 5.62 | 6.20 | -2.09 | | |
| | 4 | 5.62 | 5.21 | -3.08 | | |
| | 5 | 5.70 | 6.11 | -2.26 | | |
| | 6 | 6.11 | 6.20 | -2.59 | | |
| S3-3A | 7 | 6.03 | 8.67 | -0.03 | -0.81 | 1.82 |
| | 8 | 4.58 | 8.76 | 1.51 | | |
| | 9 | 6.62 | 10.01 | 0.72 | | |
| | 10 | 4.27 | 5.05 | -1.89 | | |
| | 11 | 4.98 | 4.43 | -3.22 | | |
| | 12 | 5.70 | 6.45 | -1.92 | | |
| S7-1 | 13 | 6.03 | 6.20 | -2.50 | -0.97 | 1.22 |
| | 14 | 5.29 | 8.67 | 0.70 | | |
| | 15 | 6.45 | 7.76 | -1.37 | | |
| | 16 | 5.13 | 8.12 | 0.31 | | |
| | 17 | 4.66 | 5.78 | -1.55 | | |
| | 18 | 5.13 | 6.37 | -1.44 | | |

(continued)

| Treatment | Cornea Number | Predose Opacity | Postdose Opacity | Corrected Opacity Change* | Mean | SD |
|---|---|---|---|---|---|---|
| Saline | 19 | 5.78 | 6.62 | -1.83 | 0.00 | 1.82 |
| | 20 | 5.78 | 11.42 | 2.97 | | |
| | 21 | 5.21 | 7.40 | -0.49 | | |
| | 22 | 4.43 | 5.29 | -1.80 | | |
| | 23 | 4.20 | 7.76 | 0.89 | | |
| | 24 | 4.82 | 7.76 | 0.27 | | |
| * Corrected for negative control opacity change (mean = 2.67) | | | | | | |

The results presented in TABLE 65 show that all treatments caused little to no change in opacity.

The permeability results, in terms of cumulative absorption of laquinimod (μg/cm$^2$) across the bovine cornea (n = 6) following exposure to formulations S3-3A and S71, and to laquinimod in saline, respectively, are listed in TABLES 66 to 69 and illustrated in Figures 15-18.

TABLE 66

| **Cumulative absorption of laquinimod (μg/cm$^2$) following exposure to laquinimod in PBS** | | | | | | | | |
|---|---|---|---|---|---|---|---|---|
| Time (h) | Cornea 1 | Cornea 2 | Cornea 3 | Cornea 4 | Cornea 5 | Cornea 6 | Mean | SD |
| 0.0 | *0.00* | *0.00* | *0.00* | *0.00* | *0.00* | *0.00* | *0.00* | *0.00* |
| 0.5 | *0.00* | *0.00* | *0.00* | *0.00* | *0.00* | *0.00* | *0.00* | *0.00* |
| 1.0 | 0.692 | 1.19 | 1.32 | 0.705 | 0.956 | 1.14 | 1.00 | 0.263 |
| 1.5 | 5.03 | 6.87 | 6.26 | 4.86 | 6.39 | 6.16 | 5.93 | 0.803 |
| 2.0 | 11.6 | 18.1 | 18.4 | 14.8 | 17.5 | 17.1 | 16.3 | 2.59 |
| 2.5 | 20.2 | 30.6 | 27.0 | 22.9 | 31.9 | 27.4 | 26.7 | 4.47 |
| 3.0 | 30.2 | 50.3 | 48.4 | 35.0 | 44.4 | 42.9 | 41.9 | 7.83 |
| 3.5 | 40.9 | 68.3 | 62.2 | 57.6 | 67.1 | 63.7 | 60.0 | 10.1 |
| 4.0 | 52.9 | 85.6 | 80.6 | 68.7 | 83.8 | 75.4 | 74.5 | 12.2 |
| Values in italics were LLOQ so have been included as zero (<50 ng/ml) | | | | | | | | |

**TABLE 67**

| **Cumulative absorption (μg/cm$^2$) following exposure to S3-3A** | | | | | | | | |
|---|---|---|---|---|---|---|---|---|
| Time (h) | Cornea 7 | Cornea 8 | Cornea 9 | Cornea 10 | Cornea 11 | Cornea 12 | Mean | SD |
| 0.0 | *0.00* | *0.00* | *0.00* | *0.00* | *0.00* | *0.00* | *0.00* | *0.00* |
| 0.5 | *0.00* | *0.00* | *0.00* | *0.00* | *0.00* | *0.00* | *0.00* | *0.00* |
| 1.0 | 2.26 | 2.38 | 2.22 | 2.29 | 2.61 | 2.91 | 2.45 | 0.266 |
| 1.5 | 11.6 | 10.2 | 11.4 | 13.7 | 12.8 | 12.8 | 12.1 | 1.27 |
| 2.0 | 27.5 | 26.2 | 28.4 | 31.3 | 27.8 | 33.2 | 29.1 | 2.62 |
| 2.5 | 46.8 | 39.6 | 49.0 | 49.5 | 43.9 | 50.7 | 46.6 | 4.19 |
| 3.0 | 72.1 | 59.7 | 77.3 | 77.0 | 64.3 | 73.6 | 70.7 | 7.15 |
| 3.5 | 97.3 | 88.1 | 108 | 108 | 91.3 | 106 | 99.7 | 8.76 |
| 4.0 | 126 | 111 | 140 | 129 | 109 | 128 | 124 | 12.0 |
| Values in italics were LLOQ so have been included as zero (<50 ng/ml) | | | | | | | | |

TABLE 68

| Cumulative absorption of laquinimod ($\mu g/cm^2$) following exposure to S7-1 | | | | | | | | |
|---|---|---|---|---|---|---|---|---|
| Time (h) | Cornea 13 | Cornea 14 | Cornea 15 | Cornea 16 | Cornea 17 | Cornea 18 | Mean | SD |
| 0.0 | *0.00* | *0.00* | *0.00* | *0.00* | *0.00* | *0.00* | *0.00* | *0.00* |
| 0.5 | 0.463 | 0.337 | 0.865 | 0.269 | 0.738 | 0.164 | 0.473 | 0.276 |
| 1.0 | 8.99 | 5.82 | 14.1 | 7.37 | 5.99 | 6.08 | 8.06 | 3.20 |
| 1.5 | 30.7 | 26.8 | 50.1 | 24.0 | 21.3 | 20.9 | 29.0 | 11.0 |
| 2.0 | 57.0 | 56.3 | 94.6 | 54.0 | 52.4 | 53.5 | 61.3 | 16.4 |
| 2.5 | 79.9 | 89.3 | 134 | 89.4 | 86.5 | 83.6 | 93.9 | 20.2 |
| 3.0 | 121 | 125 | 178 | 130 | 119 | 99.1 | 129 | 26.4 |
| 3.5 | 157 | 166 | 248 | 184 | 161 | 150 | 178 | 36.3 |
| 4.0 | 197 | 214 | 265 | 209 | 201 | 187 | 212 | 27.3 |
| Values in italics were LLOQ so have been included as zero (<50 ng/ml) | | | | | | | | |

**TABLE 69**

| Mean Cumulative absorption of laquinimod ($\mu g/cm^2$) following exposure to PBS, S3-3A and S7-1 | | | | | | |
|---|---|---|---|---|---|---|
| Time (h) | Formulation in PBS | | Formulation S3-3A | | Formulation S7-1 | |
| | Mean | SD | Mean | SD | Mean | SD |
| 0.0 | *0.00* | *0.00* | *0.00* | *0.00* | *0.00* | *0.00* |
| 0.5 | *0.00* | *0.00* | *0.00* | *0.00* | 0.473 | 0.276 |
| 1.0 | 1.00 | 0.263 | 2.45 | 0.266 | 8.06 | 3.20 |
| 1.5 | 5.93 | 0.803 | 12.1 | 1.27 | 29.0 | 11.0 |
| 2.0 | 16.3 | 2.59 | 29.1 | 2.62 | 61.3 | 16.4 |
| 2.5 | 26.7 | 4.47 | 46.6 | 4.19 | 93.9 | 20.2 |
| 3.0 | 41.9 | 7.83 | 70.7 | 7.15 | 129 | 26.4 |
| 3.5 | 60.0 | 10.1 | 99.7 | 8.76 | 178 | 36.3 |
| 4.0 | 74.5 | 12.2 | 124 | 12.0 | 212 | 27.3 |
| Values in italics were LLOQ so have been included as zero (<50 ng/ml) | | | | | | |

The results presented in TABLES 66-69 show that laquinimod cumulative absorption increased throughout the exposure period for each formulation. At 4 h post dose, the formulations could be ranked in the following order from greatest to least permeability: Formulation S7-1 > Formulation S3-3A > Laquinimod in PBS.

EXAMPLE 8

The efficacy of the inventive formulation was tested *in vivo* in a mouse model of uveitis.

*Experimental conditions*

Female B10.RIII mice (-6 weeks of age at the beginning of the study) were immunised with an emulsion containing the interphotoreceptor retinoid binding protein peptide 161-180 (IRBP 161-180) in Incomplete Freund's adjuvant (IFA) supplemented with *Mycobacterium tuberculosis* H37Ra on Day 0.

Oral laquinimod was prepared in a saline solution. The ophthalmic formulation was given as eye drops. The composition of the ophthalmic formulation was S3-3A (containing 50 mg/ml laquinimod) and the corresponding vehicle control was the S3-3A formulation without laquinimod. Treatments were administered according to the schedule shown in TABLE 70.

TABLE 70

| Groups | | Treatments | | |
|---|---|---|---|---|
| | | Dose | Route | Regimen |
| 1 (n = 10) | Vehicle | N/A | Both eyes: Topical S3-3A (without laquinimod) | BID, Day 0 - 21 |
| 2 (n = 9) | Vehicle oral gavage (water) | N/A | PO gavage (without laquinimod) | SID, Day 0 - 21 |
| 3 (n = 8) | Laquinimod | 25 mg/kg | PO gavage | SID, Day 0 - 21 |
| 4 (n = 10) | Laquinimod | 10 mg/kg | Both eyes: Topical S3-3A | BID, Day 0 - 21 |
| 5 (n = 9) | Laquinimod | 10 mg/kg<br>25 mg/kg | Both eyes: Topical S3-3A<br>PO gavage | BID Day 0 - 21<br>SID Day 0 - 21 |
| PO - Peroral; SID - once daily; BID - twice daily | | | | |

From Day 0 until the end of the experiment, animals were scored weekly for clinical signs of uveitis. Retinal images were captured using topical endoscopy fundus imaging (TEFI) in non-anaesthetised but restrained animals following pupil dilatation using tropicamide then phenylephrine hydrochloride.

Retinal images were scored using the scoring system described TABLE 71 with a maximum possible score of 20 per eye.

TABLE 71

| Score | Criteria |
|---|---|
| Optic disc | |
| 1 | Minimal inflammation |
| 2 | Mild inflammation |
| 3 | Moderate inflammation |
| 4 | Severe inflammation |
| 5 | Not visible (white-out or severe detachment) |
| Retinal vessels | |
| 1 | 1-4 mild cuffings |
| 2 | >4 mild cuffings or 1-3 moderate cuffings |
| 3 | >3 moderate cuffings |
| 4 | > 1 severe cuffings |
| 5 | Not visible (white-out or severe detachment) |
| | Retinal tissue infiltration |
| 1 | 1-4 small lesions or 1 linear lesion |
| 2 | 5-10 small lesions or 2-3 linear lesions |
| 3 | >10 small lesions or >3 linear lesions |
| 4 | Linear lesion confluent |
| 5 | Not visible (white-out or severe detachment) |

(continued)

| | Structural damage |
|---|---|
| 1 | Retinal lesions or atrophy involving ¼ to ¾ of retinal area |
| 2 | Panretinal atrophy with multiple small lesions (scars) or ≤3 linear lesions (scars) |
| 3 | Panretinal atrophy with >3 linear lesions or confluent lesions (scars) |
| 4 | Retinal detachment with folding |
| 5 | Not visible (white-out or severe detachment) |

*Results*

Analysis of the total posterior uveitis clinical signs obtained by summing up the clinical scores observed in the left (L) and right (R) eye of each animal in every experimental group, revealed a significant effect of time, thereby confirming that the disease was successfully induced, and a general effect of all the treatments in comparison to the two vehicle-treated groups.

Given the presence of two vehicles in the study design, Dunnett's multiple comparisons were performed vs. vehicle, topical, and again vs. vehicle, oral. The calculated p-values at 14, 17 and 20 days of treatment (D14, D17 and D20) are presented in TABLE 72.

TABLE 72

| **Dunnett's test (vs. vehicle, topical)** | **p-value** | | |
|---|---|---|---|
| | **D14** | **D17** | **D20** |
| Vehicle (topical) vs. laquinimod (topical) | <0.0001 | <0.0001 | <0.0001 |
| Vehicle (topical) vs. laquinimod (topical + oral) | <0.0001 | <0.0001 | 0.0001 |
| **Dunnett's test (vs. vehicle, oral)** | **p-value** | | |
| | **D14** | **D17** | **D20** |
| Vehicle (oral) vs. laquinimod (oral - 25 mg/Kg) | 0.0030 | 0.0012 | 0.0035 |
| Vehicle (oral) vs. laquinimod (topical + oral) | 0.0031 | 0.0013 | 0.0038 |

No differences were observed between the two vehicle groups. Administration of the ophthalmic formulation of laquinimod onto the eye significantly inhibited the disease course in comparison to control animals treated with vehicle only (Figure 19).

EXAMPLE 9

A gel formulation was prepared (total batch size of 50.0 l) containing 10.62 g/l of laquinimod sodium (10 g/l laquinimod base) and excipients as indicated in TABLE 73.

TABLE 73

| **Supplier** | **Supplier code** | **Component** | **Conc. g/l** |
|---|---|---|---|
| Lubrizol | PEM1005A | Carbomer copolymer (type B) Pemulen™ TR-1 | 1.65 |
| Ashland | Aqualon CMC 7MF PH BET | Sodium carboxymethylcellulose | 3.00 |
| Croda | SR48833 | Polysorbate 80 | 1.00 |
| Roquette | 450001 D-Pearlitol 160C | Mannitol | 2.70 |

(continued)

| Supplier | Supplier code | Component | Conc. g/l |
|---|---|---|---|
| Brenntag Quimica | Edenor G 99.8PH 10103102 | Glycerol | 17.05 |
| Merck | 1.37004 | Disodium edetate | 1.00 |
| Merck | 1.37124 | Benzalkonium chloride solution (50 % w/v) | 0.12 |
| Merck | 1.37036 | Disodium hydrogen phosphate dihydrate | 1.78 |
| Panreac | 192415 | Sodium hydroxide 1N | qs pH 7.4 (17.50-22.50) |
| NA | NA | Water for injection | qs 1 l |
| NA = not applicable | | | |

The obtained formulation was an opalescent gel, which was tested for osmolality, pH, viscosity, and relative density. The methods and results are as shown in TABLE 74.

TABLE 74

| Tested feature | Test method | Result |
|---|---|---|
| Osmolality | Ph. Eur. 2.2.35 | 315 mOsm/kg |
| pH | Ph. Eur. 2.2.3 | 7.41 |
| Viscosity | Ph. Eur. 2.2.8 | 30 mPas |
| Relative density | Ph. Eur. 2.2.5 | 1.015 |

When tested for drop size, drops of about 34 µl in volume were obtained.

EXAMPLE 10

The formulation of EXAMPLE 9 was filled into 5-ml bottles (LDPE bottles from Nemera La Verpilliera, (reference No. 20059681), equipped with nozzle and cap Pureflow® 200 (reference No. 20060322), for dispensing as an eye drop formulation. In total, 130 bottles were prepared, each bottle containing 5 ml of formulation.

EXAMPLE 11

The formulation of EXAMPLE 9 was submitted to a stability test in accordance with the ICH guide Q1A (R2) at the conditions established for drug products packaged in semi-permeable containers, whereby the long-term storage conditions were 2 °C to 8 °C and the accelerated conditions were 25 °C/40 % RH. The results, in terms of droplet volume, osmolality, pH, viscosity, and laquinimod assay, after a storage time t of 0, 2, 3, and 6 months are presented in TABLES 75 and 76.

TABLE 75

| Storage conditions: 2-8 °C | | | | |
|---|---|---|---|---|
| **Tested feature** | **t = 0 month** | **t = 2 months** | **t = 3 months** | **t = 6 months** |
| Droplet volume (µl) | 34 | 35 | 32 | 33 |
| Osmolality (mOsm/kg) | 315 | 298 | 292 | 298 |
| pH | 7.41 | 7.46 | 7.39 | 7.33 |
| Viscosity (mPas) | 30 | 24 | 24 | 25 |
| Laquinimod assay (%) | 99.0 | 100.7 | 99.9 | 101.4 |

TABLE 76

| Storage conditions: 25 ± 2 °C / 40 ± 5 % RH | | | | |
|---|---|---|---|---|
| **Tested feature** | **t = 0 month** | **t = 2 months** | **t = 3 months** | **t = 6 months** |
| Droplet volume (μl) | 34 | 36 | 32 | 33 |
| Osmolality (mOsm/kg) | 315 | 302 | 302 | 298 |
| pH | 7.41 | 7.37 | 7.38 | 7.33 |
| Viscosity (mPas) | 30 | 24 | 23 | 25 |
| Laquinimod assay (%) | 99.0 | 101.2 | 100.9 | 101.4 |

The formulation was visually observed at the different time points and remained an opalescent gel throughout the entire test period. As may be seen from the above TABLES 75 and 76 the inventive formulation shows no significant change in any of the tested features under either of the studied conditions. Based on the obtained stability data, therefore, a shelf-life of at least 6 months, more preferably at least 8 months, even more preferably at least 9 months, and most preferably at least 12 months, is contemplated for the inventive formulation when stored at 2°C to 8°C.

EXAMPLE 12

A gel formulation was prepared, having pH 7.4 and ingredients as indicated in TABLE 77.

TABLE 77

| **Ingredient** | **g/l** |
|---|---|
| Laquinimod | 10 |
| Carbomer Copolymer (Type B) | 1.65 |
| Sodium carboxymethylcellulose | 3.00 |
| Polysorbate 80 | 1.0 |
| Mannitol | 2.7 |
| Glycerol | 25.0 |
| Na-EDTA | 1.0 |
| Benzalkonium chloride | 0.12 |
| Disodium hydrogen phosphate dihydrate | 1.78 |

The viscosity of the formulation represented in TABLE 77 was measured at 20 °C using the method as described herein above and was found to be 17.3 mPa.s.

EXAMPLE 13

Using the formulation of EXAMPLE 12, a study was performed to determine the distribution in the eye of laquinimod following topical ocular administration to rabbits after single and repeated administrations. Briefly, the study was performed over a test period of 10 days using 7 male New Zealand white rabbits, weighing ca 2.3-3.0 kg at time of dosing. Animals were housed and maintained according to established procedures and had free access to tap water and Teklad Irradiated Certified Global Rabbit Diet® (Envigo, UK) throughout the duration of the study. The tested formulation was stored at 4 °C until use.

Of the 7 test animals, 6 were given the formulation of EXAMPLE 12 and 1 animal was given the same amount of a placebo formulation, having the same composition as the formulation of EXAMPLE 12 except for not containing any laquinimod. Each animal was treated as follows: On day 1, one topical ocular dose was administered to both eyes; on days 2 and 3, no dose was administered; on days 4-9, three topical ocular doses were administered to each eye, 2 hours apart; and on day 10, one topical ocular dose was administered to each eye. For the dosing, the animals were removed from their housing and appropriately restrained. Then, using a pipette, the topical ocular dose was administered directly

onto the cornea of each eye of each animal. The dose was 30 μl for each eye at each dosing session. Following dosing, the animals were returned to their housing. Details of the study are summarized in TABLE 78.

TABLE 78

| No. of animals | Test item | Dose (mg/eye) | Conc. (mg/ml) | Volume (ml/eye) |
|---|---|---|---|---|
| 6 | EXAMPLE 12 | 0.3 | 10 | 0.030 |
| 1 | Placebo | 0 | 0 | 0.030 |

On day 10, the animals were euthanized at 30 min, 1 h, 2 h, 4 h, and 8 h respectively after the last topical ocular administration, and immediately thereafter, the eyes were quickly enucleated, along with the surrounding eyelid tissue, embedded in tragacanth gum and snap frozen in isopentane chilled in dry ice. The resultant isolated eyes were stored in a freezer set to maintain a temperature of ≤ -65°C.

For the distribution study, the eyes (right hand eye from each animal) were sectioned with a cryostat at a temperature of -20 °C. For each tissue, sections having a thickness of 10 μm were collected on indium-tin oxide-coated (ITO) glass slides for mass spectrometry imaging (MSI). The analyses were performed using matrix assisted laser desorption/ionisation with Fourier transform ion cyclotron resonance (MALDI-FTICR) on a SolariX mass spectrometer from Bruker Daltonix. All the analyses were run at a spatial resolution of 60 μm and using the following mass spectrometer parameters:

- Mode: CASI (continuous accumulation of selected ions)
- Ionization: Positive
- Mass range: 0-1000 Dalton
- Laser frequency: 2000 Hz
- Calibration mode: 2

The monitored m/z for laquinimod was 357.10 ($[M+H]^+$).

The results are summarized in TABLE 79.

TABLE 79

| Sampling time | Region of interest | QMSI (μg/g) | QMSI (μM) |
|---|---|---|---|
| 30 min | Retina/choroid<br>Sclera<br>Cornea<br>Outer tissue | BLOD<br>BLOD<br>87.04<br>27.94 | BLOD<br>BLOD<br>243.93<br>78.32 |
| 1 h | Retina/choroid<br>Sclera<br>Cornea<br>Outer tissue | BLOD<br>BLOD<br>10.84<br>8.04 | BLOD<br>BLOD<br>30.37<br>22.53 |
| 2 h | Retina/choroid<br>Sclera<br>Cornea<br>Outer tissue | BLOD<br>BLOD<br>BLLOQ<br>BLLOQ | BLOD<br>BLOD<br>BLLOQ<br>BLLOQ |
| 4 h | Retina/choroid<br>Sclera<br>Cornea<br>Outer tissue | 3.70<br>BLLOQ<br>4.82<br>4.17 | 10.37<br>BLLOQ<br>13.5<br>11.7 |

(continued)

| Sampling time | Region of interest | QMSI (μg/g) | QMSI (μM) |
|---|---|---|---|
| 8h | Retina/choroid<br>Sclera<br>Cornea<br>Outer tissue | 3.52<br>4.99<br>5.81<br>8.59 | 9.87<br>13.99<br>16.30<br>24.07 |
| QMSI - Quantitative Mass Spectrometric Imaging, LOD - Limit of Detection level (1.23 μM), BLOD - Below Limit of Detection Level, LLOQ - Lower Limit of Quantification (3.9 μM), BLLOQ - (Below Lower Limit of Quantification Level) | | | |

The results in TABLE 79 show that after topical ocular treatment with the formulation of EXAMPLE 12, laquinimod was detected at a high concentration in the cornea and outer tissue after 30 minutes. The concentration of laquinimod in the cornea and outer tissue after 4 and 8 hours was lower, whereas at these later timepoints laquinimod could be detected in the posterior parts of the eyes, viz. the retina/choroid and sclera.

## Claims

**1.** A formulation for ocular administration comprising, in an aqueous phase:

(i) a therapeutically effective amount of laquinimod or a pharmaceutically acceptable salt thereof as active ingredient,
(ii) a pharmaceutically acceptable viscosity agent in an amount sufficient to provide a dynamic viscosity of 2 to 200 mPas, as measured at 20 °C,
(iii) a pharmaceutically acceptable tonicity adjusting agent, in an amount sufficient to provide an osmolality of 200 to 600 mOsm/kg,
(iv) a pharmaceutically acceptable humectant,
(v) a pharmaceutically acceptable antioxidant, and
(vi) a pharmaceutically acceptable pH regulating agent.

**2.** The formulation of claim 1, wherein the pharmaceutically acceptable viscosity agent is present in an amount sufficient to provide a dynamic viscosity of 5 to 100 mPas, preferably 10 to 50 mPas, more preferably 15 to 45 mPas, as measured at 20 °C.

**3.** The formulation of claim 1 or 2, wherein the pharmaceutically acceptable viscosity agent comprises one or more of the group consisting of polyvinyl alcohol, poly(acrylic acid) homo- or copolymers (carbomers), polyvinylpyrrolidone, and cellulose derivatives, such as hydroxypropylmethylcellulose and sodium carboxymethyl cellulose.

**4.** The formulation of any one of claims 1 to 3, wherein the pharmaceutically acceptable tonicity adjusting agent is present in an amount sufficient to provide an osmolality of 200 to 500 mOsm/kg, preferably 200 to 400 mOsm/kg, more preferably 250 to 375 mOsm/kg.

**5.** The formulation of any one of claims 1 to 4, wherein the pharmaceutically acceptable tonicity adjusting agent is a non-ionic tonicity adjusting agent, such as mannitol.

**6.** The formulation of any one of claims 1 to 5, wherein the pharmaceutically acceptable humectant is a polyol, preferably a C3-C6 polyol, more preferably glycerol.

**7.** The formulation of any one of claims 1 to 6, wherein the pharmaceutically acceptable pH regulating agent is present in an amount sufficient to provide a pH in the range of 6.8 to 8.5, preferably in the range of 6.8 to 8.0.

**8.** The formulation of any one of claims 1 to 7, further comprising (vii) a pharmaceutically acceptable preservative, such as benzalkonium chloride.

**9.** The formulation of any one of claims 1 to 8, further comprising (viii) a pharmaceutically acceptable surfactant, such

as a nonionic surfactant.

**10.** The formulation of any one of claims 1 to 9, further comprising (ix) a pharmaceutically acceptable solubilizing agent.

**11.** The formulation of any one of claims 1 to 10, wherein

the pharmaceutically acceptable viscosity agent is present in an amount sufficient to provide a dynamic viscosity of 10 to 45 mPas, as measured at 20 °C;
the pharmaceutically acceptable tonicity adjusting agent is present in an amount sufficient to provide an osmolality of 200 to 400 mOsm/kg; and
the pharmaceutically acceptable pH regulating agent is present in an amount sufficient to provide a pH of 6.8 to 8.0.

**12.** The formulation of any one of claims 1 to 11, in the form of a gel.

**13.** The formulation of claim 12, comprising:

(i) 5 to 100 g/l of laquinimod, or a corresponding amount of a pharmaceutically acceptable salt of laquinimod;
(ii) 1 to 10 g/l of sodium carboxymethylcellulose, and/or 0.5 to 6 g/l of a carbomer copolymer;
(iii) 1.5 to 4 g/l of mannitol;
(iv) 10 to 40 g/l of glycerol;
(v) 0.2 to 2 g/l of Na-EDTA; and
(vi) a pH buffering agent in an amount effective to provide a pH in the range of 6.8 to 8.0.

**14.** The formulation of claim 13, further comprising 0.1 to 5 g/l of polysorbate.

**15.** The formulation of any one of claims 1 to 11, in the form of a water and oil containing emulsion, preferably an oil-in-water emulsion.

**16.** The formulation of any one of claims 1 to 15, wherein the ocular administration is topical ocular administration.

**17.** The formulation of any one of claims 1 to 16, for use in the treatment of an ocular disorder, such as an ocular disorder selected from glaucoma, an ocular inflammatory disease, and a disease associated with excessive vascularization of the eye.

**18.** A dosage container containing a formulation as defined in any one of claims 1 to 17.

**Patentansprüche**

**1.** Formulierung zur okulären Verabreichung, umfassend in einer wässrigen Phase:

(i) eine therapeutisch wirksame Menge von Laquinimod oder einem pharmazeutisch verträglichen Salz davon als Wirkstoff,
(ii) ein pharmazeutisch akzeptables Viskositätsmittel in einer ausreichenden Menge, um eine dynamische Viskosität von 2 bis 200 mPas, gemessen bei 20 °C, bereitzustellen,
(iii) ein pharmazeutisch akzeptables Tonizitätsanpassungsmittel in einer ausreichenden Menge, um eine Osmolalität von 200 bis 600 mOsm/kg bereitzustellen,
(iv) ein pharmazeutisch akzeptables Feuchthaltemittel,
(v) ein pharmazeutisch akzeptables Antioxidans und
(vi) ein pharmazeutisch akzeptables pH-regulierendes Mittel.

**2.** Formulierung nach Anspruch 1, wobei das pharmazeutisch akzeptable Viskositätsmittel in einer ausreichenden Menge vorhanden ist, um eine dynamische Viskosität von 5 bis 100 mPas, vorzugsweise 10 bis 50 mPas, besonders bevorzugt 15 bis 45 mPas, gemessen bei 20 °C, bereitzustellen.

**3.** Formulierung nach Anspruch 1 oder 2, wobei das pharmazeutisch akzeptable Viskositätsmittel ein oder mehrere Elemente aus der Gruppe umfasst, die aus Polyvinylalkohol, Poly(acrylsäure)-Homo- oder -Copolymeren (Carbomeren), Polyvinylpyrrolidon und Cellulosederivaten wie Hydroxypropylmethylcellulose und Natriumcarboxymethylcellulose besteht.

4. Formulierung nach einem der Ansprüche 1 bis 3, wobei das pharmazeutisch akzeptable Tonizitätsanpassungsmittel in einer ausreichenden Menge vorhanden ist, um eine Osmolalität von 200 bis 500 mOsm/kg, vorzugsweise 200 bis 400 mOsm/kg, bevorzugter 250 bis 375 mOsm/kg bereitzustellen.

5. Formulierung nach einem der Ansprüche 1 bis 4, wobei das pharmazeutisch akzeptable Tonizitätsanpassungsmittel ein nichtionisches Tonizitätsanpassungsmittel wie Mannitol ist.

6. Formulierung nach einem der Ansprüche 1 bis 5, wobei das pharmazeutisch akzeptable Feuchthaltemittel ein Polyol, vorzugsweise ein C3-C6-Polyol, bevorzugter Glycerin ist.

7. Formulierung nach einem der Ansprüche 1 bis 6, wobei das pharmazeutisch akzeptable pH-regulierende Mittel in einer ausreichenden Menge vorhanden ist, um einen pH-Wert im Bereich von 6,8 bis 8,5, vorzugsweise im Bereich von 6,8 bis 8,0 bereitzustellen.

8. Formulierung nach einem der Ansprüche 1 bis 7, die weiter (vii) ein pharmazeutisch akzeptables Konservierungsmittel wie Benzalkoniumchlorid umfasst.

9. Formulierung nach einem der Ansprüche 1 bis 8, die weiter (viii) ein pharmazeutisch akzeptables Tensid wie ein nichtionisches Tensid umfasst.

10. Formulierung nach einem der Ansprüche 1 bis 9, die weiter (ix) einen pharmazeutisch akzeptablen Lösungsvermittler umfasst.

11. Formulierung nach einem der Ansprüche 1 bis 10, wobei

das pharmazeutisch akzeptable Viskositätsmittel in einer ausreichenden Menge vorhanden ist, um eine dynamische Viskosität von 10 bis 45 mPas, gemessen bei 20 °C, bereitzustellen;
das pharmazeutisch akzeptable Tonizitätsanpassungsmittel in einer ausreichenden Menge vorhanden ist, um eine Osmolalität von 200 bis 400 mOsm/kg bereitzustellen; und
das pharmazeutisch akzeptable pH-regulierende Mittel in einer ausreichenden Menge vorhanden ist, um einen pH-Wert von 6,8 bis 8,0 bereitzustellen.

12. Formulierung nach einem der Ansprüche 1 bis 11 in Form eines Gels.

13. Formulierung nach Anspruch 12, umfassend:

(i) 5 bis 100 g/l Laquinimod oder eine entsprechende Menge eines pharmazeutisch akzeptablen Salzes von Laquinimod;
(ii) 1 bis 10 g/l Natriumcarboxymethylcellulose und/oder 0,5 bis 6 g/l eines Carbomer-Copolymers;
(iii) 1,5 bis 4 g/l Mannitol;
(iv) 10 bis 40 g/l Glycerin;
(v) 0,2 bis 2 g/l Na-EDTA; und
(vi) ein pH-Puffermittel in einer Menge, die wirksam ist, um einen pH-Wert im Bereich von 6,8 bis 8,0 bereitzustellen.

14. Formulierung nach Anspruch 13, die weiter 0,1 bis 5 g/l Polysorbat umfasst.

15. Formulierung nach einem der Ansprüche 1 bis 11 in Form einer wasser- und ölhaltigen Emulsion, vorzugsweise einer Öl-in-Wasser-Emulsion.

16. Formulierung nach einem der Ansprüche 1 bis 15, wobei die okuläre Verabreichung eine topische okuläre Verabreichung ist.

17. Formulierung nach einem der Ansprüche 1 bis 16 zur Verwendung bei der Behandlung einer Augenerkrankung, wie etwa einer Augenerkrankung ausgewählt aus Glaukom, einer entzündlichen Augenerkrankung und einer Erkrankung, die mit übermäßiger Gefäßbildung im Auge verbunden ist.

**18.** Dosierbehälter, der eine Formulierung nach einem der Ansprüche 1 bis 17 enthält.

**Revendications**

**1.** Formulation pour administration oculaire comprenant, dans une phase aqueuse :

(i) une quantité thérapeutiquement efficace de laquinimod ou d'un sel pharmaceutiquement acceptable de celui-ci en tant qu'ingrédient actif,
(ii) un agent de viscosité pharmaceutiquement acceptable en une quantité suffisante pour fournir une viscosité dynamique de 2 à 200 mPas, telle que mesurée à 20 °C,
(iii) un agent d'ajustement de la tonicité pharmaceutiquement acceptable, en quantité suffisante pour fournir une osmolalité de 200 à 600 mOsm/kg,
(iv) un humectant pharmaceutiquement acceptable,
(v) un antioxydant pharmaceutiquement acceptable, et
(vi) un agent de régulation du pH pharmaceutiquement acceptable.

**2.** Formulation selon la revendication 1, dans laquelle l'agent de viscosité pharmaceutiquement acceptable est présent en une quantité suffisante pour fournir une viscosité dynamique de 5 à 100 mPas, de préférence de 10 à 50 mPas, plus préférentiellement de 15 à 45 mPas, telle que mesurée à 20 °C.

**3.** Formulation selon la revendication 1 ou 2, dans laquelle l'agent de viscosité pharmaceutiquement acceptable comprend un ou plusieurs éléments du groupe consistant en l'alcool polyvinylique, les homo- ou copolymères (carbomères) de poly(acide acrylique), la polyvinylpyrrolidone et des dérivés de cellulose, tels que l'hydroxypropylméthylcellulose et la carboxyméthylcellulose sodique.

**4.** Formulation selon l'une quelconque des revendications 1 à 3, dans laquelle l'agent d'ajustement de la tonicité pharmaceutiquement acceptable est présent en une quantité suffisante pour fournir une osmolalité de 200 à 500 mOsm/kg, de préférence de 200 à 400 mOsm/kg, plus préférentiellement de 250 à 375 mOsm/kg.

**5.** Formulation selon l'une quelconque des revendications 1 à 4, dans laquelle l'agent d'ajustement de la tonicité pharmaceutiquement acceptable est un agent d'ajustement de la tonicité non ionique, tel que le mannitol.

**6.** Formulation selon l'une quelconque des revendications 1 à 5, dans laquelle l'humectant pharmaceutiquement acceptable est un polyol, de préférence un polyol en C3-C6, plus préférentiellement du glycérol.

**7.** Formulation selon l'une quelconque des revendications 1 à 6, dans laquelle l'agent de régulation du pH pharmaceutiquement acceptable est présent en une quantité suffisante pour fournir un pH dans la plage de 6,8 à 8,5, de préférence dans la plage de 6,8 à 8,0.

**8.** Formulation selon l'une quelconque des revendications 1 à 7, comprenant en outre (vii) un conservateur pharmaceutiquement acceptable, tel que le chlorure de benzalkonium.

**9.** Formulation selon l'une quelconque des revendications 1 à 8, comprenant en outre (viii) un tensioactif pharmaceutiquement acceptable, tel qu'un tensioactif non ionique.

**10.** Formulation selon l'une quelconque des revendications 1 à 9, comprenant en outre (ix) un agent solubilisant pharmaceutiquement acceptable.

**11.** Formulation selon l'une quelconque des revendications 1 à 10, dans laquelle

l'agent de viscosité pharmaceutiquement acceptable est présent en une quantité suffisante pour fournir une viscosité dynamique de 10 à 45 mPas, telle que mesurée à 20 °C ;
l'agent d'ajustement de la tonicité pharmaceutiquement acceptable est présent en une quantité suffisante pour fournir une osmolalité de 200 à 400 mOsm/kg ; et
l'agent de régulation du pH pharmaceutiquement acceptable est présent en une quantité suffisante pour fournir

un pH de 6,8 à 8,0.

**12.** Formulation selon l'une quelconque des revendications 1 à 11, sous la forme d'un gel.

**13.** Formulation selon la revendication 12, comprenant :

(i) 5 à 100 g/l de laquinimod, ou une quantité correspondante d'un sel pharmaceutiquement acceptable de laquinimod ;
(ii) 1 à 10 g/l de carboxyméthylcellulose sodique, et/ou 0,5 à 6 g/l d'un copolymère carbomère ;
(iii) 1,5 à 4 g/l de mannitol ;
(iv) 10 à 40 g/l de glycérol ;
(v) 0,2 à 2 g/l de Na-EDTA; et
(vi) un agent tampon de pH en une quantité efficace pour fournir un pH dans la plage de 6,8 à 8,0.

**14.** Formulation selon la revendication 13, comprenant en outre 0,1 à 5 g/l de polysorbate.

**15.** Formulation selon l'une quelconque des revendications 1 à 11, sous la forme d'une émulsion contenant de l'eau et de l'huile, de préférence une émulsion huile dans eau.

**16.** Formulation selon l'une quelconque des revendications 1 à 15, dans laquelle l'administration oculaire est une administration oculaire topique.

**17.** Formulation selon l'une quelconque des revendications 1 à 16, destinée à être utilisée dans le traitement d'une maladie oculaire, telle qu'une maladie oculaire sélectionnée parmi un glaucome, une maladie inflammatoire oculaire et une maladie associée à une vascularisation excessive de l'œil.

**18.** Récipient de dosage contenant une formulation selon l'une quelconque des revendications 1 à 17.

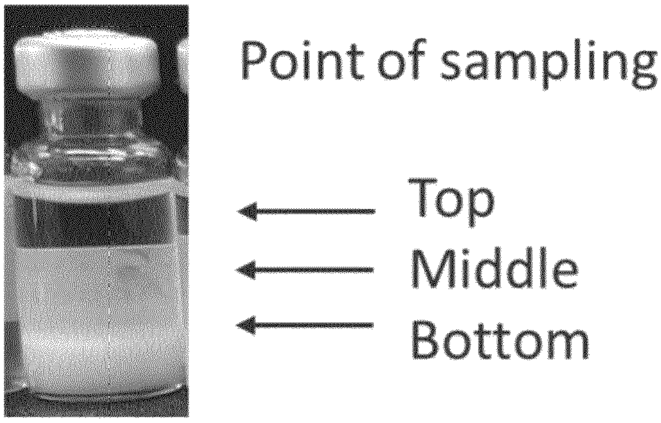
Point of sampling
Top
Middle
Bottom

FIG. 1

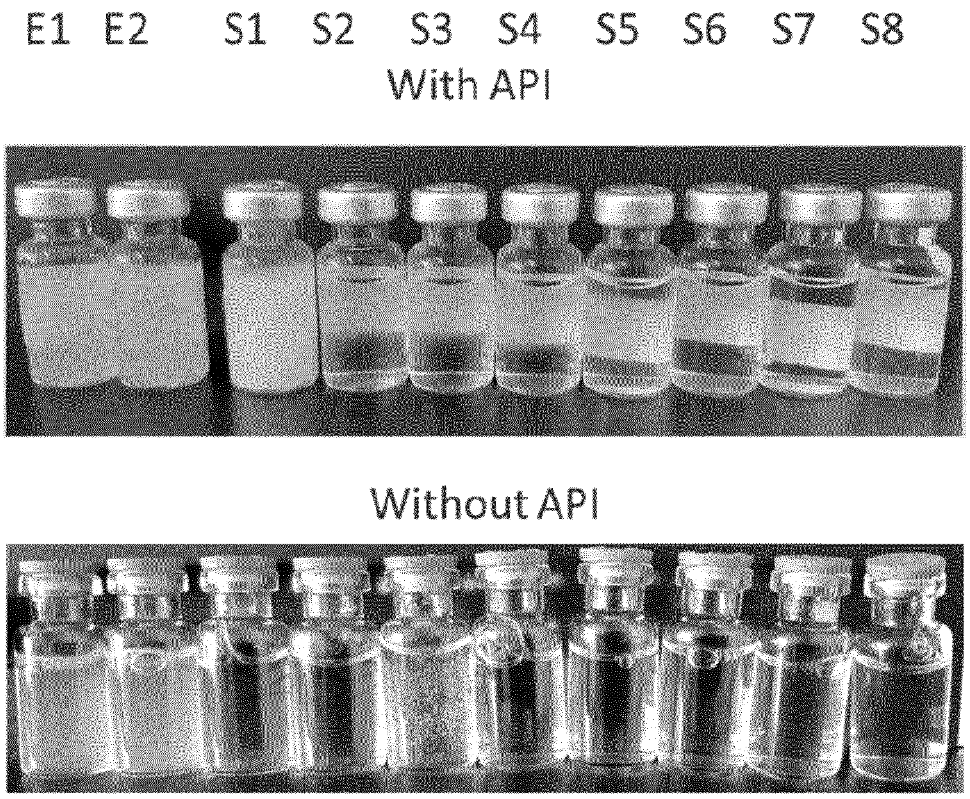
E1 E2 S1 S2 S3 S4 S5 S6 S7 S8
With API
Without API

FIG. 2

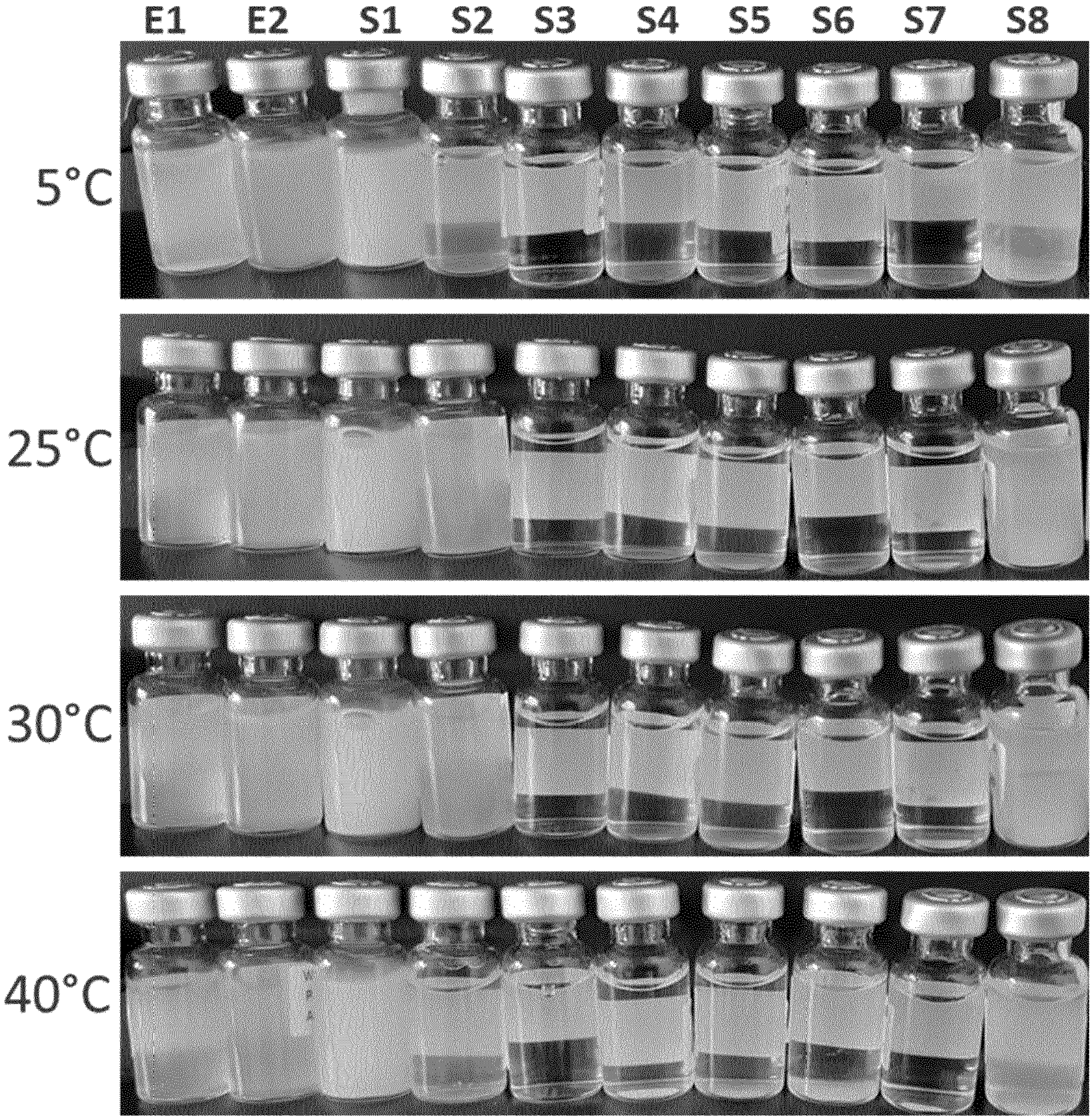


FIG. 3

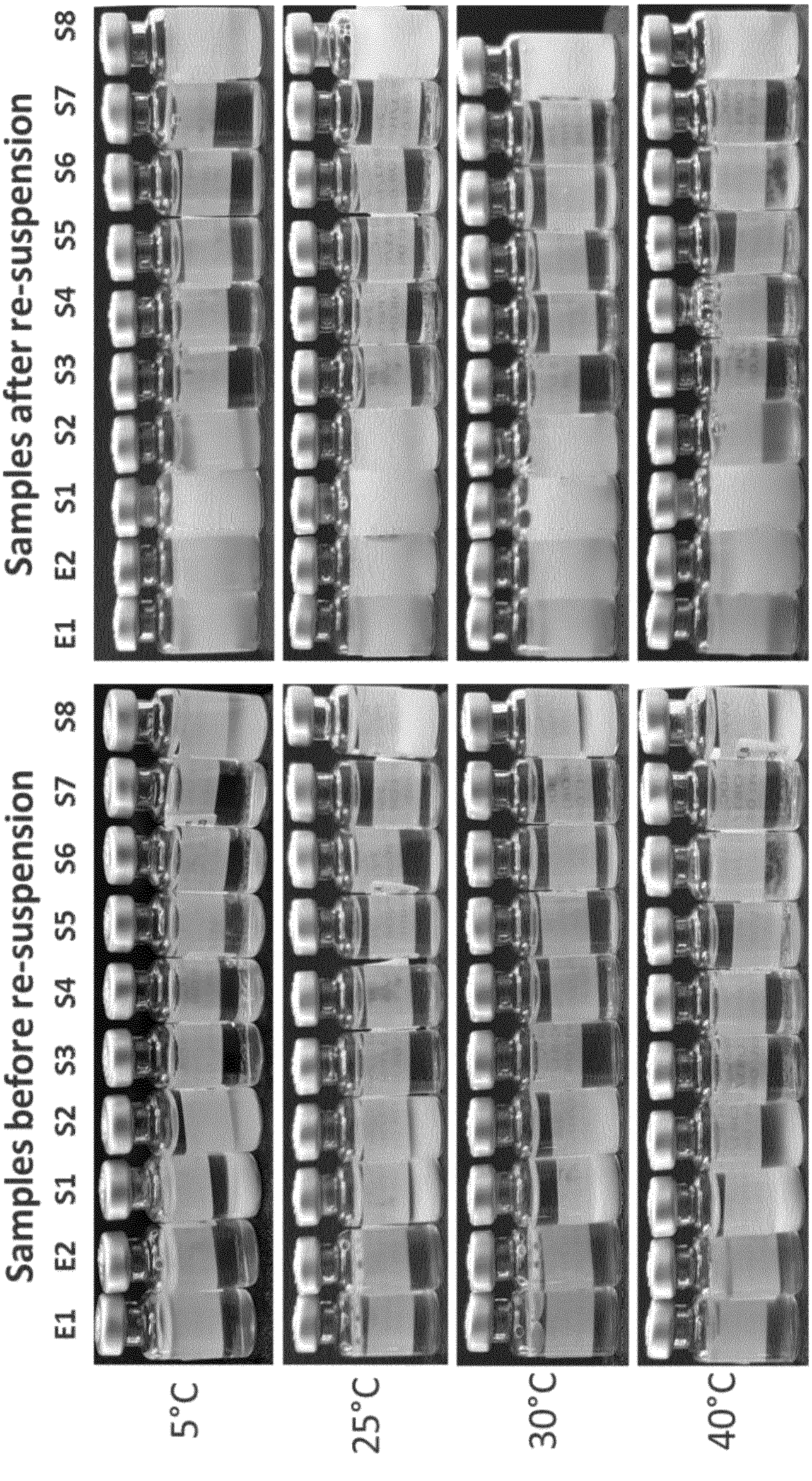
Samples before re-suspension
Samples after re-suspension
E1
E2
S1
S2
S3
S4
S5
S6
S7
S8
5°C
25°C
30°C
40°C

FIG. 4

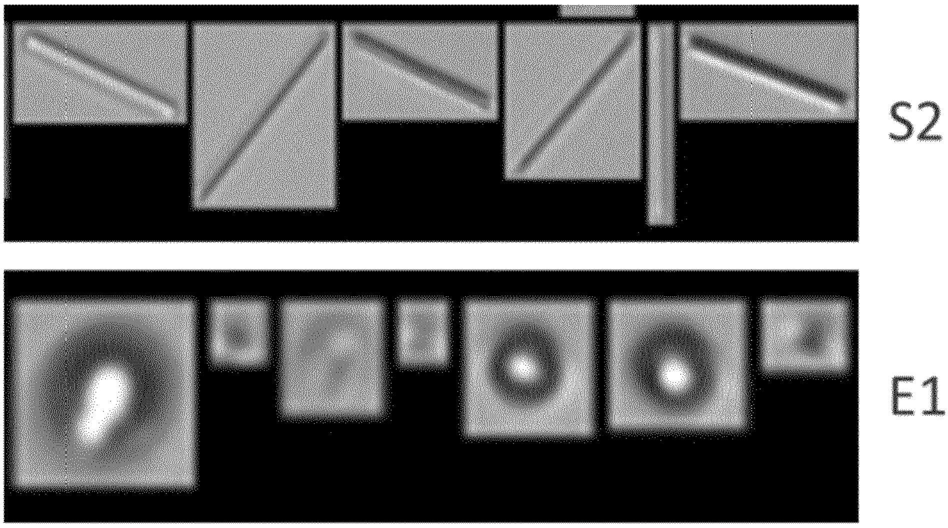


FIG. 5

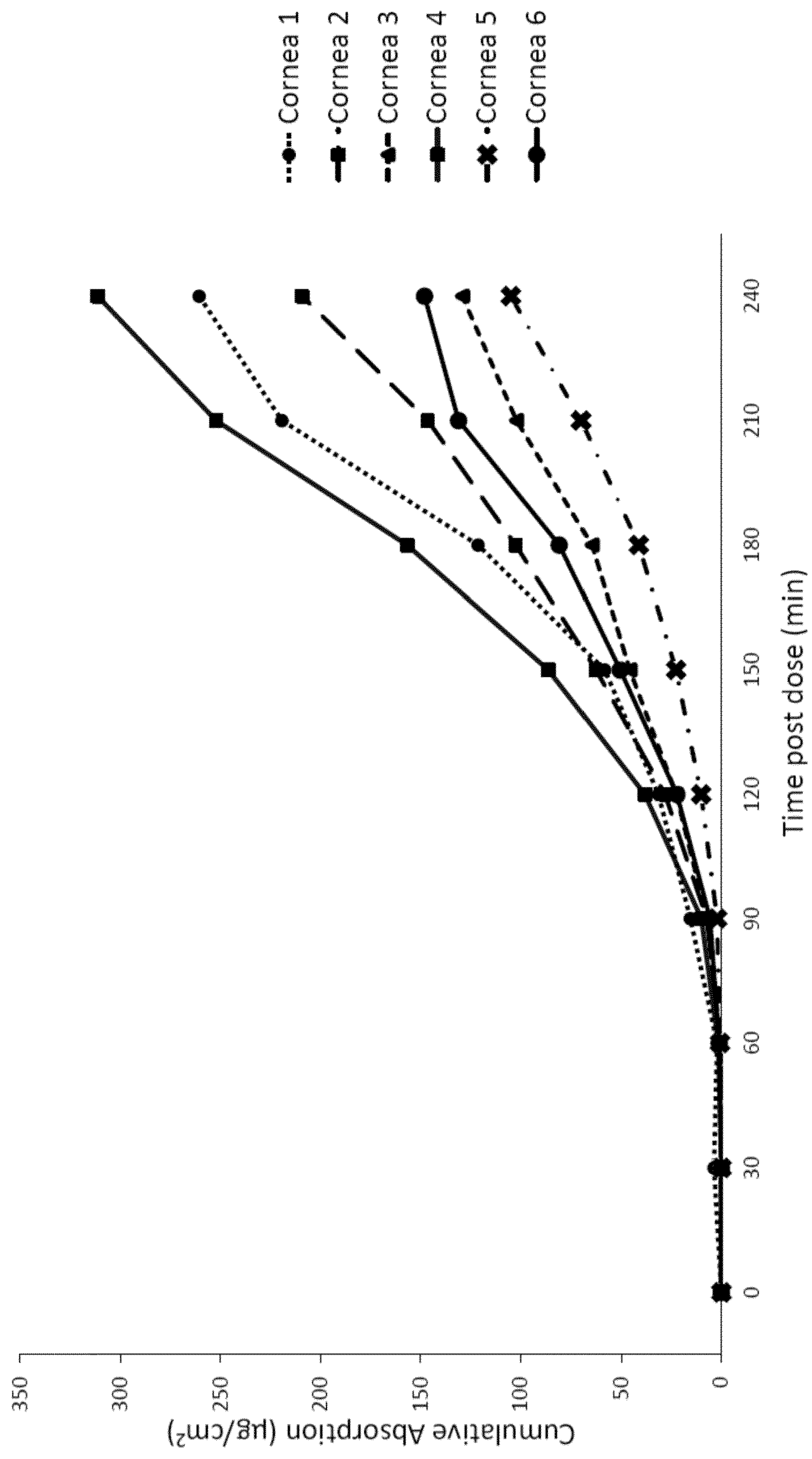
Cornea 1
Cornea 2
Cornea 3
Cornea 4
Cornea 5
Cornea 6
Cumulative Absorption (µg/cm²)
0
50
100
150
200
250
300
350
Time post dose (min)
0
30
60
90
120
150
180
210
240

FIG. 6

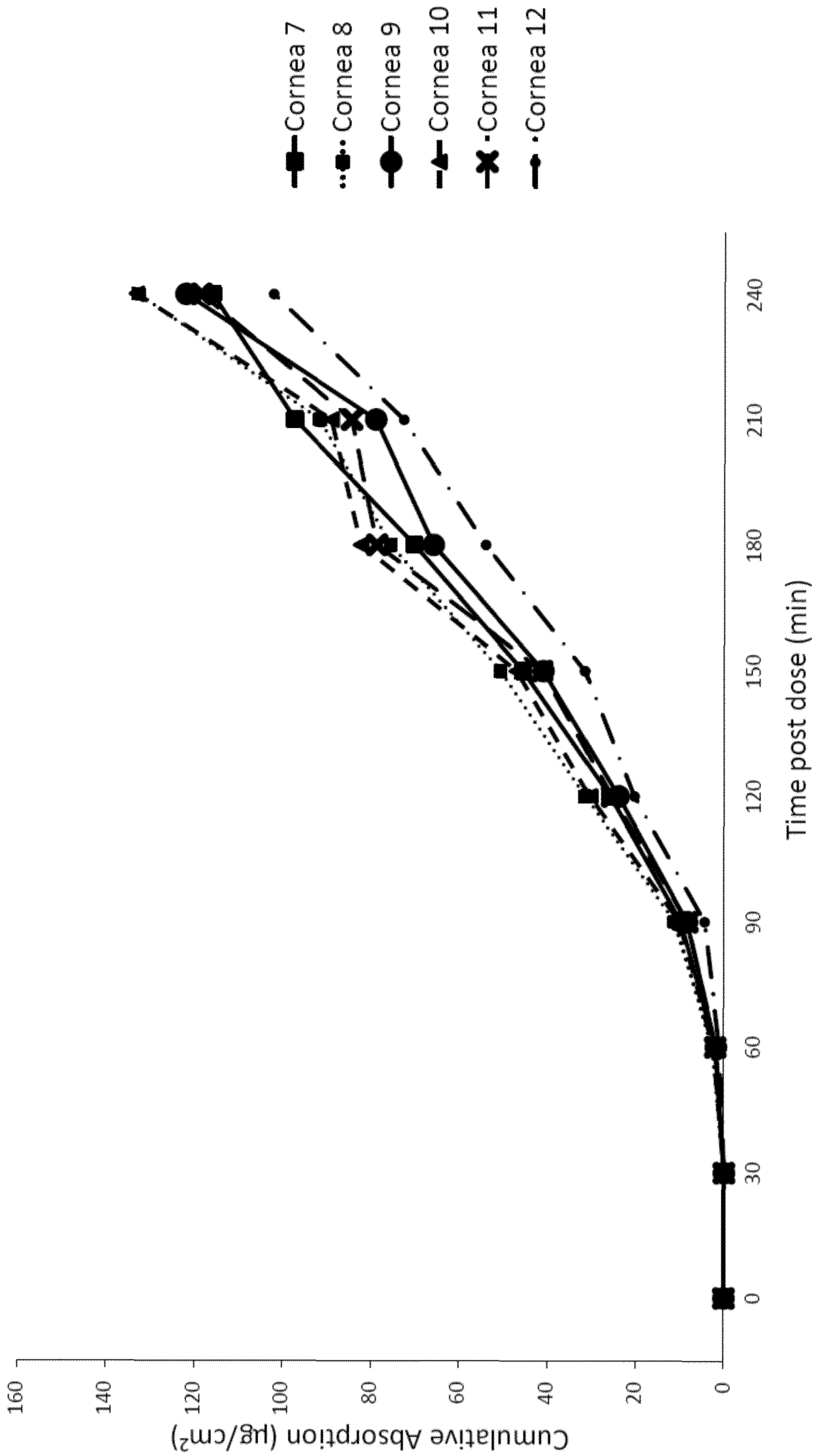
Cumulative Absorption (µg/cm²)
0
20
40
60
80
100
120
140
160
Time post dose (min)
0
30
60
90
120
150
180
210
240
Cornea 7
Cornea 8
Cornea 9
Cornea 10
Cornea 11
Cornea 12

FIG. 7

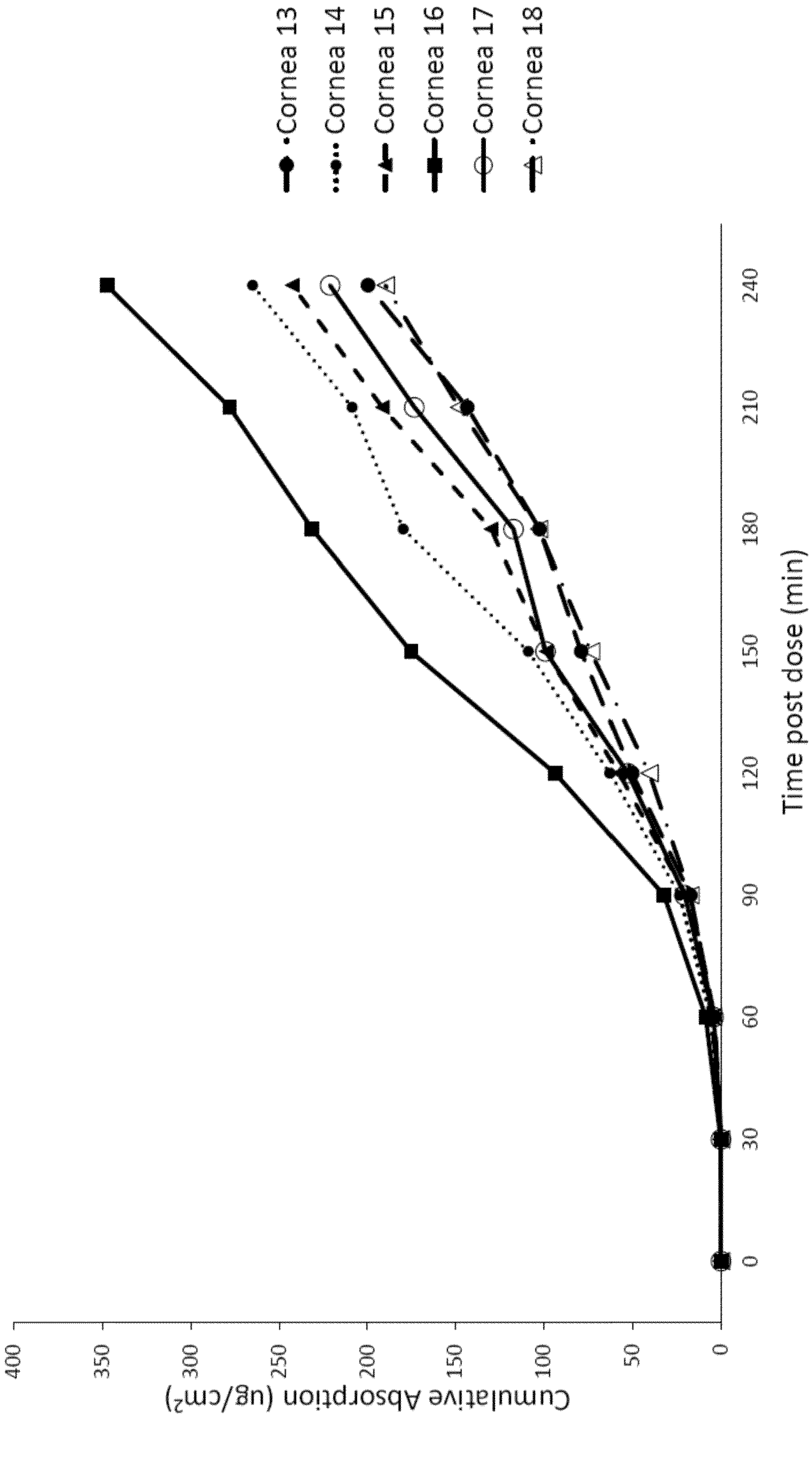

Cornea 13
Cornea 14
Cornea 15
Cornea 16
Cornea 17
Cornea 18
Cumulative Absorption (ug/cm²)
0
50
100
150
200
250
300
350
400
Time post dose (min)
0
30
60
90
120
150
180
210
240


FIG. 8

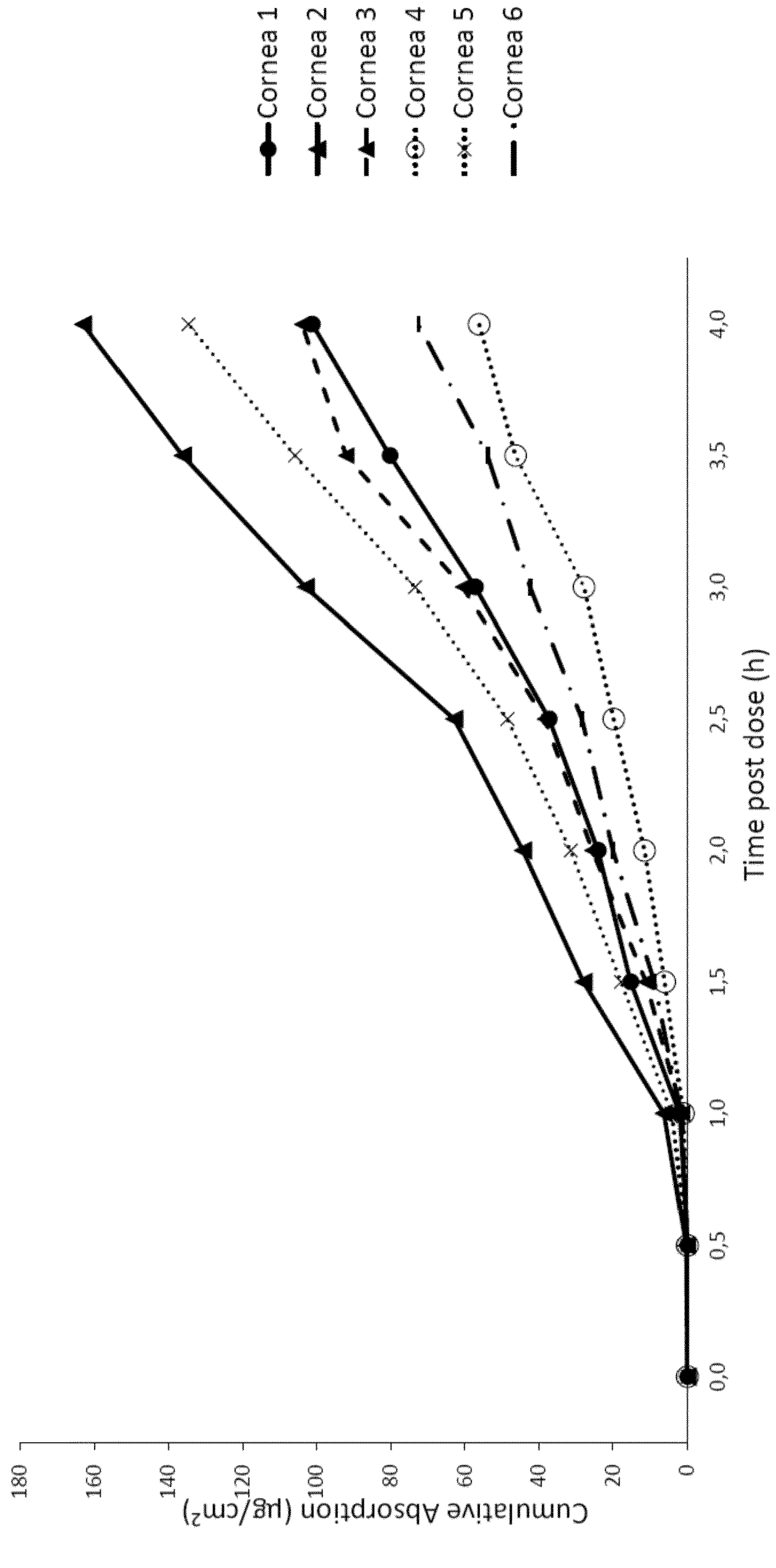
Cornea 1
Cornea 2
Cornea 3
Cornea 4
Cornea 5
Cornea 6
Cumulative Absorption (µg/cm²)
0
20
40
60
80
100
120
140
160
180
Time post dose (h)
0,0
0,5
1,0
1,5
2,0
2,5
3,0
3,5
4,0

FIG. 9

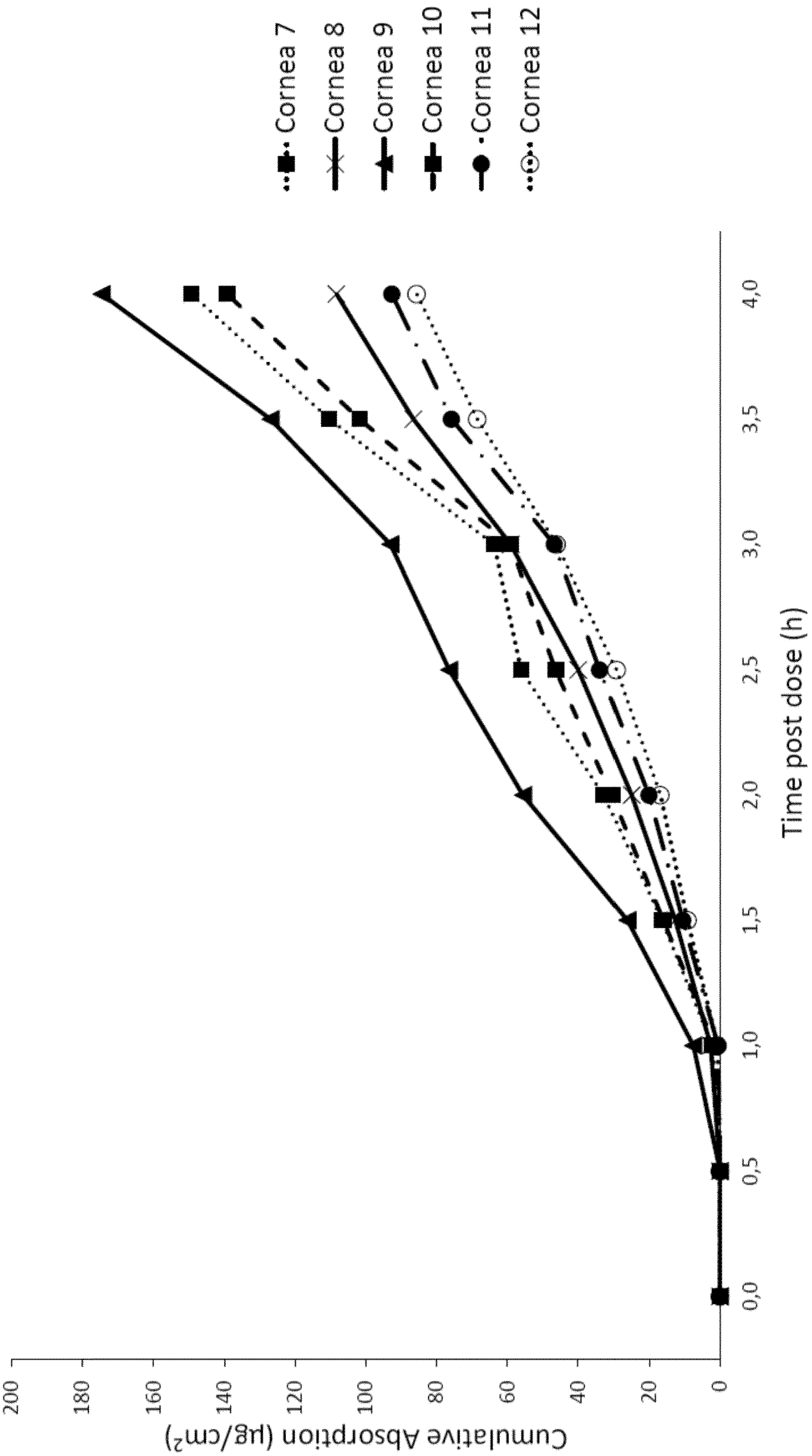
Cornea 7
Cornea 8
Cornea 9
Cornea 10
Cornea 11
Cornea 12
Cumulative Absorption (µg/cm²)
0
20
40
60
80
100
120
140
160
180
200
Time post dose (h)
0,0
0,5
1,0
1,5
2,0
2,5
3,0
3,5
4,0

FIG. 10

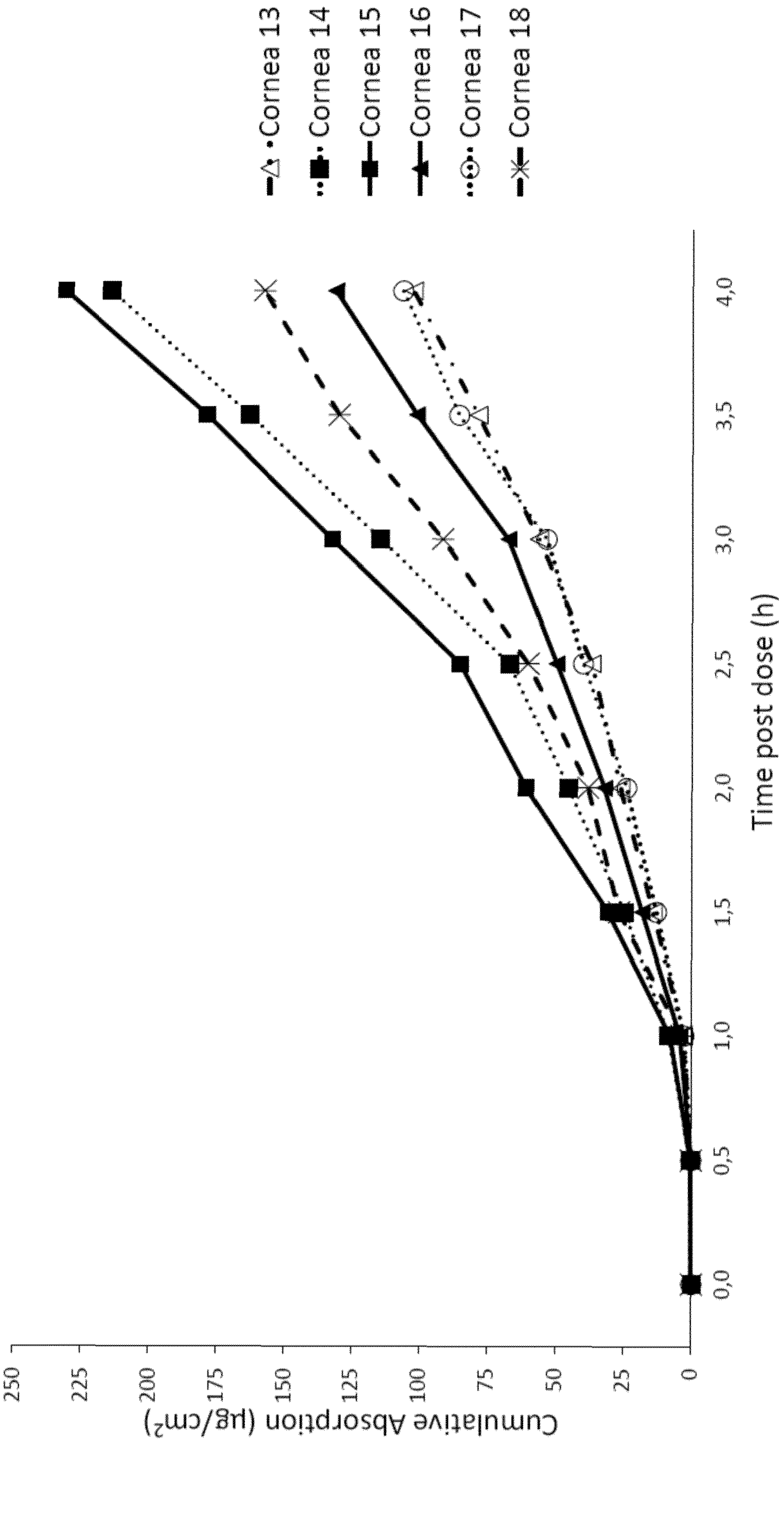
Cornea 13
Cornea 14
Cornea 15
Cornea 16
Cornea 17
Cornea 18
Cumulative Absorption (µg/cm²)
0
25
50
75
100
125
150
175
200
225
250
Time post dose (h)
0,0
0,5
1,0
1,5
2,0
2,5
3,0
3,5
4,0

FIG. 11

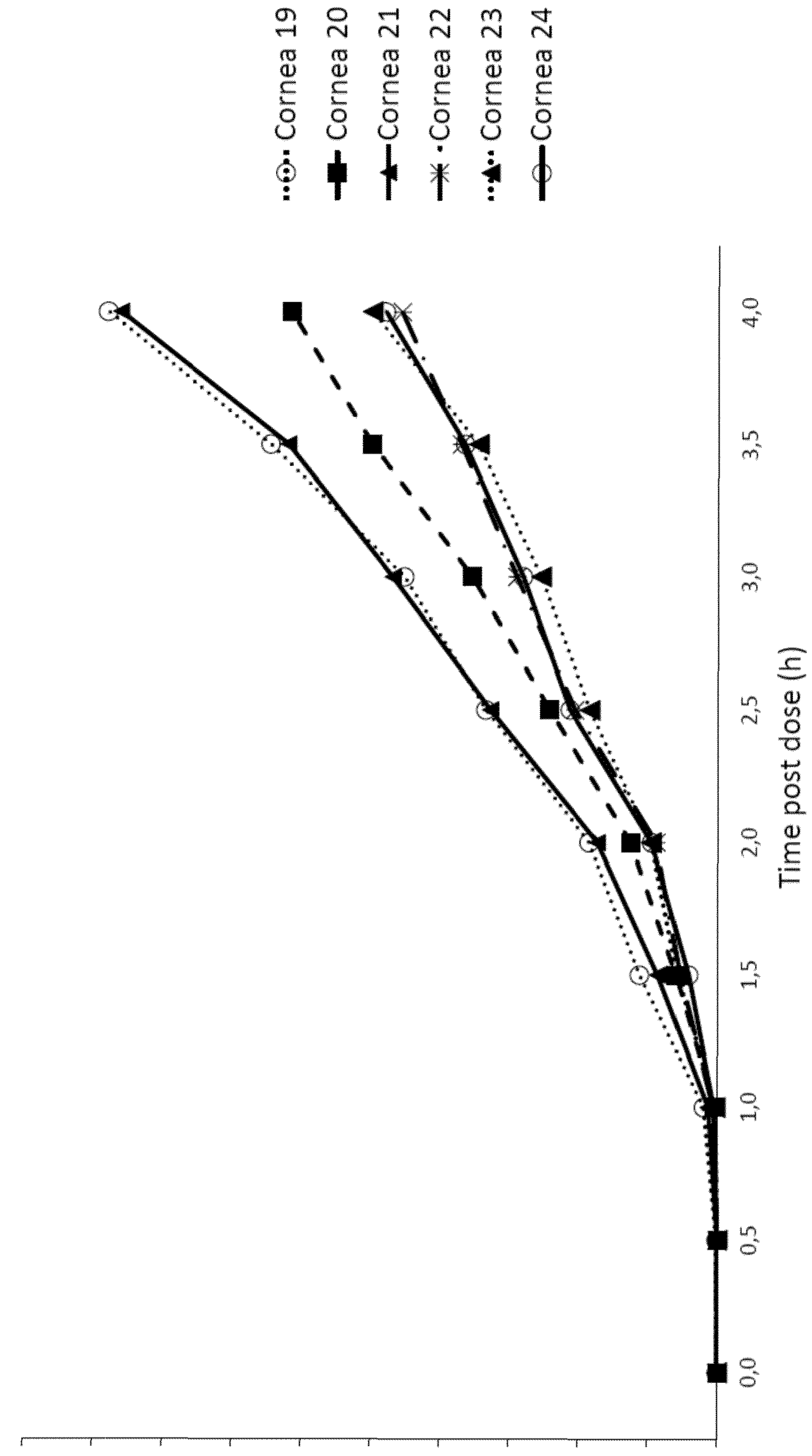
Cornea 19
Cornea 20
Cornea 21
Cornea 22
Cornea 23
Cornea 24
Cumulative Absorption (µg/cm²)
0
20
40
60
80
100
120
140
160
180
200
0,0
0,5
1,0
1,5
2,0
2,5
3,0
3,5
4,0
Time post dose (h)

FIG. 12

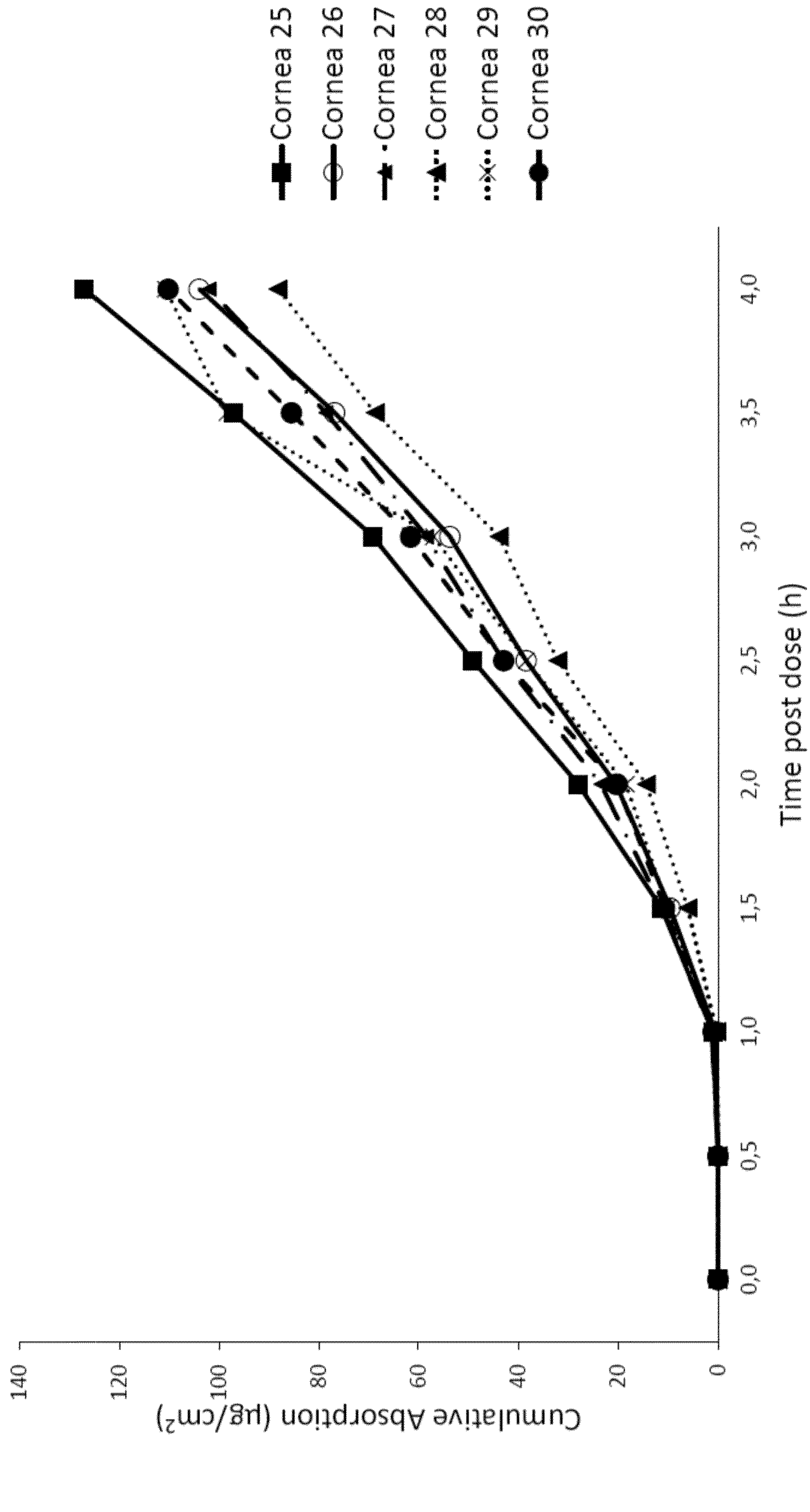
Cornea 25
Cornea 26
Cornea 27
Cornea 28
Cornea 29
Cornea 30
Cumulative Absorption (µg/cm²)
0
20
40
60
80
100
120
140
0,0
0,5
1,0
1,5
2,0
2,5
3,0
3,5
4,0
Time post dose (h)

FIG. 13

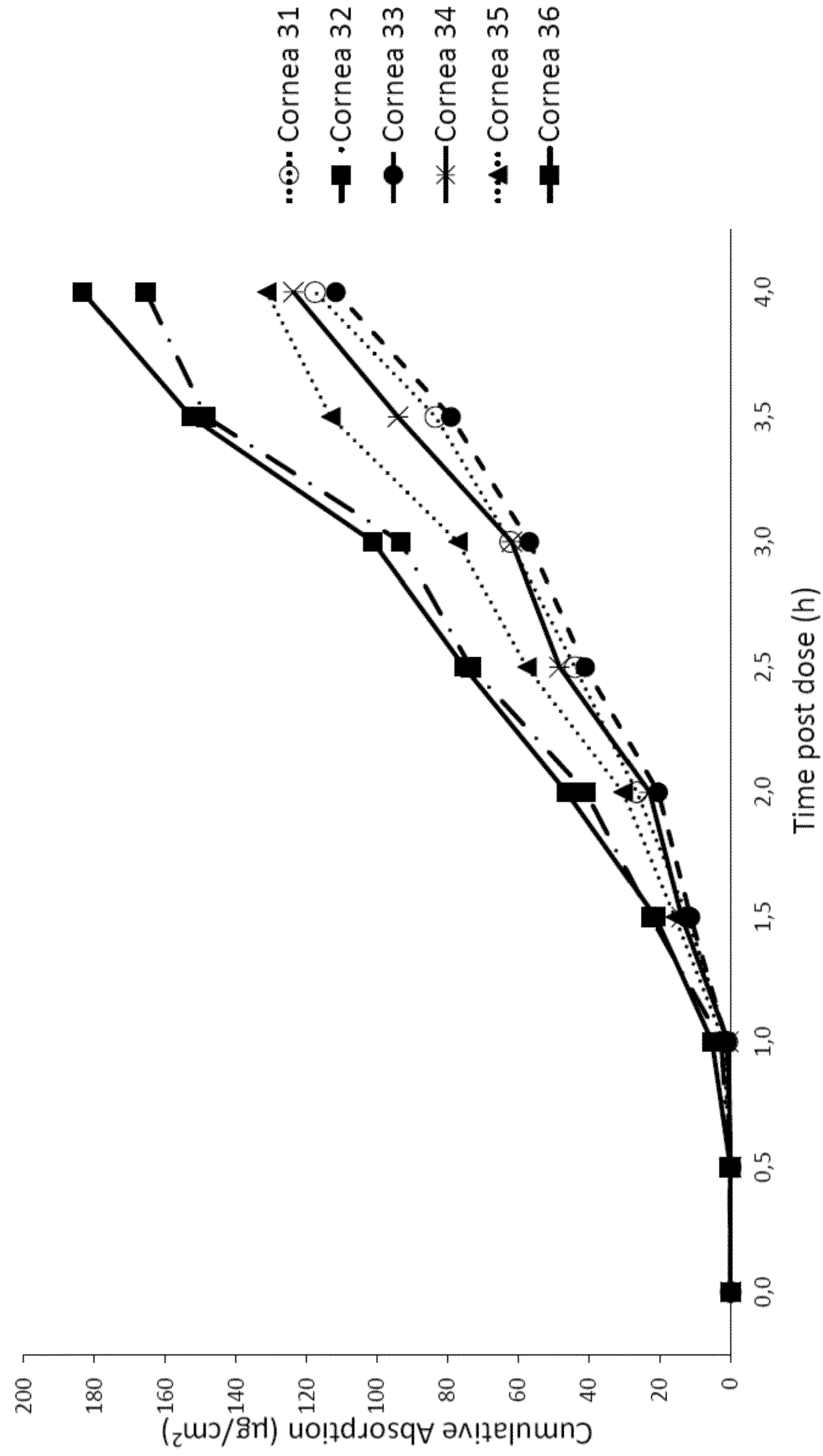
Cornea 31
Cornea 32
Cornea 33
Cornea 34
Cornea 35
Cornea 36
Cumulative Absorption (µg/cm²)
200
180
160
140
120
100
80
60
40
20
0
0,0
0,5
1,0
1,5
2,0
2,5
3,0
3,5
4,0
Time post dose (h)

FIG. 14

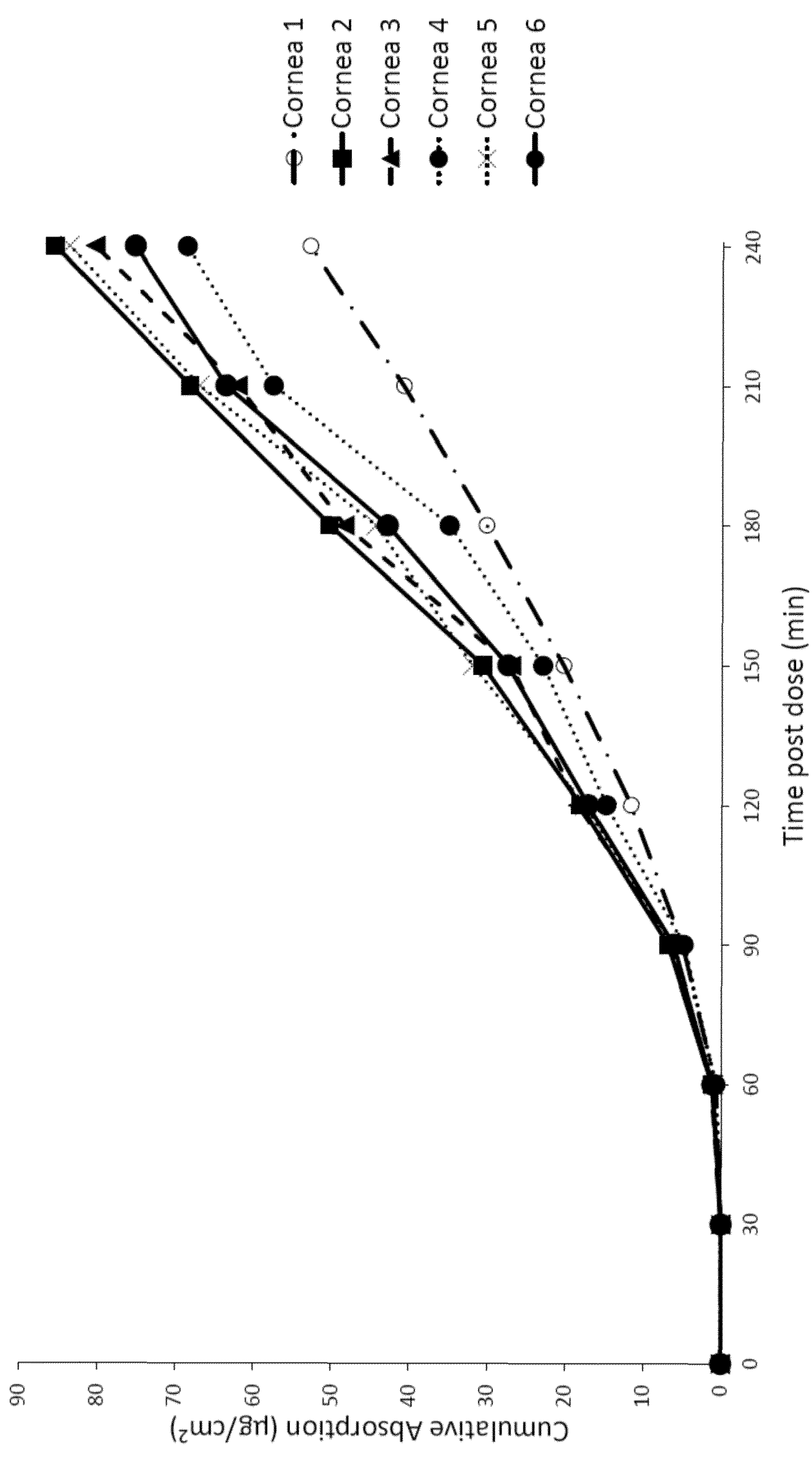
Cumulative Absorption (µg/cm²)
90
80
70
60
50
40
30
20
10
0
Time post dose (min)
0
30
60
90
120
150
180
210
240
Cornea 1
Cornea 2
Cornea 3
Cornea 4
Cornea 5
Cornea 6

FIG. 15

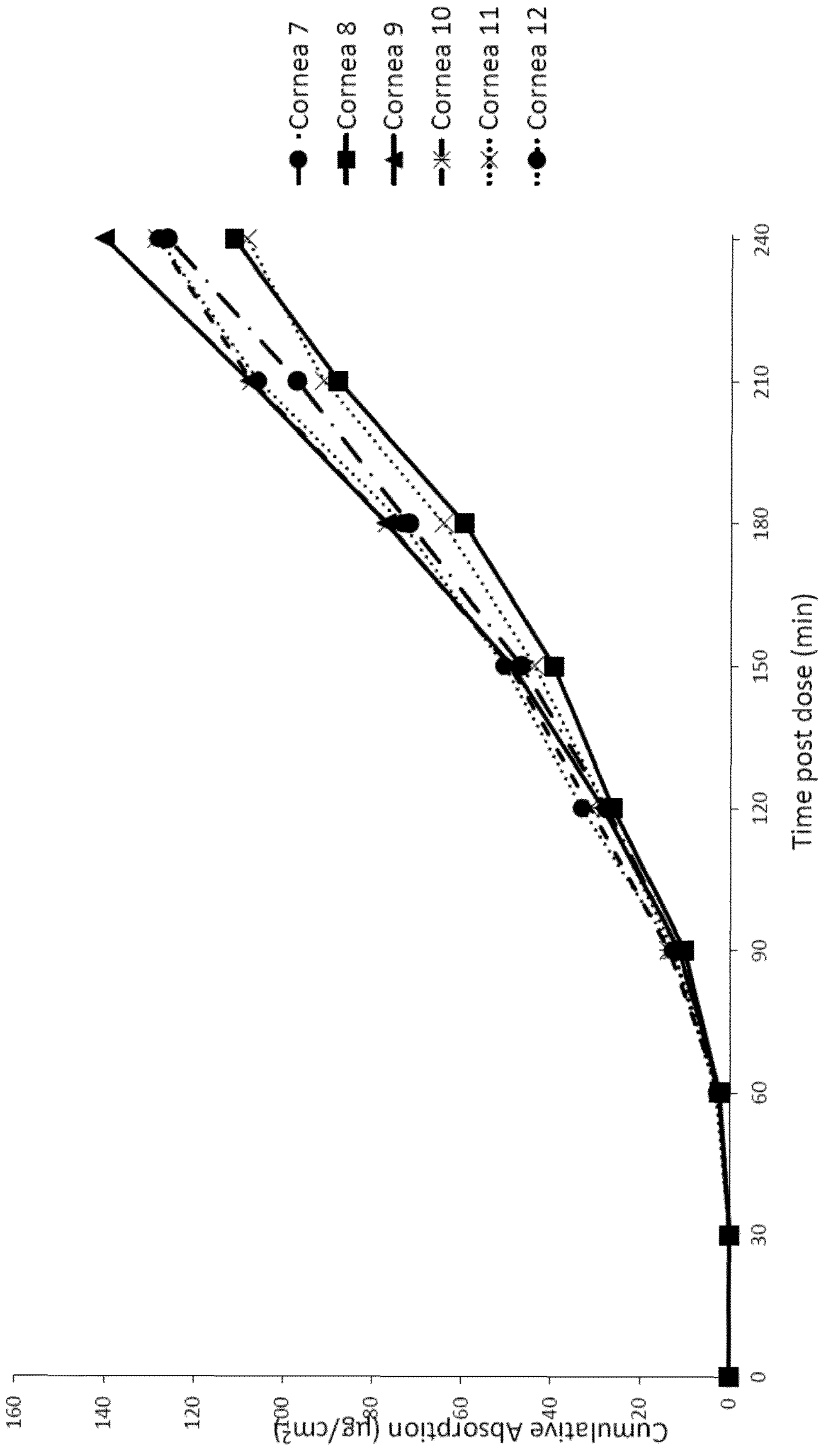
Cornea 7
Cornea 8
Cornea 9
Cornea 10
Cornea 11
Cornea 12
Cumulative Absorption (µg/cm2)
0
20
40
60
80
100
120
140
160
0
30
60
90
120
150
180
210
240
Time post dose (min)

FIG. 16

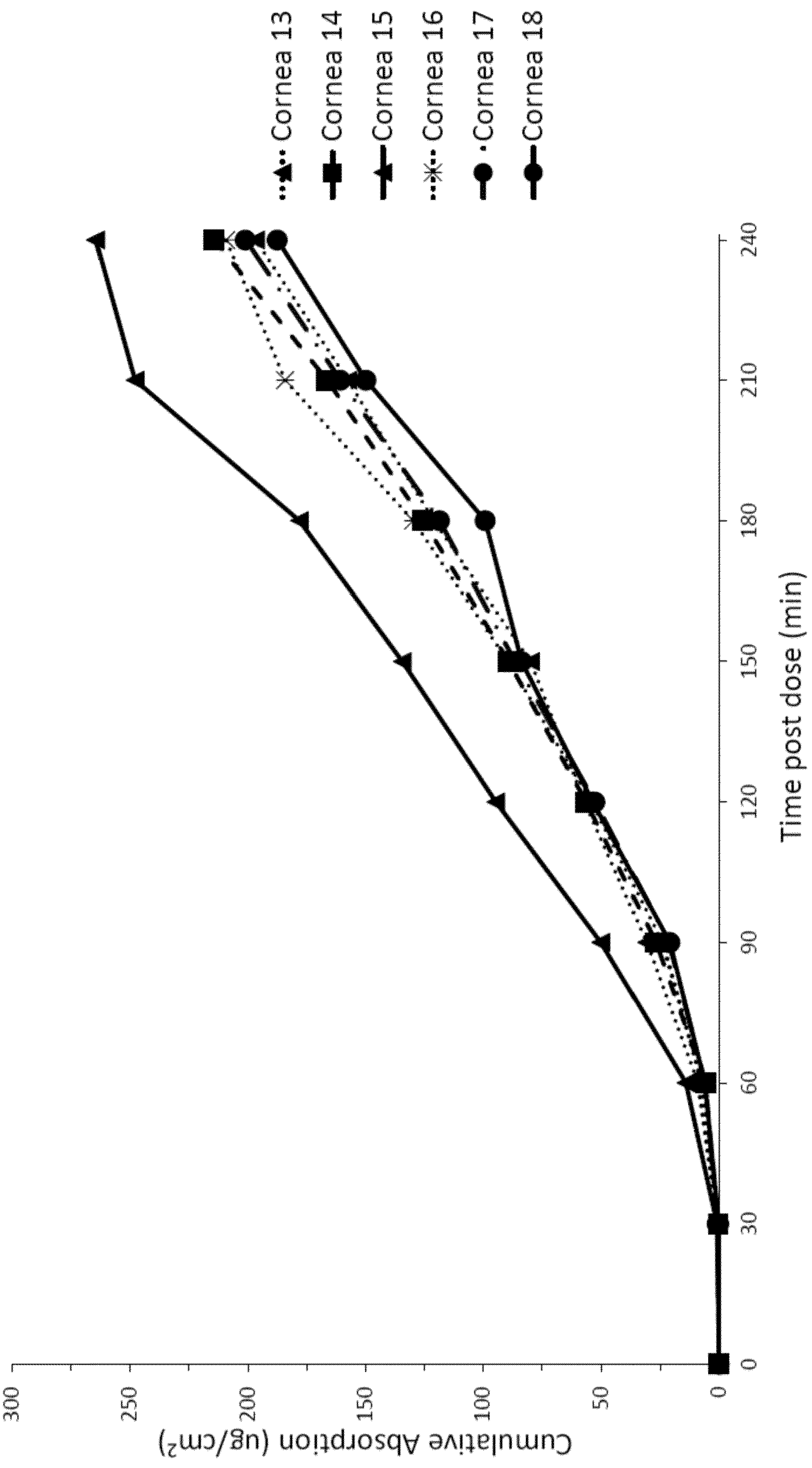
Cumulative Absorption (ug/cm²)
300
250
200
150
100
50
0
0
30
60
90
120
150
180
210
240
Time post dose (min)
Cornea 13
Cornea 14
Cornea 15
Cornea 16
Cornea 17
Cornea 18

FIG. 17

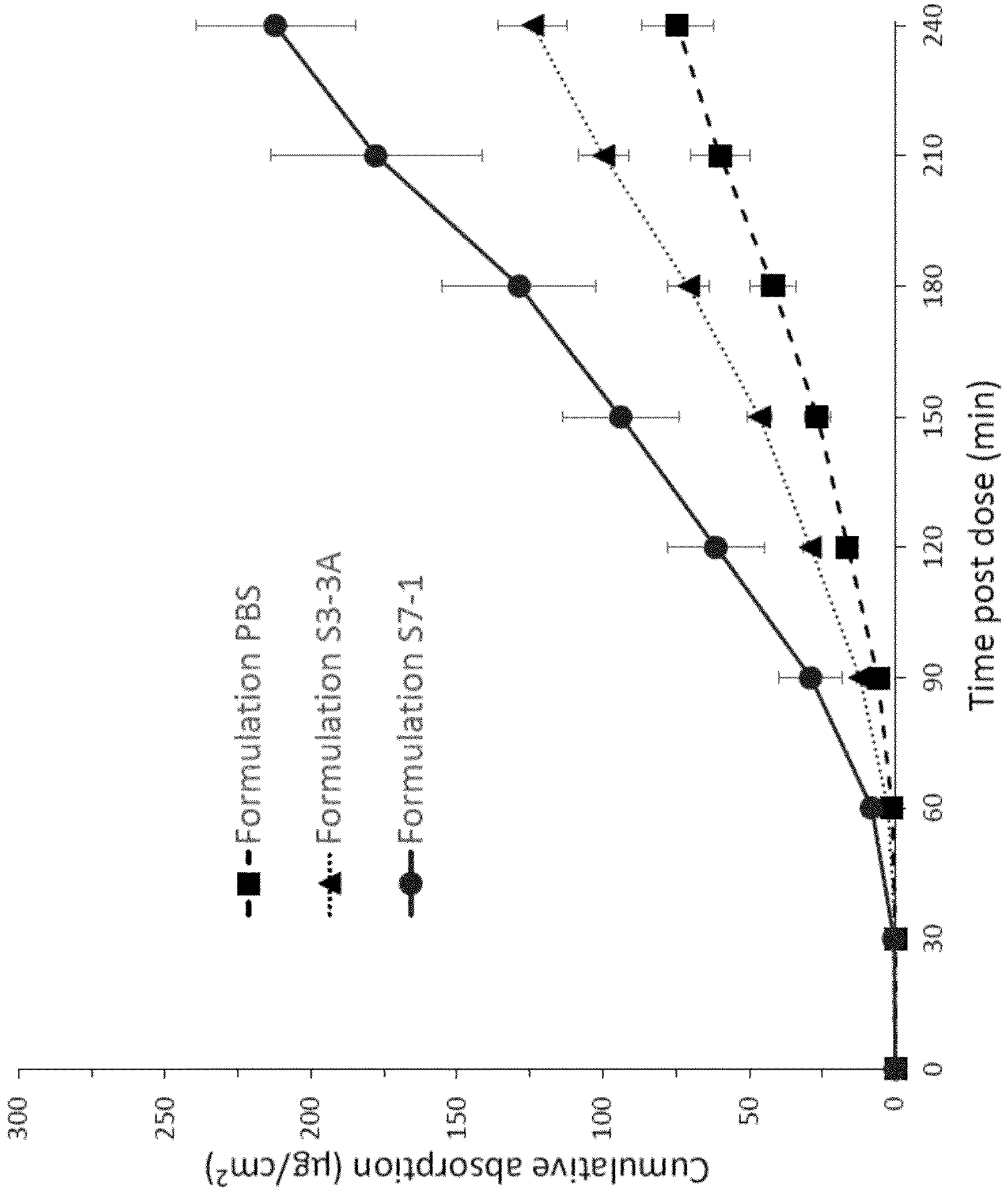
Formulation PBS
Formulation S3-3A
Formulation S7-1
Cumulative absorption (µg/cm²)
300
250
200
150
100
50
0
0
30
60
90
120
150
180
210
240
Time post dose (min)

FIG. 18

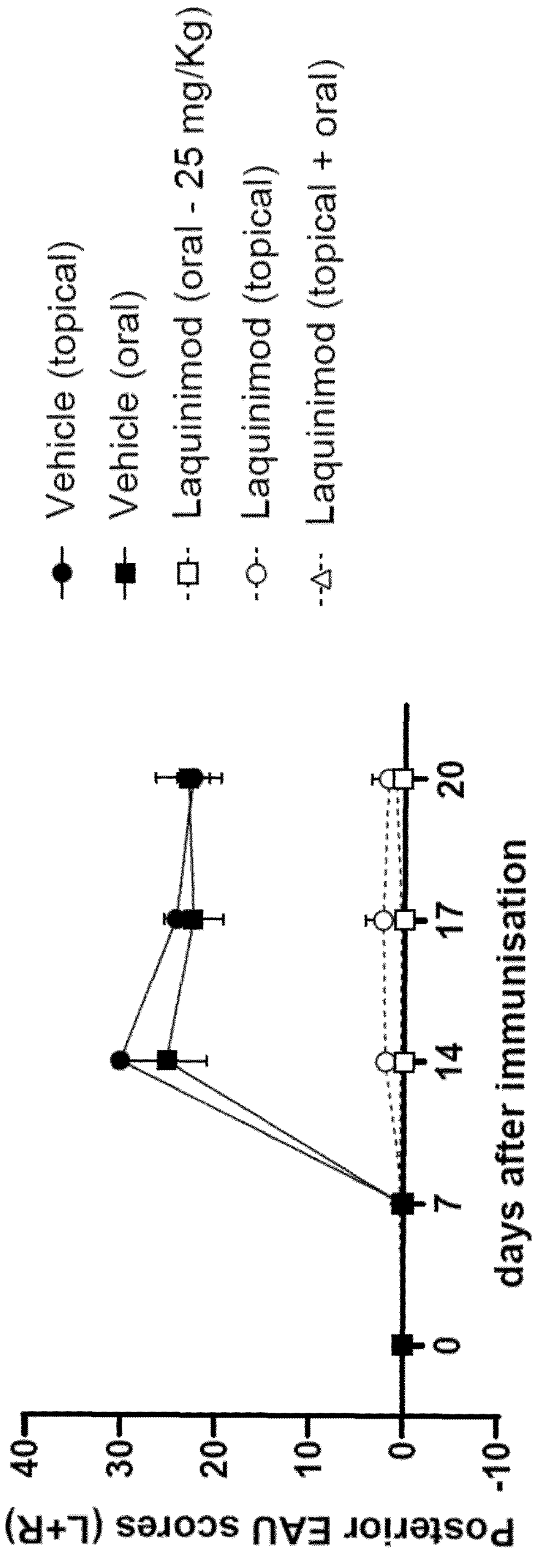
Vehicle (topical)
Vehicle (oral)
Laquinimod (oral - 25 mg/Kg)
Laquinimod (topical)
Laquinimod (topical + oral)
Posterior EAU scores (L+R)
40
30
20
10
0
-10
0
7
14
17
20
days after immunisation

FIG. 19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- US 6077851 A
- US 2014065497 W
- WO 2015073697 A
- US 816402
- US 20180071275 A1
- EP 2020086993 W
- WO 2021123142 A1

### Non-patent literature cited in the description

- *CHEMICAL ABSTRACTS,* 9003-11-6
- *CHEMICAL ABSTRACTS,* 61791-12-6
- *CHEMICAL ABSTRACTS,* 9005-65-6
- *CHEMICAL ABSTRACTS,* 8001-54-5
- OPHTHALMIC SQUEEZE DISPENSER - Eliminating the Need for Additives in Multidose Preservative-Free Eyecare Formulations. *Drug Development & Delivery,* October 2017, vol. 17 (7), 40-44
- *The Merck Manual,* 1999